# EUROPEAN PATENT SPECIFICATION

(11) **EP 1 364 005 B1**
(45) Date of publication and mention of the grant of the patent: **17.09.2008**
(21) Application number: 02709980.3
(22) Date of filing: 25.01.2002
(51) Int. Cl.: C12N 15/00

(54) **A METHOD FOR EVOLVING A CELL HAVING DESIRED PHENOTYPE AND EVOLVED CELLS**
VERFAHREN ZUR ENTWICKLUNG EINER ZELLE MIT EINEM GEWÜNSCHTEN PHÄNOTYP UND ENTWICKELTE ZELLEN
METHODE PERMETTANT DE METTRE AU POINT UNE CELLULE AYANT UN PHENOTYPE SOUHAITE ET CELLULES AINSI MISES AU POINT

(30) Priority: 25.01.2001 DK 200100129; 01.05.2001 DK 200100680; 26.06.2001 US 300438 P
(43) Date of publication of application: 26.11.2003
(73) Proprietor: Evolva Ltd., 4123 Allschwil (CH)
(72) Inventor: GOLDSMITH, Neil, Oxford OX4 1NX (GB); SOERENSEN, Alexandra, M., P., SantAna, DK-2840 Holte (DK); NIELSEN, Soeren, V., S., DK-3450 Alleroed (DK)
(74) Representative: HOEIBERG A/S
(86) International application number: PCT/DK2002/000057
(87) International publication number: WO 2002/059290

(56) References cited:
- WO-A-96/34112
- US-A- 5 316 922

### SEQUENCE LISTING

<110> Evolva Biotech AS
   Goldsmith, Neil
   Sørensen, Alexandra M. P.
   Nielsen, Søren V.S.
<120> A method for evolving a cell having desired phenotype and evolved cell s
<130> P 502 PC00
<150> DK PA 2001 00129
   <151> 2001-01-25
<150> DK PA 2001 00680
   <151> 2001-05-01
<150> US 60/300,438
   <151> 2001-06-26
<160> 5
<170> PatentIn version 3.1
<210> 1
   <211> 3417
   <212> DNA
   <213> Synthetic
<220>
   <221> misc_feature
   <222> (1902)..(2759)
   <223> Ampicillin resistance gene
<220>
   <221> rep_origin
   <222> (959)..(1899)
   <223> ColE1
<220>
   <221> misc_feature
   <222> (2891)..(3347)
   <223> f1-phage origin of replication
<220>
   <221> terminator
   <222> (495)..(823)
   <223> ADH1
<220>
   <221> promoter
   <222> (49)..(437)
   <223> Met25 promoter
<400> 1
   ctgatttgcc cgggcagttc aggctcatca ggcgcgccat gcagggattc ttcggatgca     60
   agggttcgaa tcccttagct ctcattattt tttgcttttt ctcttgaggt cacatgatcg    120
   caaaatggca aatggcacgt gaagctgtcg atattgggga actgtggtgg ttggcaaatg    180
   actaattaag ttagtcaagg cgccatcctc atgaaaactg tgtaacataa taaccgaagt    240
   gtcgaaaagg tggcaccttg tccaattgaa cacgctcgat gaaaaaaata agatatatat    300
   aaggttaagt aaagcgtctg ttagaaagga agtttttcct ttttcttgct ctcttgtctt    360
   ttcatctact atttccttcg tgtaatacag ggtcgtcaga tacatagata caattctatt    420
   acccccatcc atacaagctt ggcgccgaat tcgtcgaccc ggggatccgc ggccgcaggc    480
   ctaaattgat ctagagcttt ggacttcttc gccagaggtt tggtcaagtc tccaatcaag    540
   gttgtcggct tgtctacctt gccagaaatt tacgaaaaga tggaaaaggg tcaaatcgtt    600
   ggtagatacg ttgttgacac ttctaaataa gcgaatttct tatgatttat gatttttatt    660
   attaaataag ttataaaaaa aataagtgta tacaaatttt aaagtgactc ttaggtttta    720
   aaacgaaaat tcttgttctt gagtaactct ttcctgtagg tcaggttgct ttctcaggta    780
   tagcatgagg tcgctcttat tgaccacacc tctaccggca tgcccatggg ttaactgatc    840
   aatgcatcct gcatggcgcg cctgatgagc ctgaactgcc cgggcaaatc agctggacgt    900
   ctgcctgcat taatgaatcg gccaacgcgc ggggagaggc ggtttgcgta ttgggcgctc    960
   ttccgcttcc tcgctcactg actcgctgcg ctcggtcgtt cggctgcggc gagcggtatc   1020
   agctcactca aaggcggtaa tacggttatc cacagaatca ggggataacg caggaaagaa   1080
   catgtgagca aaaggccagc aaaaggccag gaaccgtaaa aaggccgcgt tgctggcgtt   1140
   tttccatagg ctccgccccc ctgacgagca tcacaaaaat cgacgctcaa gtcagaggtg   1200
   gcgaaacccg acaggactat aaagatacca ggcgtttccc cctggaagct ccctcgtgcg   1260
   ctctcctgtt ccgaccctgc cgcttaccgg atacctgtcc gcctttctcc cttcgggaag   1320
   cgtggcgctt tctcatagct cacgctgtag gtatctcagt tcggtgtagg tcgttcgctc   1380
   caagctgggc tgtgtgcacg aaccccccgt tcagcccgac cgctgcgcct tatccggtaa   1440
   ctatcgtctt gagtccaacc cggtaagaca cgacttatcg ccactggcag cagccactgg   1500
   taacaggatt agcagagcga ggtatgtagg cggtgctaca gagttcttga agtggtggcc   1560
   taactacggc tacactagaa ggacagtatt tggtatctgc gctctgctga agccagttac   1620
   cttcggaaaa agagttggta gctcttgatc cggcaaacaa accaccgctg gtagcggtgg   1680
   tttttttgtt tgcaagcagc agattacgcg cagaaaaaaa ggatctcaag aagatccttt   1740
   gatcttttct acggggtctg acgctcagtg gaacgaaaac tcacgttaag ggattttggt   1800
   catgagatta tcaaaaagga tcttcaccta gatcctttta aattaaaaat gaagttttaa 1860
   atcaatctaa agtatatatg agtaaacttg gtctgacagt taccaatgct taatcagtga 1920
   ggcacctatc tcagcgatct gtctatttcg ttcatccata gttgcctgac tccccgtcgt 1980
   gtagataact acgatacggg agggcttacc atctggcccc agtgctgcaa tgataccgcg 2040
   agacccacgc tcaccggctc cagatttatc agcaataaac cagccagccg gaagggccga 2100
   gcgcagaagt ggtcctgcaa ctttatccgc ctccatccag tctattaatt gttgccggga 2160
   agctagagta agtagttcgc cagttaatag tttgcgcaac gttgttgcca ttgctacagg 2220
   catcgtggtg tcacgctcgt cgtttggtat ggcttcattc agctccggtt cccaacgatc 2280
   aaggcgagtt acatgatccc ccatgttgtg caaaaaagcg gttagctcct tcggtcctcc 2340
   gatcgttgtc agaagtaagt tggccgcagt gttatcactc atggttatgg cagcactgca 2400
   taattctctt actgtcatgc catccgtaag atgcttttct gtgactggtg agtactcaac 2460
   caagtcattc tgagaatagt gtatgcggcg accgagttgc tcttgcccgg cgtcaatacg 2520
   ggataatacc gcgccacata gcagaacttt aaaagtgctc atcattggaa aacgttcttc 2580
   ggggcgaaaa ctctcaagga tcttaccgct gttgagatcc agttcgatgt aacccactcg 2640
   tgcacccaac tgatcttcag catcttttac tttcaccagc gtttctgggt gagcaaaaac 2700
   aggaaggcaa aatgccgcaa aaaagggaat aagggcgaca cggaaatgtt gaatactcat 2760
   actcttcctt tttcaatatt attgaagcat ttatcagggt tattgtctca tgagcggata 2820
   catatttgaa tgtatttaga aaaataaaca aataggggtt ccgcgcacat ttccccgaaa 2880
   agtgccacct gacgcgccct gtagcggcgc attaagcgcg gcgggtgtgg tggttacgcg 2940
   cagcgtgacc gctacacttg ccagcgccct agcgcccgct cctttcgctt tcttcccttc 3000
   ctttctcgcc acgttcgccg gctttccccg tcaagctcta aatcgggggc tccctttagg 3060
   gttccgattt agtgctttac ggcacctcga ccccaaaaaa cttgattagg gtgatggttc 3120
   acgtagtggg ccatcgccct gatagacggt ttttcgccct ttgacgttgg agtccacgtt 3180
   ctttaatagt ggactcttgt tccaaactgg aacaacactc aaccctatct cggtctattc 3240
   ttttgattta taagggattt tgccgatttc ggcctattgg ttaaaaaatg agctgattta 3300
   acaaaaattt aacgcgaatt ttaacaaaat attaacgctt acaatttcca ttcgccattc 3360
   aggctgcgca actgttggga agggcgatcg gtgcgggcct cttcgctatt acgccag 3417
<210> 2
   <211> 3501
   <212> DNA
   <213> Synthetic
<220>
   <221> misc_feature
   <222> (1986)..(2843)
   <223> Ampicillin resistance gene
<220>
   <221> rep_origin
   <222> (1043)..(1983)
   <223> ColE1
<220>
   <221> misc_feature
   <222> (2975)..(3431)
   <223> f1-phage origin of replication
<220>
   <221> terminator
   <222> (579)..(907)
   <223> ADH1
<220>
   <221> promoter
   <222> (49) .. (519)
   <223> Cup1 promoter
<400> 2
   ctgatttgcc cgggcagttc aggctcatca ggcgcgccat gcagggataa gccgatccca     60
   ttaccgacat ttgggcgcta tacgtgcata tgttcatgta tgtatctgta tttaaaacac    120
   ttttgtatta tttttcctca tatatgtgta taggtttata cggatgattt aattattact    180
   tcaccaccct ttatttcagg ctgatatctt agccttgtta ctagttagaa aaagacattt    240
   ttgctgtcag tcactgtcaa gagattcttt tgctggcatt tcttctagaa gcaaaaagag    300
   cgatgcgtct tttccgctga accgttccag caaaaaagac taccaacgca atatggattg    360
   tcagaatcat ataaaagaga agcaaataac tccttgtctt gtatcaattg cattataata    420
   tcttcttgtt agtgcaatat catatagaag tcatcgaaat agatattaag aaaaacaaac    480
   tgtacaatca atcaatcaat catcacataa aatgttcaaa gcttggcgcc gaattcgtcg    540
   acccggggat ccgcggccgc aggcctaaat tgatctagag ctttggactt cttcgccaga    600
   ggtttggtca agtctccaat caaggttgtc ggcttgtcta ccttgccaga aatttacgaa    660
   aagatggaaa agggtcaaat cgttggtaga tacgttgttg acacttctaa ataagcgaat    720
   ttcttatgat ttatgatttt tattattaaa taagttataa aaaaaataag tgtatacaaa    780
   ttttaaagtg actcttaggt tttaaaacga aaattcttgt tcttgagtaa ctctttcctg    840
   taggtcaggt tgctttctca ggtatagcat gaggtcgctc ttattgacca cacctctacc 900
   ggcatgccca tgggttaact gatcaatgca tcctgcatgg cgcgcctgat gagcctgaac 960
   tgcccgggca aatcagctgg acgtctgcct gcattaatga atcggccaac gcgcggggag 1020
   aggcggtttg cgtattgggc gctcttccgc ttcctcgctc actgactcgc tgcgctcggt 1080
   cgttcggctg cggcgagcgg tatcagctca ctcaaaggcg gtaatacggt tatccacaga 1140
   atcaggggat aacgcaggaa agaacatgtg agcaaaaggc cagcaaaagg ccaggaaccg 1200
   taaaaaggcc gcgttgctgg cgtttttcca taggctccgc ccccctgacg agcatcacaa 1260
   aaatcgacgc tcaagtcaga ggtggcgaaa cccgacagga ctataaagat accaggcgtt 1320
   tccccctgga agctccctcg tgcgctctcc tgttccgacc ctgccgctta ccggatacct 1380
   gtccgccttt ctcccttcgg gaagcgtggc gctttctcat agctcacgct gtaggtatct 1440
   cagttcggtg taggtcgttc gctccaagct gggctgtgtg cacgaacccc ccgttcagcc 1500
   cgaccgctgc gccttatccg gtaactatcg tcttgagtcc aacccggtaa gacacgactt 1560
   atcgccactg gcagcagcca ctggtaacag gattagcaga gcgaggtatg taggcggtgc 1620
   tacagagttc ttgaagtggt ggcctaacta cggctacact agaaggacag tatttggtat 1680
   ctgcgctctg ctgaagccag ttaccttcgg aaaaagagtt ggtagctctt gatccggcaa 1740
   acaaaccacc gctggtagcg gtggtttttt tgtttgcaag cagcagatta cgcgcagaaa 1800
   aaaaggatct caagaagatc ctttgatctt ttctacgggg tctgacgctc agtggaacga 1860
   aaactcacgt taagggattt tggtcatgag attatcaaaa aggatcttca cctagatcct 1920
   tttaaattaa aaatgaagtt ttaaatcaat ctaaagtata tatgagtaaa cttggtctga 1980
   cagttaccaa tgcttaatca gtgaggcacc tatctcagcg atctgtctat ttcgttcatc 2040
   catagttgcc tgactccccg tcgtgtagat aactacgata cgggagggct taccatctgg 2100
   ccccagtgct gcaatgatac cgcgagaccc acgctcaccg gctccagatt tatcagcaat 2160
   aaaccagcca gccggaaggg ccgagcgcag aagtggtcct gcaactttat ccgcctccat 2220
   ccagtctatt aattgttgcc gggaagctag agtaagtagt tcgccagtta atagtttgcg 2280
   caacgttgtt gccattgcta caggcatcgt ggtgtcacgc tcgtcgtttg gtatggcttc 2340
   attcagctcc ggttcccaac gatcaaggcg agttacatga tcccccatgt tgtgcaaaaa 2400
   agcggttagc tccttcggtc ctccgatcgt tgtcagaagt aagttggccg cagtgttatc 2460
   actcatggtt atggcagcac tgcataattc tcttactgtc atgccatccg taagatgctt 2520
   ttctgtgact ggtgagtact caaccaagtc attctgagaa tagtgtatgc ggcgaccgag 2580
   ttgctcttgc ccggcgtcaa tacgggataa taccgcgcca catagcagaa ctttaaaagt 2640
   gctcatcatt ggaaaacgtt cttcggggcg aaaactctca aggatcttac cgctgttgag 2700
   atccagttcg atgtaaccca ctcgtgcacc caactgatct tcagcatctt ttactttcac 2760
   cagcgtttct gggtgagcaa aaacaggaag gcaaaatgcc gcaaaaaagg gaataagggc 2820
   gacacggaaa tgttgaatac tcatactctt cctttttcaa tattattgaa gcatttatca 2880
   gggttattgt ctcatgagcg gatacatatt tgaatgtatt tagaaaaata aacaaatagg 2940
   ggttccgcgc acatttcccc gaaaagtgcc acctgacgcg ccctgtagcg gcgcattaag 3000
   cgcggcgggt gtggtggtta cgcgcagcgt gaccgctaca cttgccagcg ccctagcgcc 3060
   cgctcctttc gctttcttcc cttcctttct cgccacgttc gccggctttc cccgtcaagc 3120
   tctaaatcgg gggctccctt tagggttccg atttagtgct ttacggcacc tcgaccccaa 3180
   aaaacttgat tagggtgatg gttcacgtag tgggccatcg ccctgataga cggtttttcg 3240
   ccctttgacg ttggagtcca cgttctttaa tagtggactc ttgttccaaa ctggaacaac 3300
   actcaaccct atctcggtct attcttttga tttataaggg attttgccga tttcggccta 3360
   ttggttaaaa aatgagctga tttaacaaaa atttaacgcg aattttaaca aaatattaac 3420
   gcttacaatt tccattcgcc attcaggctg cgcaactgtt gggaagggcg atcggtgcgg 3480
   gcctcttcgc tattacgcca g 3501
<210> 3
   <211> 4188
   <212> DNA
   <213> Synthetic
<220>
   <221> misc_feature
   <222> (2673)..(3530)
   <223> Ampicillin resistance gene
<220>
   <221> rep_origin
   <222> (1730)..(2670)
   <223> ColEl
<220>
   <221> misc_feature
   <222> (3662)..(4118)
   <223> f1-phage origin of replication
<220>
   <221> terminator
   <222> (1027)..(1355)
   <223> ADH1
<220>
   <221> promoter
   <222> (582)..(969)
   <223> Met25 promoter
<220>
   <221> misc_feature
   <222> (1365)..(1603)
   <223> ARS1 (autonomous replicating sequence) for Yeast replication
<220>
   <221> misc_feature
   <222> (49)..(574)
   <223> lambda spacer DNA (22428-22923)
<400> 3
   ctgatttgcc cgggcagttc aggctcatca ggcgcgccat gcagggattc tggaaattgc     60
   aacgaaggaa gaaacctcgt tgctggaagc ctggaagaag tatcgggtgt tgctgaaccg    120
   tgttgataca tcaactgcac ctgatattga gtggcctgct gtccctgtta tggagtaatc    180
   gttttgtgat atgccgcaga aacgttgtat gaaataacgt tctgcggtta gttagtatat    240
   tgtaaagctg agtattggtt tatttggcga ttattatctt caggagaata atggaagttc    300
   tatgactcaa ttgttcatag tgtttacatc accgccaatt gcttttaaga ctgaacgcat    360
   gaaatatggt ttttcgtcat gttttgagtc tgctgttgat atttctaaag tcggtttttt    420
   ttcttcgttt tctctaacta ttttccatga aatacatttt tgattattat ttgaatcaat    480
   tccaattacc tgaagtcttt catctataat tggcattgta tgtattggtt tattggagta    540
   gatgcttgct tttctgagcc atagctctga tatcagatct tcttcggatg caagggttcg    600
   aatcccttag ctctcattat tttttgcttt ttctcttgag gtcacatgat cgcaaaatgg    660
   caaatggcac gtgaagctgt cgatattggg gaactgtggt ggttggcaaa tgactaatta    720
   agttagtcaa ggcgccatcc tcatgaaaac tgtgtaacat aataaccgaa gtgtcgaaaa    780
   ggtggcacct tgtccaattg aacacgctcg atgaaaaaaa taagatatat ataaggttaa    840
   gtaaagcgtc tgttagaaag gaagtttttc ctttttcttg ctctcttgtc ttttcatcta    900
   ctatttcctt cgtgtaatac agggtcgtca gatacataga tacaattcta ttacccccat    960
   ccatacaagc ttggcgccga attcgtcgac ccggggatcc gcggccgcag gcctaaattg   1020
   atctagagct ttggacttct tcgccagagg tttggtcaag tctccaatca aggttgtcgg   1080
   cttgtctacc ttgccagaaa tttacgaaaa gatggaaaag ggtcaaatcg ttggtagata   1140
   cgttgttgac acttctaaat aagcgaattt cttatgattt atgattttta ttattaaata   1200
   agttataaaa aaaataagtg tatacaaatt ttaaagtgac tcttaggttt taaaacgaaa   1260
   attcttgttc ttgagtaact ctttcctgta ggtcaggttg ctttctcagg tatagcatga 1320
   ggtcgctctt attgaccaca cctctaccgg catgcccatg ggttcttttg aaaagcaagc 1380
   ataaaagatc taaacataaa atctgtaaaa taacaagatg taaagataat gctaaatcat 1440
   ttggcttttt gattgattgt acaggaaaat atacatcgca gggggttgac ttttaccatt 1500
   tcaccgcaat ggaatcaaac ttgttgaaga gaatgttcac aggcgcatac gctacaatga 1560
   cccgattctt gctagccttt tctcggtctt gcaaacaacc gccaactgat caatgcatcc 1620
   tgcatggcgc gcctgatgag cctgaactgc ccgggcaaat cagctggacg tctgcctgca 1680
   ttaatgaatc ggccaacgcg cggggagagg cggtttgcgt attgggcgct cttccgcttc 1740
   ctcgctcact gactcgctgc gctcggtcgt tcggctgcgg cgagcggtat cagctcactc 1800
   aaaggcggta atacggttat ccacagaatc aggggataac gcaggaaaga acatgtgagc 1860
   aaaaggccag caaaaggcca ggaaccgtaa aaaggccgcg ttgctggcgt ttttccatag 1920
   gctccgcccc cctgacgagc atcacaaaaa tcgacgctca agtcagaggt ggcgaaaccc 1980
   gacaggacta taaagatacc aggcgtttcc ccctggaagc tccctcgtgc gctctcctgt 2040
   tccgaccctg ccgcttaccg gatacctgtc cgcctttctc ccttcgggaa gcgtggcgct 2100
   ttctcatagc tcacgctgta ggtatctcag ttcggtgtag gtcgttcgct ccaagctggg 2160
   ctgtgtgcac gaaccccccg ttcagcccga ccgctgcgcc ttatccggta actatcgtct 2220
   tgagtccaac ccggtaagac acgacttatc gccactggca gcagccactg gtaacaggat 2280
   tagcagagcg aggtatgtag gcggtgctac agagttcttg aagtggtggc ctaactacgg 2340
   ctacactaga aggacagtat ttggtatctg cgctctgctg aagccagtta ccttcggaaa 2400
   aagagttggt agctcttgat ccggcaaaca aaccaccgct ggtagcggtg gtttttttgt 2460
   ttgcaagcag cagattacgc gcagaaaaaa aggatctcaa gaagatcctt tgatcttttc 2520
   tacggggtct gacgctcagt ggaacgaaaa ctcacgttaa gggattttgg tcatgagatt 2580
   atcaaaaagg atcttcacct agatcctttt aaattaaaaa tgaagtttta aatcaatcta 2640
   aagtatatat gagtaaactt ggtctgacag ttaccaatgc ttaatcagtg aggcacctat 2700
   ctcagcgatc tgtctatttc gttcatccat agttgcctga ctccccgtcg tgtagataac 2760
   tacgatacgg gagggcttac catctggccc cagtgctgca atgataccgc gagacccacg 2820
   ctcaccggct ccagatttat cagcaataaa ccagccagcc ggaagggccg agcgcagaag 2880
   tggtcctgca actttatccg cctccatcca gtctattaat tgttgccggg aagctagagt 2940
   aagtagttcg ccagttaata gtttgcgcaa cgttgttgcc attgctacag gcatcgtggt 3000
   gtcacgctcg tcgtttggta tggcttcatt cagctccggt tcccaacgat caaggcgagt 3060
   tacatgatcc cccatgttgt gcaaaaaagc ggttagctcc ttcggtcctc cgatcgttgt   3120
   cagaagtaag ttggccgcag tgttatcact catggttatg gcagcactgc ataattctct   3180
   tactgtcatg ccatccgtaa gatgcttttc tgtgactggt gagtactcaa ccaagtcatt   3240
   ctgagaatag tgtatgcggc gaccgagttg ctcttgcccg gcgtcaatac gggataatac   3300
   cgcgccacat agcagaactt taaaagtgct catcattgga aaacgttctt cggggcgaaa   3360
   actctcaagg atcttaccgc tgttgagatc cagttcgatg taacccactc gtgcacccaa   3420
   ctgatcttca gcatctttta ctttcaccag cgtttctggg tgagcaaaaa caggaaggca   3480
   aaatgccgca aaaaagggaa taagggcgac acggaaatgt tgaatactca tactcttcct   3540
   ttttcaatat tattgaagca tttatcaggg ttattgtctc atgagcggat acatatttga   3600
   atgtatttag aaaaataaac aaataggggt tccgcgcaca tttccccgaa aagtgccacc   3660
   tgacgcgccc tgtagcggcg cattaagcgc ggcgggtgtg gtggttacgc gcagcgtgac   3720
   cgctacactt gccagcgccc tagcgcccgc tcctttcgct ttcttccctt cctttctcgc   3780
   cacgttcgcc ggctttcccc gtcaagctct aaatcggggg ctccctttag ggttccgatt   3840
   tagtgcttta cggcacctcg accccaaaaa acttgattag ggtgatggtt cacgtagtgg   3900
   gccatcgccc tgatagacgg tttttcgccc tttgacgttg gagtccacgt tctttaatag   3960
   tggactcttg ttccaaactg gaacaacact caaccctatc tcggtctatt cttttgattt   4020
   ataagggatt ttgccgattt cggcctattg gttaaaaaat gagctgattt aacaaaaatt   4080
   taacgcgaat tttaacaaaa tattaacgct tacaatttcc attcgccatt caggctgcgc   4140
   aactgttggg aagggcgatc ggtgcgggcc tcttcgctat tacgccag                4188
<210> 4
   <211> 11466
   <212> DNA
   <213> Synthetic
<220>
   <221> misc_feature
   <222> (3560)..(4247)
   <223> Tetrahymena thermophila macronuclear telomere
<220>
   <221> misc_feature
   <222> (6024)..(6711)
   <223> Tetrahymena thermophila macronuclear telomere
<220>
   <221> misc_feature
   <222> (9644)..(10388) <223> Autonomous replicating sequence
<220>
   <221> misc_feature
   <222> (10488)..(11465)
   <223> Centromere IV
<220>
   <221> rep_origin
   <222> (7198)..(7198)
   <223> Origin of replication, PMB1
<220>
   <221> misc_feature
   <222> (1962)..(2765)
   <223> URA3, orotidine-5'-phosphate decarboxylase coding sequence
<220>
   <221> misc_feature
   <222> (4893)..(5552)
   <223> HIS3, imidazoleglycerolphosphate dehydratase, coding sequence
<220>
   <221> misc_feature
   <222> (7956)..(8816)
   <223> AP(R), beta-lactamase, ampR ampicillin resistance, coding sequenc e
<220>
   <221> misc_feature
   <222> (9129)..(9803)
   <223> TRP1, phosphoribosylanthranilate isomerase, coding sequence
<400> 4
   ttctcatgtt tgacagctta tcatcgataa gctttaatgc ggtagtttat cacagttaaa     60
   ttgctaacgc agtcaggcac cgtgtatgaa atctaacaat gcgctcatcg tcatcctcgg    120
   caccgtcacc ctggatgctg taggcatagg cttggttatg ccggtactgc cgggcctctt    180
   gcgggatatc gtccattccg acagcatcgc cagtcactat ggcgtgctgc tagcgctata    240
   tgcgttgatg caatttctat gcgcacccgt tctcggagca ctgtccgacc gctttggccg    300
   ccgcccagtc ctgctcgctt cgctacttgg agccactatc gactacgcga tcatggcgac    360
   cacacccgtc ctgtggatca attcccttta gtataaattt cactctgaac catcttggaa    420
   ggaccggtaa ttatttcaaa tctctttttc aattgtatat gtgttatgtt atgtagtata    480
   ctctttcttc aacaattaaa tactctcggt agccaagttg gtttaaggcg caagacttta    540
   atttatcact acggaattgg cgcgccaatt ccgtaatctt gagatcgggc gttcgatcgc    600
   cccgggagat ttttttgttt tttatgtctt ccattcactt cccagacttg caagttgaaa 660
   tatttctttc aagggaattg atcctctacg ccggacgcat cgtggccggc atcaccggcg 720
   ccacaggtgc ggttgctggc gcctatatcg ccgacatcac cgatggggaa gatcgggctc 780
   gccacttcgg gctcatgagc gcttgtttcg gcgtgggtat ggtggcaggc cccgtggccg 840
   ggggactgtt gggcgccatc tccttgcatg caccattcct tgcggcggcg gtgctcaacg 900
   gcctcaacct actactgggc tgcttcctaa tgcaggagtc gcataaggga gagcgtcgac 960
   cgatgccctt gagagccttc aacccagtca gctccttccg gtgggcgcgg ggcatgacta 1020
   tcgtcgccgc acttatgact gtcttcttta tcatgcaact cgtaggacag gtgccggcag 1080
   cgctctgggt cattttcggc gaggaccgct ttcgctggag cgcgacgatg atcggcctgt 1140
   cgcttgcggt attcggaatc ttgcacgccc tcgctcaagc cttcgtcact ggtcccgcca 1200
   ccaaacgttt cggcgagaag caggccatta tcgccggcat ggcggccgac gcgctgggct 1260
   acgtcttgct ggcgttcgcg acgcgaggct ggatggcctt ccccattatg attcttctcg 1320
   cttccggcgg catcgggatg cccgcgttgc aggccatgct gtccaggcag gtagatgacg 1380
   accatcaggg acagcttcaa ggatcgctcg cggctcttac cagcctaact tcgatcactg 1440
   gaccgctgat cgtcacggcg atttatgccg cctcggcgag cacatggaac gggttggcat 1500
   ggattgtagg cgccgcccta taccttgtct gcctccccgc gttgcgtcgc ggtgcatgga 1560
   gccgggccac ctcgacctga atggaagccg gcggcacctc gctaacggat tcaccactcc 1620
   aagaattgga gccaatcaat tcttgcggag aactgtgaat gcgcaaacca acccttggca 1680
   gaacatatcc atcgcgtccg ccatctccag cagccgcacg cggcgcatcc ccccccccct 1740
   ttcaattcaa ttcatcattt ttttttattt cttttttttg atttcggttt ctttgaaatt 1800
   tttttgattc ggtaatctcc gaacagaagg aagaacgaag gaaggagcac agacttagat 1860
   tggtatatat acgcatatgt agtgttgaag aaacatgaaa ttgcccagta ttcttaaccc 1920
   aactgcacag aacaaaaacc tgcaggaaac gaagataaat catgtcgaaa gctacatata 1980
   aggaacgtgc tgctactcat cctagtcctg ttgctgccaa gctatttaat atcatgcacg 2040
   aaaagcaaac aaacttgtgt gcttcattgg atgttcgtac caccaaggaa ttactggagt 2100
   tagttgaagc attaggtccc aaaatttgtt tactaaaaac acatgtggat atcttgactg 2160
   atttttccat ggagggcaca gttaagccgc taaaggcatt atccgccaag tacaattttt 2220
   tactcttcga agacagaaaa tttgctgaca ttggtaatac agtcaaattg cagtactctg 2280
   cgggtgtata cagaatagca gaatgggcag acattacgaa tgcacacggt gtggtgggcc 2340
   caggtattgt tagcggtttg aagcaggcgg cagaagaagt aacaaaggaa cctagaggcc 2400
   ttttgatgtt agcagaattg tcatgcaagg gctccctatc tactggagaa tatactaagg   2460
   gtactgttga cattgcgaag agcgacaaag attttgttat cggctttatt gctcaaagag   2520
   acatgggtgg aagagatgaa ggttacgatt ggttgattat gacacccggt gtgggtttag   2580
   atgacaaggg agacgcattg ggtcaacagt atagaaccgt ggatgatgtg gtctctacag   2640
   gatctgacat tattattgtt ggaagaggac tatttgcaaa gggaagggat gctaaggtag   2700
   agggtgaacg ttacagaaaa gcaggctggg aagcatattt gagaagatgc ggccagcaaa   2760
   actaaaaaac tgtattataa gtaaatgcat gtatactaaa ctcacaaatt agagcttcaa   2820
   tttaattata tcagttatta ctcgggcgta atgattttta taatgacgaa aaaaaaaaaa   2880
   ttggaaagaa aagggggggg gggcagcgtt gggtcctggc cacgggtgcg catgatcgtg   2940
   ctcctgtcgt tgaggacccg gctaggctgg cggggttgcc ttactggtta gcagaatgaa   3000
   tcaccgatac gcgagcgaac gtgaagcgac tgctgctgca aaacgtctgc gacctgagca   3060
   acaacatgaa tggtcttcgg tttccgtgtt tcgtaaagtc tggaaacgcg gaagtcagcg   3120
   ccctgcacca ttatgttccg gatctgcatc gcaggatgct gctggctacc ctgtggaaca   3180
   cctacatctg tattaacgaa gcgctggcat tgaccctgag tgatttttct ctggtcccgc   3240
   cgcatccata ccgccagttg tttaccctca caacgttcca gtaaccgggc atgttcatca   3300
   tcagtaaccc gtatcgtgag catcctctct cgtttcatcg gtatcattac ccccatgaac   3360
   agaaattccc ccttacacgg aggcatcaag tgaccaaaca ggaaaaaacc gcccttaaca   3420
   tggcccgctt tatcagaagc cagacattaa cgcttctgga gaaactcaac gagctggacg   3480
   cggatgaaca ggcagacatc tgtgaatcgc ttcacgacca cgctgatgag ctttaccgca   3540
   gccctcgagg gataagcttc atttttagat aaaatttatt aatcatcatt aatttcttga   3600
   aaaacatttt atttattgat cttttataac aaaaaaccct tctaaaagtt tatttttgaa   3660
   tgaaaaactt ataaaaattt atgaaaacta caaaaaataa aatttttaat taaaataatt   3720
   ttgataagaa cttcaatctt tgactagcta gcttagtcat ttttgagatt taattaatat    3780
   tttatgttta ttcatatata aactattcaa aatattatag aatttaaaca ttttaacatc   3840
   ttaatcattc ataaataact aaaaatcaaa gtattacatc aataaataac ttttactcaa   3900
   tgtcaaagaa ttattggggt tggggttggg gttggggttg gggttggggt tggggttggg   3960
   gttggggttg gggttggggt tggggttggg gttggggttg gggttggggt tggggttggg   4020
   gttggggttg gggttggggt tggggttggg gttggggttg gggttggggt tggggttggg   4080
   gttggggttg gggttggggt tggggttggg gttggggttg gggttggggt tggggttggg   4140
   gttggggttg gggttggggt tggggttggg gttggggttg gggtgggaaa acagcattca   4200
   ggtattagaa gaatatcctg attcaggtga aaatattgtt gatgcgcggg atcctcgggg 4260
   acaccaaata tggcgatctc ggccttttcg tttcttggag ctgggacatg tttgccatcg 4320
   atccatctac caccagaacg gccgttagat ctgctgccac cgttgtttcc accgaagaaa 4380
   ccaccgttgc cgtaaccacc acgacggttg ttgctaaaga agctgccacc gccacggcca 4440
   ccgttgtagc cgccgttgtt gttattgtag ttgctcatgt tatttctggc acttcttggt 4500
   tttcctctta agtgaggagg aacataacca ttctcgttgt tgtcgttgat gcttaaattt 4560
   tgcacttgtt cgctcagttc agccataata tgaaatgctt ttcttgttgt tcttacggaa 4620
   taccacttgc cacctatcac cacaactaac tttttcccgt tcctccatct cttttatatt 4680
   ttttttctcg atcgagttca agagaaaaaa aaagaaaaag caaaaagaaa aaaggaaagc 4740
   gcgcctcgtt cagaatgaca cgtatagaat gatgcattac cttgtcatct tcagtatcat 4800
   actgttcgta tacatactta ctgacattca taggtataca tatatacaca tgtatatata 4860
   tcgtatgctg cagctttaaa taatcggtgt cactacataa gaacaccttt ggtggaggga 4920
   acatcgttgg taccattggg cgaggtggct tctcttatgg caaccgcaag agccttgaac 4980
   gcactctcac tacggtgatg atcattcttg cctcgcagac aatcaacgtg gagggtaatt 5040
   ctgctagcct ctgcaaagct ttcaagaaaa tgcgggatca tctcgcaaga gagatctcct 5100
   actttctccc tttgcaaacc aagttcgaca actgcgtacg gcctgttcga aagatctacc 5160
   accgctctgg aaagtgcctc atccaaaggc gcaaatcctg atccaaacct ttttactcca 5220
   cgcgccagta gggcctcttt aaaagcttga ccgagagcaa tcccgcagtc ttcagtggtg 5280
   tgatggtcgt ctatgtgtaa gtcaccaatg cactcaacga ttagcgacca gccggaatgc 5340
   ttggccagag catgtatcat atggtccaga aaccctatac ctgtgtggac gttaatcact 5400
   tgcgattgtg tggcctgttc tgctactgct tctgcctctt tttctgggaa gatcgagtgc 5460
   tctatcgcta ggggaccacc ctttaaagag atcgcaatct gaatcttggt ttcatttgta 5520
   atacgcttta ctagggcttt ctgctctgtc atctttgcct tcgtttatct tgcctgctca 5580
   ttttttagta tattcttcga agaaatcaca ttactttata taatgtataa ttcattatgt 5640
   gataatgcca atcgctaaga aaaaaaaaga gtcatccgct aggtggaaaa aaaaaaatga 5700
   aaatcattac cgaggcataa aaaaatatag agtgtactag aggaggccaa gagtaataga 5760
   aaaagaaaat tgcgggaaag gactgtgtta tgacttccct gactaatgcc gtgttcaaac 5820
   gatacctggc agtgactcct agcgctcacc aagctcttaa aacgagaatt aagaaaaagt 5880
   cgtcatcttt cgataagttt ttcccacagc aaagcaatag tagaaaaaaa caatgggaaa 5940
   cgttgaatga agacaaagcg tcgtggttta aaaggaaata cgctcacgta catgctaggg 6000
   aacaggaccg tgcagcggat cccgcgcatc aacaatattt tcacctgaat caggatattc 6060
   ttctaatacc tgaatgctgt tttcccaccc caaccccaac cccaacccca accccaaccc 6120
   caaccccaac cccaacccca accccaaccc caaccccaac cccaacccca accccaaccc 6180
   caaccccaac cccaacccca accccaaccc caaccccaac cccaacccca accccaaccc 6240
   caaccccaac cccaacccca accccaaccc caaccccaac cccaacccca accccaaccc 6300
   caaccccaac cccaacccca accccaaccc caaccccaac cccaacccca accccaataa 6360
   ttctttgaca ttgagtaaaa gttatttatt gatgtaatac tttgattttt agttatttat 6420
   gaatgattaa gatgttaaaa tgtttaaatt ctataatatt ttgaatagtt tatatatgaa 6480
   taaacataaa atattaatta aatctcaaaa atgactaagc tagctagtca aagattgaag 6540
   ttcttatcaa aattatttta attaaaaatt ttattttttg tagttttcat aaatttttat 6600
   aagtttttca ttcaaaaata aacttttaga agggtttttt gttataaaag atcaataaat 6660
   aaaatgtttt tcaagaaatt aatgatgatt aataaatttt atctaaaaat gaagcttatc 6720
   cctcgagggc tgcctcgcgc gtttcggtga tgacggtgaa aacctctgac acatgcagct 6780
   cccggagacg gtcacagctt gtctgtaagc ggatgccggg agcagacaag cccgtcaggg 6840
   cgcgtcagcg ggtgttggcg ggtgtcgggg cgcagccatg acccagtcac gtagcgatag 6900
   cggagtgtat actggcttaa ctatgcggca tcagagcaga ttgtactgag agtgcaccat 6960
   atgcggtgtg aaataccgca cagatgcgta aggagaaaat accgcatcag gcgctcttcc 7020
   gcttcctcgc tcactgactc gctgcgctcg gtcgttcggc tgcggcgagc ggtatcagct 7080
   cactcaaagg cggtaatacg gttatccaca gaatcagggg ataacgcagg aaagaacatg 7140
   tgagcaaaag gccagcaaaa ggccaggaac cgtaaaaagg ccgcgttgct ggcgtttttc 7200
   cataggctcc gcccccctga cgagcatcac aaaaatcgac gctcaagtca gaggtggcga 7260
   aacccgacag gactataaag ataccaggcg tttccccctg gaagctccct cgtgcgctct 7320
   cctgttccga ccctgccgct taccggatac ctgtccgcct ttctcccttc gggaagcgtg 7380
   gcgctttctc atagctcacg ctgtaggtat ctcagttcgg tgtaggtcgt tcgctccaag 7440
   ctgggctgtg tgcacgaacc ccccgttcag cccgaccgct gcgccttatc cggtaactat 7500
   cgtcttgagt ccaacccggt aagacacgac ttatcgccac tggcagcagc cactggtaac 7560
   aggattagca gagcgaggta tgtaggcggt gctacagagt tcttgaagtg gtggcctaac 7620
   tacggctaca ctagaaggac agtatttggt atctgcgctc tgctgaagcc agttaccttc 7680
   ggaaaaagag ttggtagctc ttgatccggc aaacaaacca ccgctggtag cggtggtttt 7740
   tttgtttgca agcagcagat tacgcgcaga aaaaaaggat ctcaagaaga tcctttgatc 7800
   ttttctacgg ggtctgacgc tcagtggaac gaaaactcac gttaagggat tttggtcatg 7860
   agattatcaa aaaggatctt cacctagatc cttttaaatt aaaaatgaag ttttaaatca 7920
   atctaaagta tatatgagta aacttggtct gacagttacc aatgcttaat cagtgaggca 7980
   cctatctcag cgatctgtct atttcgttca tccatagttg cctgactccc cgtcgtgtag 8040
   ataactacga tacgggaggg cttaccatct ggccccagtg ctgcaatgat accgcgagac 8100
   ccacgctcac cggctccaga tttatcagca ataaaccagc cagccggaag ggccgagcgc 8160
   agaagtggtc ctgcaacttt atccgcctcc atccagtcta ttaattgttg ccgggaagct 8220
   agagtaagta gttcgccagt taatagtttg cgcaacgttg ttgccattgc tgcaggcatc 8280
   gtggtgtcac gctcgtcgtt tggtatggct tcattcagct ccggttccca acgatcaagg 8340
   cgagttacat gatcccccat gttgtgcaaa aaagcggtta gctccttcgg tcctccgatc 8400
   gttgtcagaa gtaagttggc cgcagtgtta tcactcatgg ttatggcagc actgcataat 8460
   tctcttactg tcatgccatc cgtaagatgc ttttctgtga ctggtgagta ctcaaccaag 8520
   tcattctgag aatagtgtat gcggcgaccg agttgctctt gcccggcgtc aacacgggat 8580
   aataccgcgc cacatagcag aactttaaaa gtgctcatca ttggaaaacg ttcttcgggg 8640
   cgaaaactct caaggatctt accgctgttg agatccagtt cgatgtaacc cactcgtgca 8700
   cccaactgat cttcagcatc ttttactttc accagcgttt ctgggtgagc aaaaacagga 8760
   aggcaaaatg ccgcaaaaaa gggaataagg gcgacacgga aatgttgaat actcatactc 8820
   ttcctttttc aatattattg aagcatttat cagggttatt gtctcatgag cggatacata 8880
   tttgaatgta tttagaaaaa taaacaaata ggggttccgc gcacatttcc ccgaaaagtg 8940
   ccacctgacg tctaagaaac cattattatc atgacattaa cctataaaaa taggcgtatc 9000
   acgaggccct ttcgtcttca agaattaatt cggtcgaaaa aagaaaagga gagggccaag 9060
   agggagggca ttggtgacta ttgagcacgt gagtatacgt gattaagcac acaaaggcag 9120
   cttggagtat gtctgttatt aatttcacag gtagttctgg tccattggtg aaagtttgcg 9180
   gcttgcagag cacagaggcc gcagaatgtg ctctagattc cgatgctgac ttgctgggta 9240
   ttatatgtgt gcccaataga aagagaacaa ttgacccggt tattgcaagg aaaatttcaa 9300
   gtcttgtaaa agcatataaa aatagttcag gcactccgaa atacttggtt ggcgtgtttc 9360
   gtaatcaacc taaggaggat gttttggctc tggtcaatga ttacggcatt gatatcgtcc 9420
   aactgcatgg agatgagtcg tggcaagaat accaagagtt cctcggtttg ccagttatta 9480
   aaagactcgt atttccaaaa gactgcaaca tactactcag tgcagcttca cagaaacctc 9540
   attcgtttat tcccttgttt gattcagaag caggtgggac aggtgaactt ttggattgga 9600
   actcgatttc tgactgggtt ggaaggcaag agagccccga aagcttacat tttatgttag 9660
   ctggtggact gacgccagaa aatgttggtg atgcgcttag attaaatggc gttattggtg 9720
   ttgatgtaag cggaggtgtg gagacaaatg gtgtaaaaga ctctaacaaa atagcaaatt 9780
   tcgtcaaaaa tgctaagaaa taggttatta ctgagtagta tttatttaag tattgtttgt 9840
   gcacttgcct gcaggccttt tgaaaagcaa gcataaaaga tctaaacata aaatctgtaa 9900
   aataacaaga tgtaaagata atgctaaatc atttggcttt ttgattgatt gtacaggaaa 9960
   atatacatcg cagggggttg acttttacca tttcaccgca atggaatcaa acttgttgaa 10020
   gagaatgttc acaggcgcat acgctacaat gacccgattc ttgctagcct tttctcggtc 10080
   ttgcaaacaa ccgccggcag cttagtatat aaatacacat gtacatacct ctctccgtat 10140
   cctcgtaatc attttcttgt atttatcgtc ttttcgctgt aaaaacttta tcacacttat 10200
   ctcaaataca cttattaacc gcttttacta ttatcttcta cgctgacagt aatatcaaac 10260
   agtgacacat attaaacaca gtggtttctt tgcataaaca ccatcagcct caagtcgtca 10320
   agtaaagatt tcgtgttcat gcagatagat aacaatctat atgttgataa ttagcgttgc 10380
   ctcatcaatg cgagatccgt ttaaccggac cctagtgcac ttaccccacg ttcggtccac 10440
   tgtgtgccga acatgctcct tcactatttt aacatgtgga attaattcta aatcctcttt 10500
   atatgatctg ccgatagata gttctaagtc attgaggttc atcaacaatt ggattttctg 10560
   tttactcgac ttcaggtaaa tgaaatgaga tgatacttgc ttatctcata gttaactcta 10620
   agaggtgata cttatttact gtaaaactgt gacgataaaa ccggaaggaa gaataagaaa 10680
   actcgaactg atctataatg cctattttct gtaaagagtt taagctatga aagcctcggc 10740
   attttggccg ctcctaggta gtgctttttt tccaaggaca aaacagtttc tttttcttga 10800
   gcaggtttta tgtttcggta atcataaaca ataaataaat tatttcattt atgtttaaaa 10860
   ataaaaaata aaaaagtatt ttaaattttt aaaaaagttg attataagca tgtgaccttt 10920
   tgcaagcaat taaattttgc aatttgtgat tttaggcaaa agttacaatt tctggctcgt 10980
   gtaatatatg tatgctaaag tgaactttta caaagtcgat atggacttag tcaaaagaaa 11040
   ttttcttaaa aatatatagc actagccaat ttagcacttc tttatgagat atattataga 11100
   ctttattaag ccagatttgt gtattatatg tatttacccg gcgaatcatg gacatacatt 11160
   ctgaaatagg taatattctc tatggtgaga cagcatagat aacctaggat acaagttaaa 11220
   agctagtact gttttgcagt aatttttttc ttttttataa gaatgttacc acctaaataa 11280
   gttataaagt caatagttaa gtttgatatt tgattgtaaa ataccgtaat atatttgcat 11340
   gatcaaaagg ctcaatgttg actagccagc atgtcaacca ctatattgat caccgatata 11400
   tggacttcca caccaactag taatatgaca ataaattcaa gatattcttc atgagaatgg 11460
   cccaga 11466
<210> 5
   <211> 4313
   <212> DNA
   <213> Synthetic
<220>
   <221> misc_feature
   <222> (3787)..(4243)
   <223> f1-phage origin of replication
<220>
   <221> misc_feature
   <222> (2798)..(3655)
   <223> Ampicillin resistance gene
<220>
   <221> terminator
   <222> (1100)..(1428)
   <223>
<220>

   <221> promoter
   <222> (655)..(1042)
   <223> Met25 promotor
<220>
   <221> rep_origin
   <222> (1855)..(2795)
   <223> ColEl
<400> 5
   ctgatttgcc cgggcagttc aggctcatca ggcgcgccat gcagggatcg gcgttttccg     60
   gaactggaaa accgacatgt tgatttcctg aaacgggata tcatcaaagc catgaacaaa    120
   gcagccgcgc tggatgaact gataccgggg ttgctgagtg aatatatcga acagtcaggt    180
   taacaggctg cggcattttg tccgcgccgg gcttcgctca ctgttcaggc cggagccaca    240
   gaccgccgtt gaatgggcgg atgctaatta ctatctcccg aaagaatccg cataccagga    300
   agggcgctgg gaaacactgc cctttcagcg ggccatcatg aatgcgatgg gcagcgacta    360
   catccgtgag gtgaatgtgg tgaagtctgc ccgtgtcggt tattccaaaa tgctgctggg    420
   tgtttatgcc tactttatag agcataagca gcgcaacacc cttatctggt tgccgacgga    480
   tggtgatgcc gagaacttta tgaaaaccca cgttgagccg actattcgtg atattccgtc    540
   gctgctggcg ctggccccgt ggtatggcaa aaagcaccgg gataacacgc tcaccatgaa 600
   gcgtttcact aatgggcgtg gcttctggtg cctgggcggt aaagcggaga tcttcttcgg 660
   atgcaagggt tcgaatccct tagctctcat tattttttgc tttttctctt gaggtcacat 720
   gatcgcaaaa tggcaaatgg cacgtgaagc tgtcgatatt ggggaactgt ggtggttggc 780
   aaatgactaa ttaagttagt caaggcgcca tcctcatgaa aactgtgtaa cataataacc 840
   gaagtgtcga aaaggtggca ccttgtccaa ttgaacacgc tcgatgaaaa aaataagata 900
   tatataaggt taagtaaagc gtctgttaga aaggaagttt ttcctttttc ttgctctctt 960
   gtcttttcat ctactatttc cttcgtgtaa tacagggtcg tcagatacat agatacaatt 1020
   ctattacccc catccataca agcttggcgc cgaattcgtc gacccgggga tccgcggccg 1080
   caggcctaaa ttgatctaga gctttggact tcttcgccag aggtttggtc aagtctccaa 1140
   tcaaggttgt cggcttgtct accttgccag aaatttacga aaagatggaa aagggtcaaa 1200
   tcgttggtag atacgttgtt gacacttcta aataagcgaa tttcttatga tttatgattt 1260
   ttattattaa ataagttata aaaaaaataa gtgtatacaa attttaaagt gactcttagg 1320
   ttttaaaacg aaaattcttg ttcttgagta actctttcct gtaggtcagg ttgctttctc 1380
   aggtatagca tgaggtcgct cttattgacc acacctctac cggcatgccc atggatgacc 1440
   cctccagcgt gttttatctc tgcgagcata atgcctgcgt catccgccag caggagctgg 1500
   actttactga tgcccgttat atctgcgaaa agaccgggat ctggacccgt gatggcattc 1560
   tctggttttc gtcatccggt gaagagattg agccacctga cagtgtgacc tttcacatct 1620
   ggacagcgta cagcccgttc accacctggg tgcagattgt caaagactgg atgaaaacga 1680
   aaggggatac gggaaaacgt aaaaccttcg taaacaccac gctcggtgag atgatcaatg 1740
   catcctgcat ggcgcgcctg atgagcctga actgcccggg caaatcagct ggacgtctgc 1800
   ctgcattaat gaatcggcca acgcgcgggg agaggcggtt tgcgtattgg gcgctcttcc 1860
   gcttcctcgc tcactgactc gctgcgctcg gtcgttcggc tgcggcgagc ggtatcagct 1920
   cactcaaagg cggtaatacg gttatccaca gaatcagggg ataacgcagg aaagaacatg 1980
   tgagcaaaag gccagcaaaa ggccaggaac cgtaaaaagg ccgcgttgct ggcgtttttc 2040
   cataggctcc gcccccctga cgagcatcac aaaaatcgac gctcaagtca gaggtggcga 2100
   aacccgacag gactataaag ataccaggcg tttccccctg gaagctccct cgtgcgctct 2160
   cctgttccga ccctgccgct taccggatac ctgtccgcct ttctcccttc gggaagcgtg 2220
   gcgctttctc atagctcacg ctgtaggtat ctcagttcgg tgtaggtcgt tcgctccaag 2280
   ctgggctgtg tgcacgaacc ccccgttcag cccgaccgct gcgccttatc cggtaactat 2340
   cgtcttgagt ccaacccggt aagacacgac ttatcgccac tggcagcagc cactggtaac 2400
   aggattagca gagcgaggta tgtaggcggt gctacagagt tcttgaagtg gtggcctaac 2460
   tacggctaca ctagaaggac agtatttggt atctgcgctc tgctgaagcc agttaccttc 2520
   ggaaaaagag ttggtagctc ttgatccggc aaacaaacca ccgctggtag cggtggtttt 2580
   tttgtttgca agcagcagat tacgcgcaga aaaaaaggat ctcaagaaga tcctttgatc 2640
   ttttctacgg ggtctgacgc tcagtggaac gaaaactcac gttaagggat tttggtcatg 2700
   agattatcaa aaaggatctt cacctagatc cttttaaatt aaaaatgaag ttttaaatca 2760
   atctaaagta tatatgagta aacttggtct gacagttacc aatgcttaat cagtgaggca 2820
   cctatctcag cgatctgtct atttcgttca tccatagttg cctgactccc cgtcgtgtag 2880
   ataactacga tacgggaggg cttaccatct ggccccagtg ctgcaatgat accgcgagac 2940
   ccacgctcac cggctccaga tttatcagca ataaaccagc cagccggaag ggccgagcgc 3000
   agaagtggtc ctgcaacttt atccgcctcc atccagtcta ttaattgttg ccgggaagct 3060
   agagtaagta gttcgccagt taatagtttg cgcaacgttg ttgccattgc tacaggcatc 3120
   gtggtgtcac gctcgtcgtt tggtatggct tcattcagct ccggttccca acgatcaagg 3180
   cgagttacat gatcccccat gttgtgcaaa aaagcggtta gctccttcgg tcctccgatc 3240
   gttgtcagaa gtaagttggc cgcagtgtta tcactcatgg ttatggcagc actgcataat 3300
   tctcttactg tcatgccatc cgtaagatgc ttttctgtga ctggtgagta ctcaaccaag 3360
   tcattctgag aatagtgtat gcggcgaccg agttgctctt gcccggcgtc aatacgggat 3420
   aataccgcgc cacatagcag aactttaaaa gtgctcatca ttggaaaacg ttcttcgggg 3480
   cgaaaactct caaggatctt accgctgttg agatccagtt cgatgtaacc cactcgtgca 3540
   cccaactgat cttcagcatc ttttactttc accagcgttt ctgggtgagc aaaaacagga 3600
   aggcaaaatg ccgcaaaaaa gggaataagg gcgacacgga aatgttgaat actcatactc 3660
   ttcctttttc aatattattg aagcatttat cagggttatt gtctcatgag cggatacata 3720
   tttgaatgta tttagaaaaa taaacaaata ggggttccgc gcacatttcc ccgaaaagtg 3780
   ccacctgacg cgccctgtag cggcgcatta agcgcggcgg gtgtggtggt tacgcgcagc 3840
   gtgaccgcta cacttgccag cgccctagcg cccgctcctt tcgctttctt cccttccttt 3900
   ctcgccacgt tcgccggctt tccccgtcaa gctctaaatc gggggctccc tttagggttc 3960
   cgatttagtg ctttacggca cctcgacccc aaaaaacttg attagggtga tggttcacgt 4020
   agtgggccat cgccctgata gacggttttt cgccctttga cgttggagtc cacgttcttt 4080
   aatagtggac tcttgttcca aactggaaca acactcaacc ctatctcggt ctattctttt 4140
   gatttataag ggattttgcc gatttcggcc tattggttaa aaaatgagct gatttaacaa   4200
   aaatttaacg cgaattttaa caaaatatta acgcttacaa tttccattcg ccattcaggc   4260
   tgcgcaactg ttgggaaggg cgatcggtgc gggcctcttc gctattacgc cag           4313

## Description

This application is a nonprovisional of U.S. provisional application Serial No. 60/300,438 filed 26 June 2001, It claims the priority of Danish patent application number PA 2001 00129 filed on 25 January 2001.

The present invention relates to the field of evolving new genomes in cells to acquire cells producing novel metabolites and/or novel pathways.

### Background of the Invention

Recombination of cells in order to optimise or produce heterologous proteins is a well-established practice in molecular biology.

The traditional approach to engineered molecular evolution relates to optimisation of an individual gene having a specific phenotype..The strategy is to clone a gene, identify a function for the gene and an assay for selecting the gene, mutate selected positions in the gene and select variants of the gene for improvement in the known function of the gene. A variant having a desired function may then be expressed in a suitable host cell.

However, the traditional approach has several drawbacks when it comes to evolution of cells having new properties, since the approach only relates to discrete genes. Multiple genes that cooperatively confer a single phenotype cannot be optimised in this manner. Furthermore, the traditional approach only leads to a very limited number of combinations or permutations in or cell or even for a single gene.

Evolution of cells having new properties have been described in for example WO 98/31837 wherein a method of evolving cells towards acquisition of new properties employing iterative cycles of recombination and selection/screening for evolution is discussed.

In WO 97/35966 a process of recursive sequence recombination in order to evolve new metabolic pathways are discussed, and in WO 00/04190 a process of recursive sequence recombination in order to evolve whole cells and organisms having desired properties.

Whether using the traditional approach of optimising individual genes or conducting iterative cycles of recombination, the individual genes in the cells in question are recombined, i.e. changed with foreign genetic material evolving new genes.

A major drawback when evolving new genes in this manner is, that each cycle of recombination may as well result in a failure leading to a nonsense gene as a success leading to an optimised gene.

### Summary of the invention

It is an aim by the present invention to evolve a cell or a composition of cells having a desired property or functionality. The principle behind the evolution of cells according to the invention is to produce a great diversity of genes in each cell subjected to evolution and a great diversity of genes among the cells in a composition according to the invention and to exchange the genes between the cells from time to time.

Accordingly the invention relates to a method for evolving a cell having a desired functionality, said method comprising the steps of
a) obtaining a composition of cells, at least one cell of said composition comprising
   a1) In the range of 10 to 1000 heterologous expressible nucleotide sequences, at least one of said sequences being incorporated into an artificial chromosome in the cell, and/or
   a2) at least two expression cassettes of the following formula:

      [rs₂-SP-PR-X-TR-SP-rs₁]

      wherein
      rs₁ and rs₂ together denotes a restriction site,
      SP individually denotes a spacer,
      PR denotes a promoter, capable of functioning in the first cell,
      X denotes an expressible nucleotide sequence,
      TR denotes a terminator, and/or
b) determining at least one screening functionality,
c) screening the cells of the composition with respect to at least one screening criterion related to the determined screening functionality.
d) selecting cells meeting the at least one screening criterion related to the determined screening functionality,
e) combining the expressible nucleotide sequences of the selected cells with expressible sequences from another composition of cells, and
f) repeating steps b) to e) as required until at least one cell has acquired the desired functionality.

The term "expressible sequence" is used with its normal meaning, i.e. a sequence capable of being expressed in the host cells in question.

In step c) the cells may be screened for more than one screening criterion related to the determined functionality, for example the cells may be screened sequentially for first one screening criteria and subsequently another screening criteria, before the cells having the determined screening functionality are selected. In another embodiment the two or more screening criteria are applied to the cells at the same time.

Also the combination of expressible sequences may be combined in a one-step process, or by a process of several steps of mixing or combining the expressible sequences, independent of whether the combination relates to combination of expressible sequences as such or combination of expression cassettes or combination of chromosomes.

Step f) may be repeated until cells having the desired functionality are obtained. Thereby step f) may be repeated from 0 to at least 200 times, preferably from 0 to 150 times, such as from 0 to 100 times, such as from 0 to 80 times, such as from 0 to 60 times, such as from 0 to 20 times.

In the present context the desired functionality is the functionality of the cell(s) when having been evolved to for example produce a desired compound, wherein the compound may be known, but not previously produced by the cell or the compound may be novel. Furthermore, the desired functionality may be production of a series of compounds, such as compounds having a synergistic effect, for example intermediates and metabolites in a pathway. The screening functionality is the functionality during the screening rounds. The screening functionality is normally different from the desired functionality, but in some embodiments the screening functionality is identical with the desired functionality. The screening functionality is also referred to as the predetermined functionality in the present context.

Yet a further aspect of the invention relates to the cells evolved by the methods according to the present invention. Accordingly, the invention also relates to a cell comprising at least one concatemer of individual oligonucleotide cassettes, each concatemer individually comprising an oligonucleotide sequence of the following formula in 5'→3' direction:

[rs₂-SP-PR-X-TR-SP-rs₁]ₙ

wherein
rs₁ and rs₂ together denote a restriction site,
SP individually denotes a spacer of at least two nucleotide bases,
PR denotes a promoter, capable of functioning in the cell,
X denotes an expressible nucleotide sequence,
TR denotes a terminator, and
wherein n ≥ 2,
wherein rs₁-rs₂ in at least two cassettes is recognised by the same restriction enzyme, and
said cell being capable of producing at least one substance, which is heterologous to the cell.

In another aspect the invention relates to an evolved cell comprising at least one artificial chromosome comprising at least a first and a second expressible nucleotide sequence under the control of a controllable promoter, the promoter of the first expressible nucleotide sequence being controllable independently from the promoter of the other expressible nucleotide sequence, said cell being capable of producing at least one substance, which is heterologous to the cell.

By the term substance is meant any substance produced by the cell, intracellularly as well as extracellularly located substances, such as primary and secondary metabolites, proteins, polypeptides, enzymes, carbohydrates, lipids, proteoglycans, poly- and oligosaccharides and ribonucleic acids.

Furthermore, the cell evolved according to the invention may also be defined in relation to a functionality of the cell, such as a cell comprising at least one concatemer of individual oligonucleotide cassettes, each concatemer individually comprising an oligonucleotide sequence of the following formula in 5'→3' direction:

[rs₂-SP-PR-X-TR-SP-rs₁]ₙ

wherein
rs₁ and rs₂ together denote a restriction site,
SP denotes a spacer of at least two nucleotide bases,
PR denotes a promoter, capable of functioning in the cell,
X denotes an expressible nucleotide sequence,
TR denotes a terminator, and
wherein n ≥ 2,
wherein rs₁-rs₂ in at least two cassettes is recognised by the same restriction enzyme, and
said cell being capable of metabolising at least one compound, which is not metabolisable by the native cell.

In another aspect the cell may be defined as a cell comprising at least one artificial chromosome comprising at least a first and a second expressible nucleotide sequence under the control of a controllable promoter, the promoter of the first expressible nucleotide sequence being controllable independently from the promoter of the other expressible nucleotide sequence, said cell being capable of metabolising at least one compound, which is not metabolisable by the native cell.

Due to the evolutionary pressure placed on the cells by means of subjecting the cells to a series of selection rounds or cycles, progressively selecting for the screening functionality, it is possible to evolve cells having the desired property or functionality.

### Detailed description of the drawings

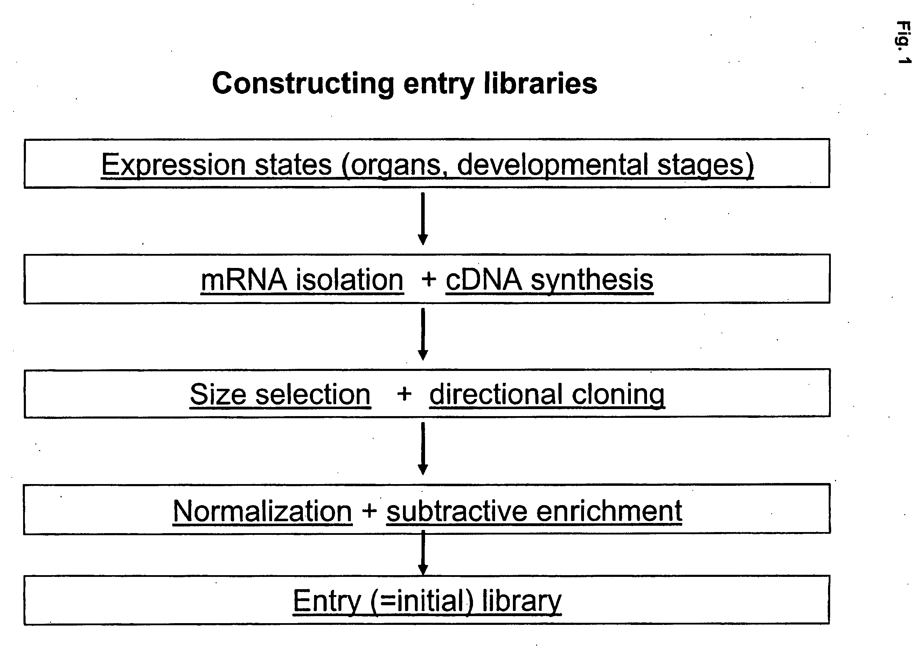

Fig. 1 shows a flow chart of the steps leading from an expression state to incorporation of the expressible nucleotide sequences in an entry library (a nucleotide library according to the invention).

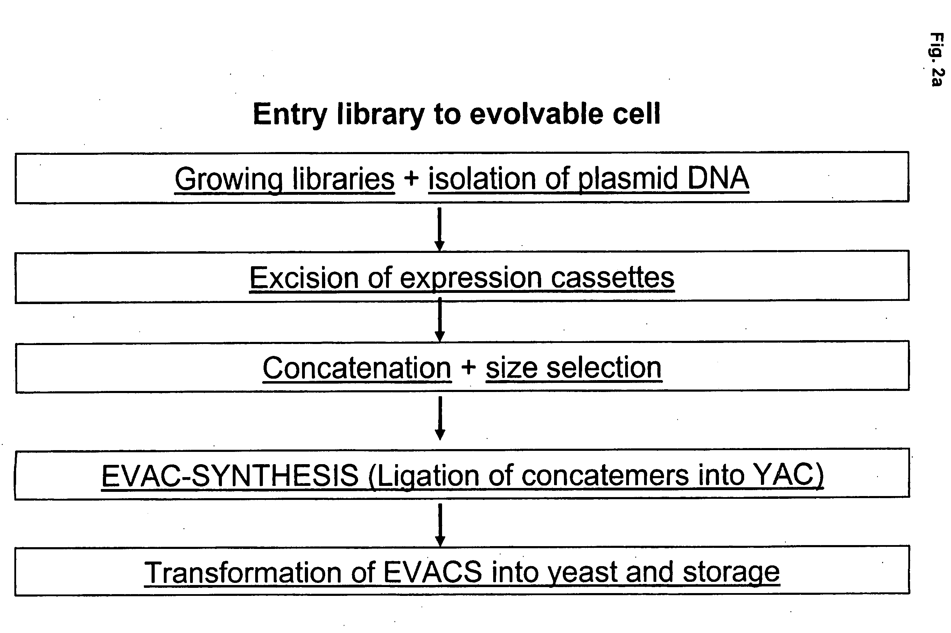

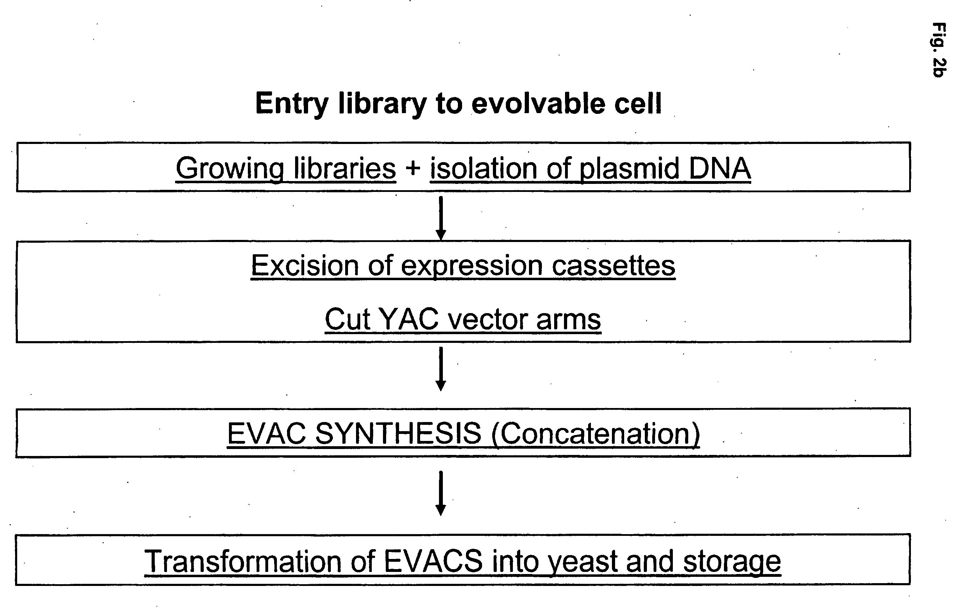

Fig. 2 shows a flow chart of the steps leading from an entry library comprising expressible nucleotide sequences to evolvable artificial chromosomes (EVAC) transformed into an appropriate host cell. Fig. 2a shows one way of producing the EVACs which includes concatenation, size selection and insertion into an artificial chromosome vector. Fig. 2b shows a one step procedure for concatenation and ligation of vector arms to obtain EVACs.

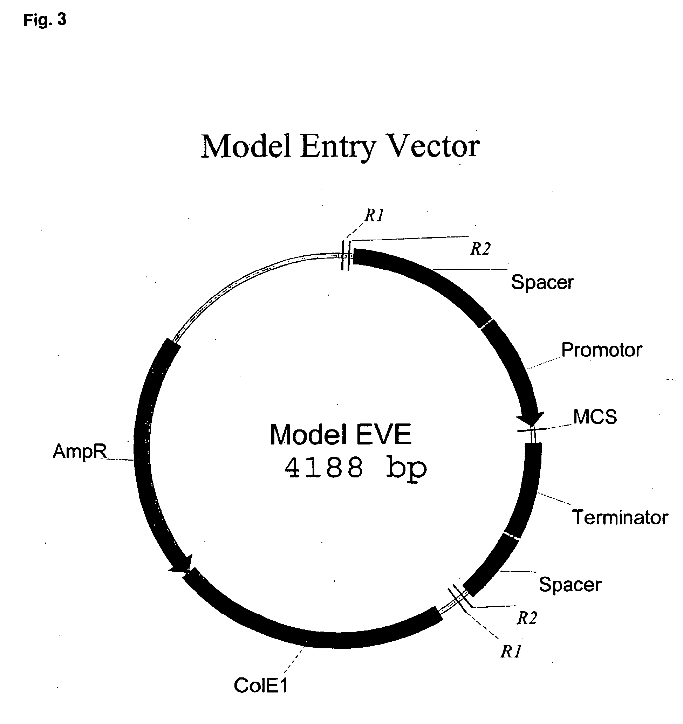

Fig. 3 shows a model entry vector. MCS is a multi cloning site for inserting expressible nucleotide sequences. Amp R is the gene for ampicillin resistance. Col E is the origin of replication in E. coli. R1 and R2 are restriction enzyme recognition sites.

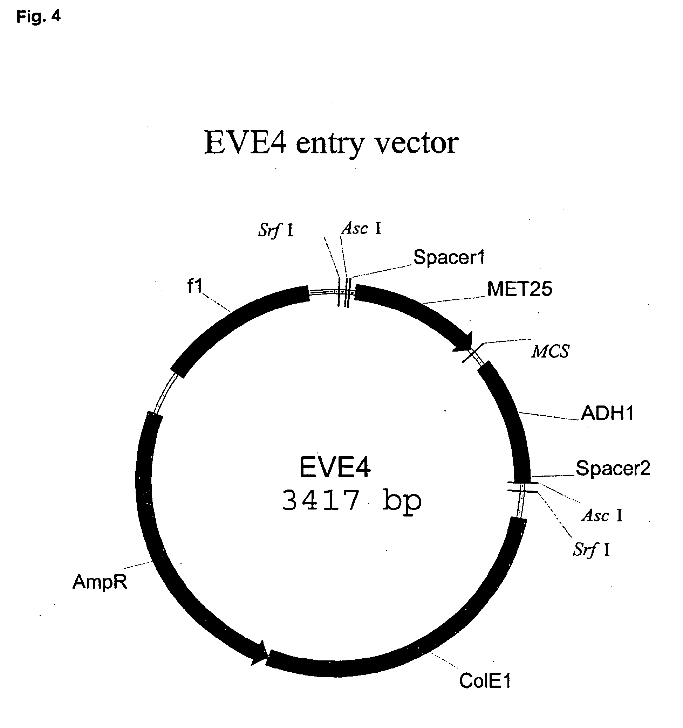

Fig. 4 shows an example of an entry vector according to the invention, EVE4. MET25 is a promoter, ADH1 is a terminator, f1 is an origin of replication for filamentous phages, e.g. M13. Spacer 1 and spacer 2 are constituted by a few nucleotides deriving from the multiple cloning site, MCS, Scfl and Ascl are restriction enzyme recognition sites. Other abbreviations, see Fig. 3. The sequence of the vector is set forth in SEQ ID NO 1.

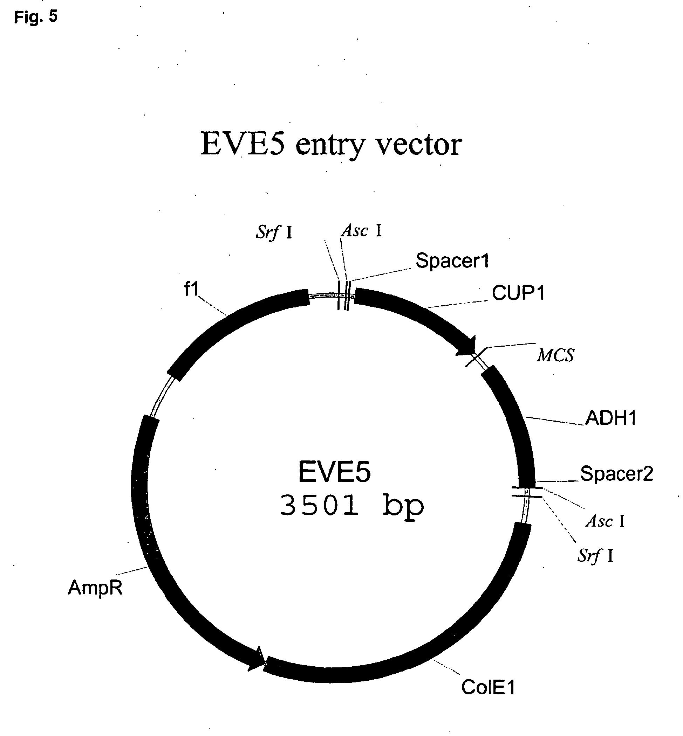

Fig 5 shows an example of an entry vector according to the invention, EVE5. CUP1 is a promoter, ADH1 is a terminator, f1 is an origin of replication for filamentous phages, e.g. M13. Spacer 1 and spacer 2 are constituted by a few nucleotides deriving from the multiple cloning site, MCS, Scfl and Ascl are restriction enzyme recognition sites. Other abbreviations, see Fig. 3. The sequence of the vector is set forth in SEQ ID NO 2.

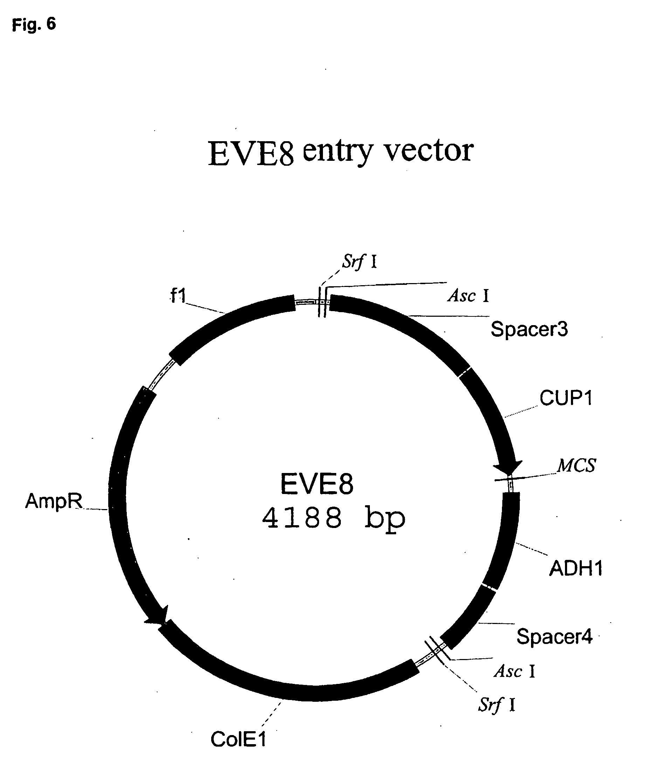

Fig 6 shows an example of an entry vector according to the invention, EVE8. CUP1 is a promoter, ADH1 is a terminator, f1 is an origin of replication for filamentous phages, e.g. M13. Spacer3 is a 550 bp fragment of lambda phage DNA fragment. Spacer4 is a ARS1 sequence from yeast. Scfl and Ascl are restriction enzyme recognition sites. Other abbreviations, see Fig. 3. The sequence of the vector is set forth in SEQ ID NO 3.

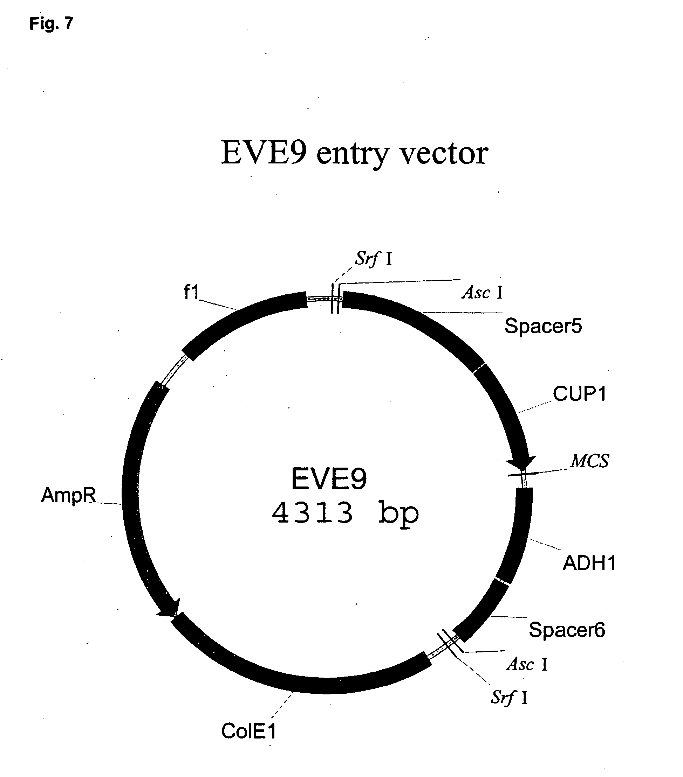

Fig. 7 shows an example of an entry vector according to the invention, EVE9. Met25 is a promoter, ADH1 is a terminator. Spacer 5 and 6 are lambda phage DNA. SEQ ID NO 5.

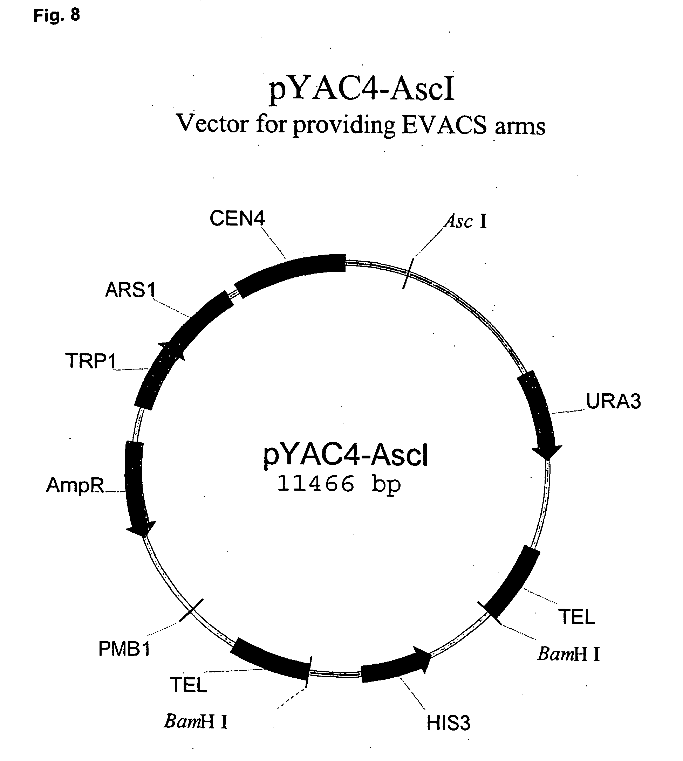

Fig. 8 shows a vector (pYAC4-Ascl) for providing arms for an evolvable artificial chromosome (EVAC) into which a concatemer according to the invention can be cloned. TRP1, URA3, and HIS3 are yeast auxotrophic marker genes, and AmpR is an E. coli antibiotic marker gene. CEN4 is a centromere and TEL are telomeres. ARS1 and PMB1 allow replication in yeast and E. coli respectively. BamH I and Asc I are restriction enzyme recognition sites. The nucleotide sequence of the vector is set forth in SEQ ID NO 4.

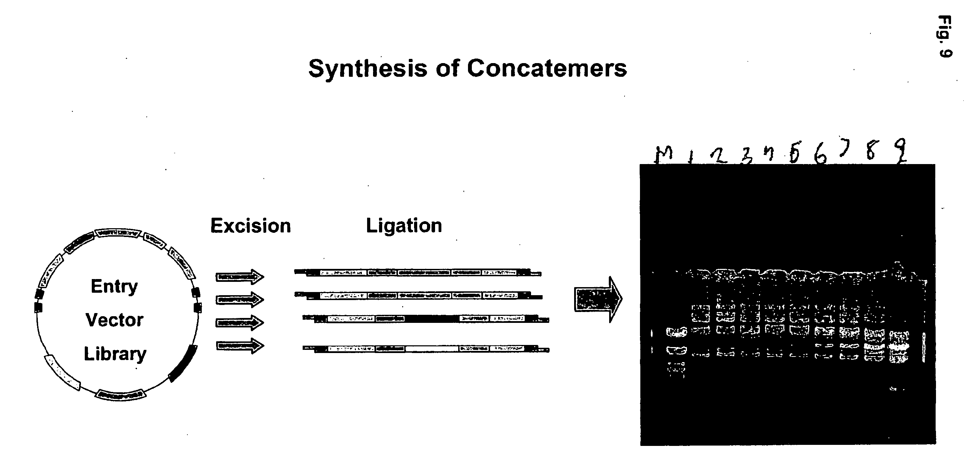

Fig. 9. shows the general concatenation strategy. On the left is shown a circular entry vector with restriction sites, spacers, promoter, expressible nucleotide sequence and terminator. These are excised and ligated randomly.

| Lane | F/Y |
|---|---|
| 1 | 100/1 |
| 2 | 50/1 |
| 3 | 20/1 |
| 4 | 10/1 |
| 5 | 5/1 |
| 6 | 2/1 |
| 7 | 1/1 |
| 8 | 1/2 |
| 9 | 1/5 |

| | |
|---|---|
| Legend: Lane M: molecular weight marker, λ-phage DNA digested w. Pst1. Lanes 1-9, concatenation reactions. Ratio of fragments to yac-arms(F/Y) as in table. | |

Fig 10a and 10b. illustrates the integration of concatenation with synthesis of evolvable artificial chromosomes and how concatemer size can be controlled by controlling the ratio of vector arms to expression cassettes, as described in example 7.

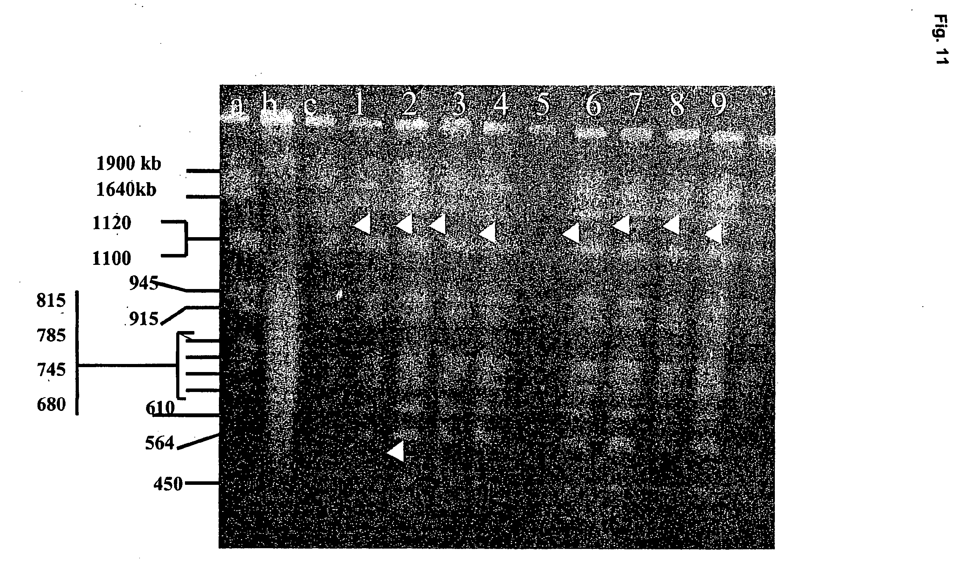

Fig 11. EVAC gel Legend: PFGE of EVAC containing clones : Lanes. a: Yeast DNA PFGE markers(strain YNN295), b: lambda ladder, c: non-transformed host yeast, 1 - 9 : EVAC containing clones. EVACs in size range 1400-1600 kb. Lane 2 shows a clone containing 2 EVACs sized ∼1500 kb and -550 kb respectively. The 550kb EVAC is comigrating with the 564kb yeast chromosome and is resulting in an increased intensity of the band at 564 kb relative to the other bands in the lane. Arrows point up to EVAC bands.

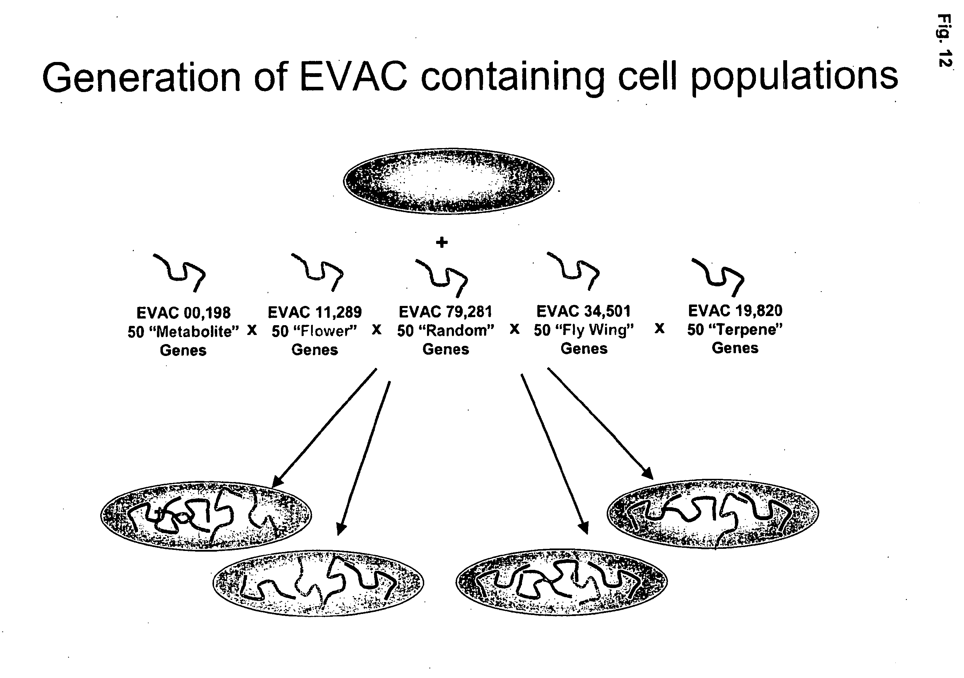

Fig. 12 shows an example of generation of an EVAC containing cell population. EVACs (Evolvable Artificial Chromosome) are artificial chromosomes composed of concatemers of expression cassettes containing heterologous DNA, so that each gene is under the control of an externally controllable promoter. Large numbers of heterologous genes from multiple sources can thus be combined in a single host cell.
Fig. 13 shows the general principle for screening EVAC containing cell populations. amplified. The cell population is amplified and subjected to a panel of screens that are relevant to a desired functionality. Positive subpopulations are selected.
Fig. 14 shows how cell populations evolve through a tiered set of selection conditions, always taking the best performing cell populations further in the process until an optimal functionality/property is evolved.

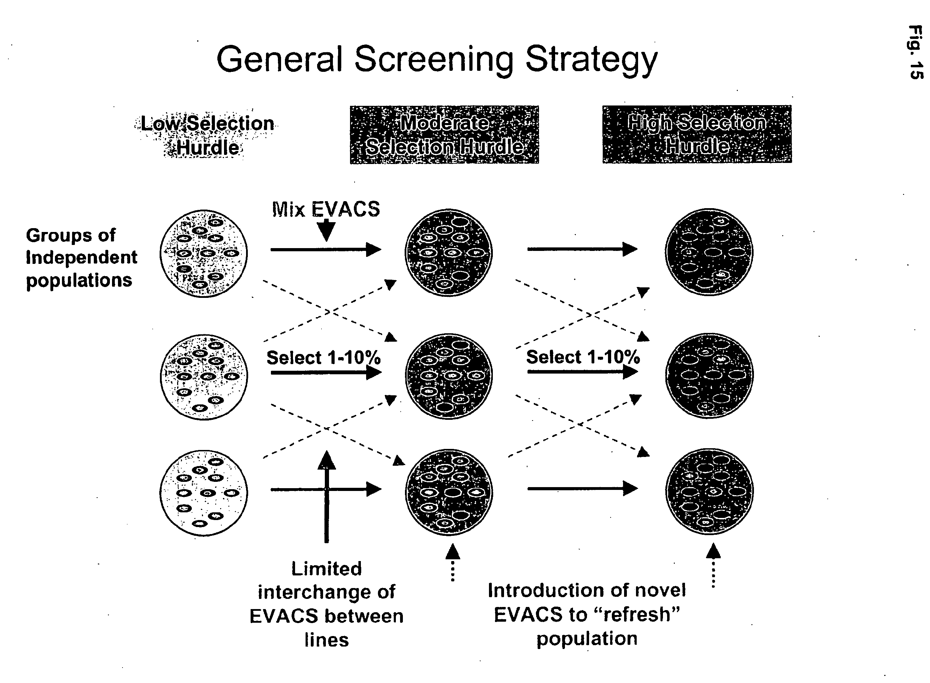

Fig. 15 shows a general screening strategy. Independent populations are subjected to the same set of screens, and genetic material from the different selected subpopulations is combined together with novel genetic diversity introduced between selection rounds.

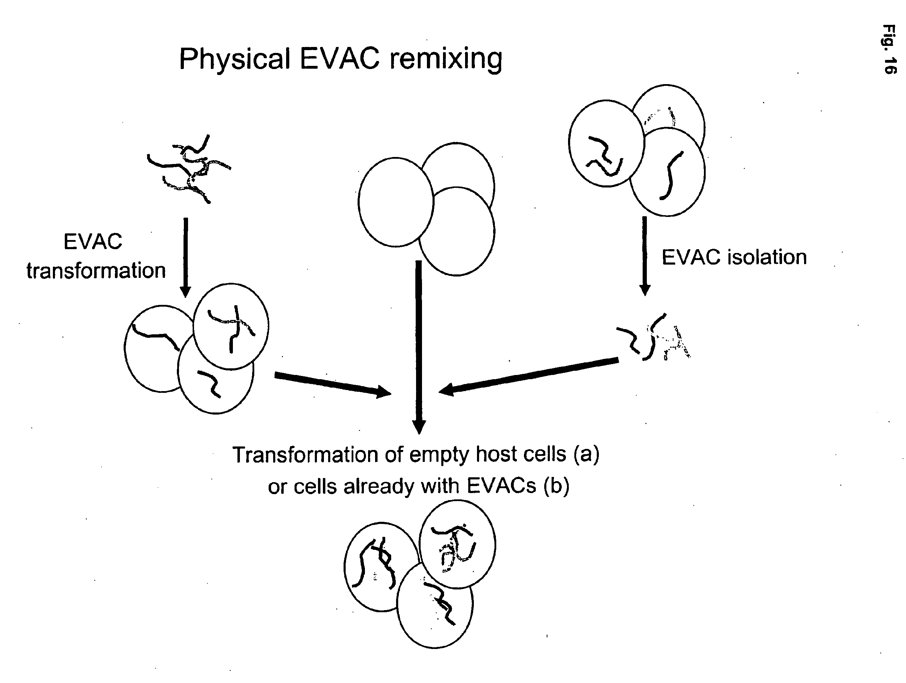

Fig. 16 shows physical remixing of EVACs. EVACs are isolated from the host and used for transformation of either empty host cells or for transformation of host cells already containing EVACS to obtain new combinations of EVACs in each host cell.

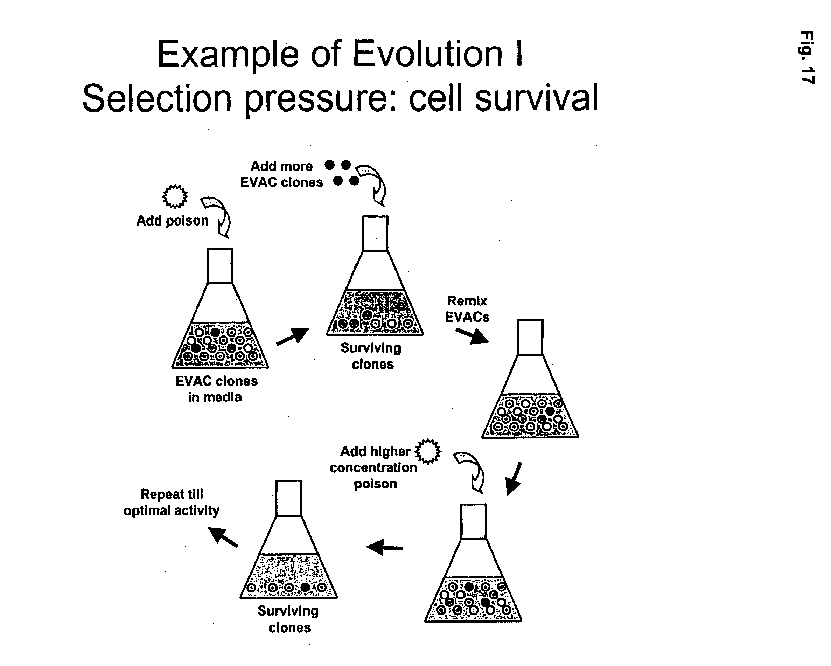

Fig. 17 shows one example of evolution. Cells that are resistant to a poison may be selected in liquid media. The surviving cells are cells containing EVACs that result in the production of compounds that prevent the poison from interacting with its target.

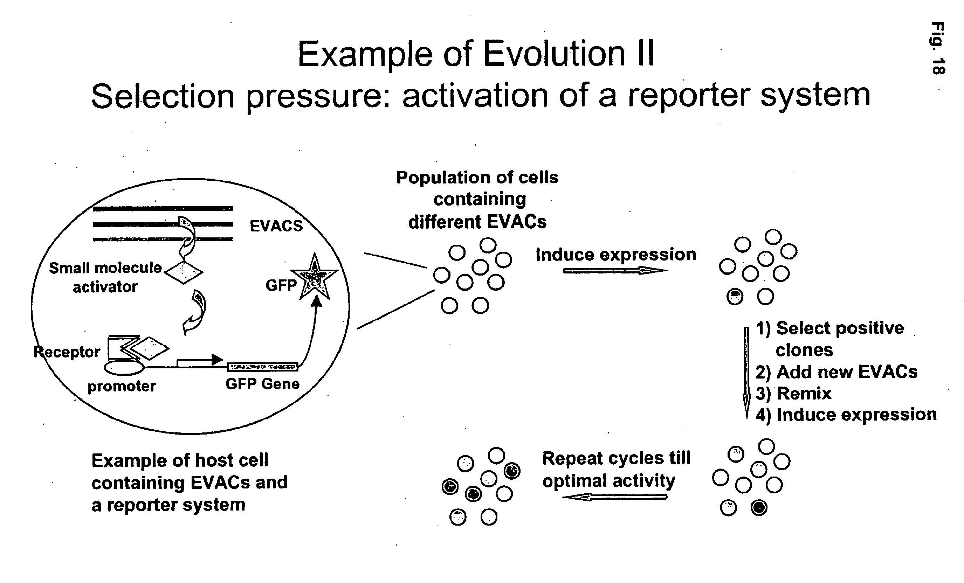

Fig. 18 shows how an evolution programme based on a screen for compounds that activate (or prevents) activation of a reporter system may be designed. Using the appropriate marker (e.g. GFP) positive clones can be selected using e.g. flow cytometry.

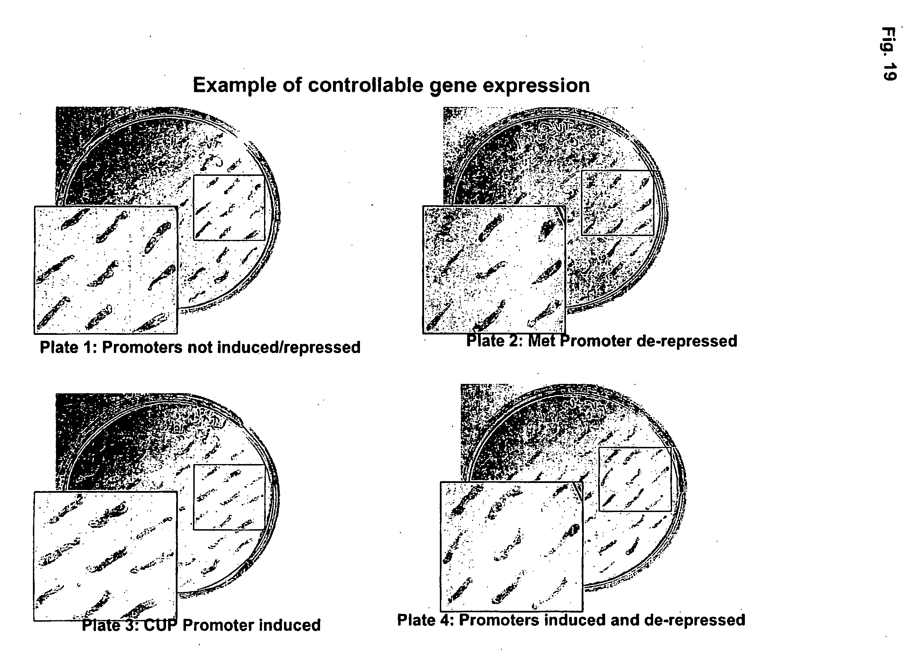

Fig. 19 shows an example of controllable gene expression in a cell population containing EVACs enriched in genes that code for carotenoid synthetic enzymes. The expression cassettes contain either a Met 25 or a CUP I promoter. Orange and red colonies ar obtained as a function of the promoter activation. Intensity of colour and number of coloured colonies increases in the following order: CUP + Met > CUP > Met. Uninduced colonies are white.

### Detailed Description of the Invention

The present invention relates to methods of evolving cells that produce novel substances and/or metabolic pathways. The evolution may lead to the production of novel molecules with various levels of functional characterisation and/or optimised molecules as well as production at various scales of compounds of commercial value, such as pharmaceuticals, cosmetics, flavours, other food and animal feed ingredients, agricultural chemicals, colouring agents, diagnostic markers, industrial chemials and intermediates for industrial purposes.

Thus, by "Evolution of a cell" is meant change of a cells phenotype towards a novel phenotype due to expression of a novel combination of genes. By "evolution of a composition" is meant change of the properties of a composition due to a novel combination of cells expressing a novel combination of genes.

### Evolution and Fitness

Evolution at its most general is a process, whereby a set of replicating and varying patterns are subjected to a selection process that favours the replication of certain of the variant patterns. The selection process acts on an emergent property (phenotype) that is encoded by the pattern and that varies as a consequence of the underlying variation in the pattern. Over the course of a series of replication events those patterns whose replication is most favoured come to dominate the population. Variation in the patterns occurs as the result of changes in individual patterns or as the result of mixing of individual patterns. Which patterns come to dominate the population is partly a consequence of the selection criteria used and partly a function of the starting population.

In living organisms and cells the predominant replicating pattern consists of nucleotide sequences (DNA or - in some vira - RNA) and the criteria on which selection acts it typically mediated through other molecules such as (but not limited to) proteins, metabolites, and structural macromolecules that are encoded by the nucleotide sequence either directly or indirectly.

In genetic algorithms the replicating pattern consists of software defined magnetic states and the variation on which selection acts is typically (but not limited to) the solution of a mathematical algorithm encoded by the magnetic states either directly or indirectly.

The ability of a pattern to replicate in a given set of environmental parameters is often referred to as the "fitness" of the pattern. Fitness can be regarded as a mathematical property that replicating patterns "attempt to" optimise. The higher the fitness of any given pattern, the greater the chance it will produce one or more copies of itself, the higher the number of copies it will on average produce, and the lower the chance it will be destroyed prior to replication. As with any mathematical function the property that is optimised may itself be a complex function of otherwise independent properties. Thus evolution can optimise across more than one criteria. For instance the mating calls of many male insects are optimised to attract females of the same species whilst not attracting predators. The oxygen binding proteins in whale blood are optimised to bind oxygen under one set of conditions and release it under another set of conditions.

Cells containing genetic material are thus in principle able to evolve by virtue of the variations in the genetic sequence that occur within each cell and the consquences of this variation upon the fitness of the cell in a given set of environmental parameters and the ability of the cell to pass these genetic sequences on to descendant cells

For the purposes of this invention the term "Fitness Function" shall be taken to mean a mathemetical or algebraic equation that calculates a score and where the variable elements in the equation are output variables that vary between different cells within a *cell population.*

For the purposes of this invention the term "Fitness Score" shall be the score generated by the *fitness function* equation.

It shall be understood that any selection process conducted on cells may therefore be conducted according to the following general procedure:
- The fitness function (F') is defined so that it encapsulates the desired phenotype of the cell and mathematically relates this to measurable parameters
- Each cell or group of cells is measured on one or more parameters
- F' for the cell is calculated according to the measured parameters
- Those cells with the highest F' scores are removed from the screening locality and allowed to grow. Cells with lower F' scores are discarded. By the highest F' score is meant a predetermined percentage of the cells with the highest score, such as the best 1%, 5%, 10 % or 50%, or for very high selection pressures the best 1‰, the best 0.1 ‰, the best 0.01 %o, the best 0.001 %o, or the best 0.0001‰.

It is an important teaching of evolution that the criteria on which certain patterns are selected over other patterns is essentially arbitrary - in principle any criterion can be used. That arbitrary, human imposed criteria can be used to generate an evolutionary process in a whole organism is exemplified by the evolution of melanism in moths as a result of industrialisation, the evolution of pedigree dogs with various properties and the evolution of plants with e.g. enhanced levels of commercially valuable oils or more even fruiting times or more attractive scents and colours. The term "breeding" is often used to describe human imposed evolution. Such organisms have increased their fitness according to a given set of human imposed criteria. It shall be obvious from the these examples that it is not necessary for the fitness function equation to be explicitly described for the evolution to take place.

It is a further teaching that fitness functions and consequent selection pressures can lead to the organism expressing phenotypes that impose high costs on (and even in some cases kill) the organism. All that is required for this to be the case is that they confer a countervailing benefit that allows the underlying pattern that produces the phenotype to spread. One example is the evolution of the peacock's tail, which whilst making it highly visible and vulnerable to competitors and predators, improves its ability to attract mates and hence replicate. In organisms with diploid or higher ploidy and with sexual reproduction it is even possible for patterns that have a net cost to be maintained in the population at reasonable levels. One example of this is the maintenance of the sickle cell anaemia mutation in west african human populations. The heterozygote form of the mutation confers a benefit (by making the carrier more resistant to malaria) whilst the homozygote is costly (causing severe anaemia). The positive benefit of the heterozygote results in the underlying pattern being maintained in the population at a relatively high frequency.

It is a further teaching that multiple selection pressures, acting on a population at different locations and times help develop and maintain the variability of replicating patterns in the population.

It is a further teaching that if two identical selection pressures are applied to two independent but apparently identical populations then although such populations will each evolve similar phenotypes the genetic patterns that come to dominate the population (and that confer the evolved phenotype) may differ between the populations. An example of different genetic patterns conferring the same phenotype is streptomycin resistance in bacteria.

From the above it should be clear that organisms are capable of complex evolutionary responses to a wide range of environmental pressures.

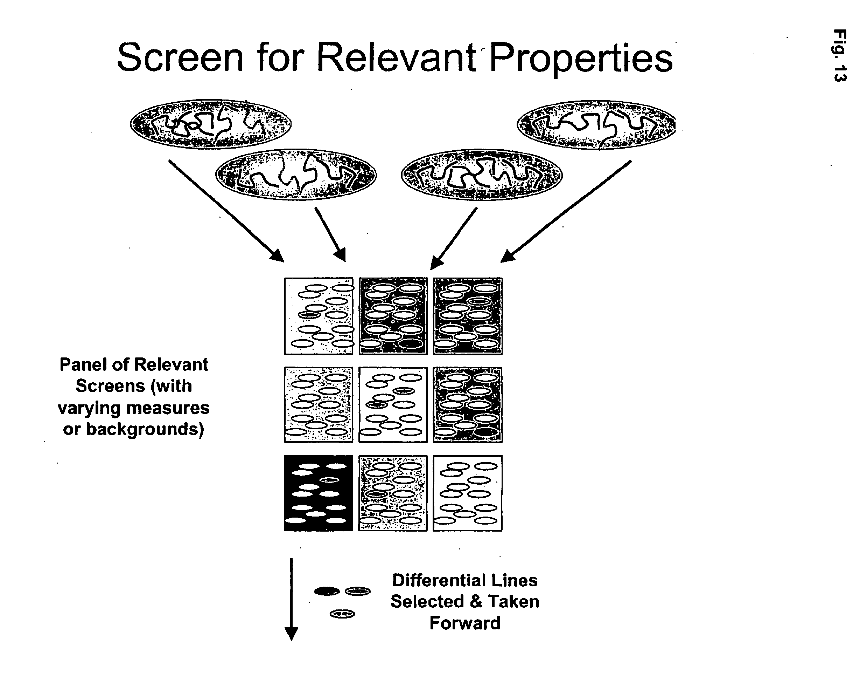

The evolution according to the present invention is based on a series or cycle of steps of subjecting a composition of cells to screening and selecting cells exhibiting a predetermined functionality, as shown in Fig. 13. The cycles are repeated until the desired functionality, for example a target specificity and activity is obtained.

In other words, the method of evolution according to the present invention is based on the provision of
1. a suitable set of diverse genetic patterns and also
2. a way of selecting for those genetic patterns within this set that encode for phenotypes that are consistent with these properties and also
3. a way of generating novel genetic patterns from those patterns that were selected in step 2.

These steps may then be combined sequentially or in parallel or in some other essentially iterative basis. The present invention sets out how to achieve these requirements.

In another aspect of the invention, the methods may be applied to the generation of a pathway derived from sources from multiple natural kingdoms, phyla or orders in the host cell. An example of this would be the generation of a pathway to produce retinoids or other molecules by means of introduction of genes encoding for the production of careotenoid pathways (obtained from fungi, algae and/or plants) as well as genes encoding for the synthesis of Vitamin A (obtained from mammals) or genes encoding for the production of visual pigments (obtained from insects). By such targeted selection and combination of elements of biochemical pathways across kingdoms or phyla the likelihood of obtaining novel metabolites may be further increased.

Examples of groups of species and individual species known to produce compounds with structural or functional utility include without limitation
- Bacteria: Streptomyces , Micromonospora, Norcadia, Actinomadura, Actinoplanes, Streptosporangium, Microbispora, Kitasatosporiam, Azobacterium, Rhizobium, Achromobacterium, Enterobacterium, Brucella, Micrococcus, Lactobacillus, Bacillus (B.t. toxins), Clostridium (toxins), Brevibacterium, Pseudomonas, Aerobacter, Vibrio, Halobacterium, Mycoplasma, Cytophaga, Myxococcus
- Fungi: Amanita muscaria (fly agaric, ibotenic acid, muscimol), Psilocybe (psilocybin) Physarium, Fuligo, Mucor, Phytophtora, Rhizopus, Aspergillus, Penicillium (penicillin), Coprinus, Phanerochaete, Acremonium (Cephalosporin), Trochoderma, Helminthosporium, Fusarium, Altemaria, Myrothecium, Saccharomyces
- Algae: Digenea simplex (kainic acid, antihelminthic), Laminaria anqustata (laminine, hypotensive)
- Lichens: Usnea fasciata (vulpinicacid, antimicrobial; usnic acid, antitumor)
- Higher Plants: Artemisia (artemisinin), Coleus (forskolin), Desmodium (K channel agonist), Catharanthus (Vinca alkaloids), Digitalis (cardiac glycosides), Podophyllum (podophyllotoxin), Taxus (taxol), Cephalotaxus (homoharringtonine), Camptotheca (Camptothecin), Camellia sinensis (Tea), Cannabis indica, Cannabis sativa (Hemp), Erythroxylum coca (Coca), Lophophora williamsii (PeyoteMyristica fragrans (Nutmeg), Nicotiana, Papaver somniferum (Opium Poppy), Phalaris arundinacea (Reed canary grass)
- Protozoa: Ptychodiscus brevis; Dinoflagellates (brevitoxin, cardiovascular)
- Sponges: Microciona prolifera (ectyonin, antimicrobial) Cryptotethya cryta (D-arabino furanosides)
- Coelenterata: Portuguese Man o War & other jellyfish and medusoid toxins.
- Corals: Pseudoterogonia species (Pseudoteracins, anti-inflammatory), Erythropodium (erythrolides, anti-inflammatory)
- Aschelminths: Nematode secretory compounds
- Molluscs: Conus toxins, sea slug toxins, cephalapod neurotransmitters, squid inks
- Annelida: Lumbriconereis heteropa (nereistoxin, insecticidal)
- Arachnids: Dolomedes ("fishing spider" venoms)
- Crustacea: Xenobalanus (skin adhesives)
- Insects: Epilachna (mexican bean beetle alkaloids)
- Spinunculida: Bonellia viridis (bonellin,neuroactive)
- Bryozoans: Bugula neritina (bryostatins,anti cancer)
- Echinoderms: Crinoid chemistry
- Tunicates: Trididemnum solidum (didemnin,anti-tumor and anti-viral; Ecteinascidia turbinata ecteinascidins, anti-tumor)
- Vertebrates: Eptatretus stoutii (eptatretin,cardioactive). Trachinus draco (proteinaceous toxins, reduce blood pressure, respiration and reduce heart rate). Dendrobatid frogs (batrachotoxins, pumiliotoxins, histrionicotoxins, and other polyamines); Snake venom toxins; Orinthorhynohus anatinus (duck-billed platypus venom), modified carotenoids, retinoids and steroids; Avians: histrionicotoxins, modified carotenoids, retinoids and steroids

### Diverse Genetic Patterns

Given that evolution is a statistical process it is necessary to provide sufficient genetic variation on which selection processes can act. In the present invention, this comprises two elements
- Providing a sufficiently large and diverse population
- Controlling the genetic basis of the diversity and how it expresses

Selection requires genetic diversity on which to operate. Thus the first requirement of the current invention is to provide a population of cells that embodies a genetic diversity. The term "*genetic diversity*" means that substantially all cells are different, in that they comprise different genes, and/or identical genes under control of different control system, such as different promoters, such that almost each cell initially represents a genotype not represented in any of the other cells. Of course due to cell division a few cells may be substantially identical.

The term "Cell Population" shall be taken to mean a population of cells where at least 10⁴ cells, such as at least 10⁵ cells, such as at least 10⁶ cells, such as at least 10⁷ cells, such as at least 10⁸ cells, such as at least 10⁹ cells, such as at least 10¹⁰ cells, such as at least 10¹¹ cells, such as at least 10¹² cells in the population represent a genotype not represented in any of the other cells.

Thus, the principle of the evolution method according to the invention is to obtain a population of cells having a very high genetic diversity.

One particular embodiment of this principle is to produce cells with combinations of concatemers comprising cassettes with expressible nucleotide sequences from a number of different expression states, which may be from any number of unrelated or distantly or closely related species, or from species from different kingdoms or phylae, novel and random combinations of gene products are produced in one single cell.

By inserting novel genes into the host cell, and especially by inserting a high number of novel genes from different expression states, such as from a wide variety of species into a host cell, the gene products from this array of novel genes will interact with the pool of metabolites of the host cell and with each other and modify known metabolites and/or intermediates in novel ways to create novel compounds. Due to the high number of substantially different cells that can be generated using the methods according to the present invention, for example at least 10⁴ cells, such as at least 10⁵ cells, such as at least 10⁶ cells, such as at least 10⁷ cells, such as at least 10⁸, such as at least 10⁹, for example at least 10¹⁰, such as at least 10¹², it is more or less inevitable or at least likely that such large populations will lead to a sub-population having such an interaction. The sub-population having such interaction may comprise at most 10¹⁰ cells, such as at most 10⁹ cells, such as at most 10⁸, such as at most 10⁷ cells, such as at most 10⁶ cells, such as at most 10⁵ cells, such as at most 10⁴ cells, such as at most 10³ cells, such as at most 10² cells or just 10 cells.

The host cells selected for this purpose are preferably cultivable under standard laboratory conditions using standard culture conditions, such as standard media and protocols. Preferably the host cells comprise a substantially stable cell line, in which the concatemers can be maintained for generations of cell division in a suitable manner. It is also of great advantage that standard techniques for transformation of the host cells are available, especially that methods are known for insertion of artificial chromosomes into the host cells.

It is also of advantage if the host cells are capable of undergoing meiosis to perform sexual recombination. It is also advantageous that meiosis is controllable through external manipulations of the cell culture. One especially advantageous host cell type is one where the cells can be manipulated through external manipulations into different mating types.

The host cell should preferably be conditionally deficient in the abilities to undergo homologous recombination. The host cell should preferably have a codon usage similar to that of the donor organisms. Furthermore, in the case of heterologous genomic DNA, if eukaryotic donor organisms are used, it is preferable that the host cell has the ability to process the donor messenger RNA properly, e.g., splice out introns.

The cells can be bacterial, archaebacteria, or eukaryotic and can constitute a homogeneous cell line or mixed culture. Suitable cells include the bacterial and eukaryotic cell lines commonly used in genetic engineering and protein expression. Suitable mammalian cells include those from, e.g., mouse, rat, hamster, primate, and human, both cell lines and primary cultures.

Preferred prokaryotic host organisms may include but are not limited to Escherichia coli, Bacillus subtilis, B licehniformis, B. cereus, Streptomyces lividans, Streptomyces coelicolor, Pseudomonas aeruginosa, Myxococcus xanthus. Rhodococcus, Streptomycetes, Actinomycetes, Corynebacteria, Bacillus, Pseudomonas, Salmonella, and Erwinia. The complete genome sequences of E. coli and Bacillus subtilis are described by Blattner et al., Science 277, 1454-1462 (1997); Kunst et al., Nature 390, 249-256 (1997)).

Preferred eukaryotic host organisms are mammals, fish, insects, plants, algae and fungi.

Examples of mammalian cells include those from, e.g., monkey, mouse, rat, hamster, primate, and human, both cell lines and primary cultures. Preferred mammalian host cells include but are not limited to those derived from humans, monkeys and rodents, such as chinese hamster ovary (CHO) cells, NIH/3T3, COS, 293, VERO, HeLa etc (see Kriegler M. in "Gene Transfer and Expression: A Laboratory Manual", New York, Freeman & Co. 1990), and stem cells, including embryonic stem cells and hemopoietic stem cells, zygotes, fibroblasts, lymphocytes, kidney, liver, muscle, and skin cells.

Examples of insect cells include baculo lepidoptera.

Examples of plant cells include maize, rice, wheat, cotton, soybean, and sugarcane. Plant cells such as those derived from Nicotiana and Arabidopsis are preferred

Examples of fungi include penicillium, aspergillus, such as Aspergillus nidulans, podospora, neurospora, such as Neurospora crassa, saccharomyces, such as Saccharomyces cerevisiae (budding yeast), Schizosaccharomyces, such as Schizosaccharomyces pombe (fission yeast), Pichia spp, such as Pichia pastoris, and Hansenula polymorpha (methylotropic yeasts).

The choice of host will depend on a number of factors, depending on the intended use of the engineered host, including pathogenicity, substrate range; environmental hardiness, presence of key intermediates, ease of genetic manipulation, and likelihood of promiscuous transfer of genetic information to other organisms. Particularly advantageous hosts are E. coli, lactobacilli, Streptomycetes, Actinomycetes and filamentous fungi.

A preferred host cell is yeast due to the following characteristics: it is fast growing, eukaryotic, allows scalable culture capabilities, genetic tools are available, it is metabolically flexible, can have a relatively permeable cell membrane/wall (yeast strains exist that are permable to most (>70%) of drug like molecules) and folds more heterologous eukaryotic proteins correctly than prokaryotic cells.

Thus, an illustrative and not limiting list of suitable yeast host cells comprise: baker's yeast, Kluyveromyces marxianus, K. lactis, Candida utilis, Phaffia rhodozyma, Saccharomyces boulardii, Pichia pastoris, Hansenula polymorpha, Yarrowia lipolytica, Candida paraffinica, Schwanniomyces castellii, Pichia stipitis, Candida shehatae, Rhodotorula glutinis, Lipomyces lipofer, Cryptococcos curvatus, Candida spp. (e.g. C. palmioleophila), Yarrowia lipolytica, Candida guilliermondii, Candida, Rhodotorula spp., Saccharomycopsis spp., Aureobasidium pullulans, Candida brumptii, Candida hydrocarbofumarica, Torulopsis, Candida tropicalis, Saccharomyces cerevisiae, Rhodotorula rubra, Candida flaveri, Eremothecium ashbyii, Pichia spp., Pichia pastoris, Kluyveromyces, Hansenula, Kloeckera, Pichia, Pachysolen spp., or Torulopsis bombicola.

In any one host cell it is possible to make all sorts of combinations of expressible nucleotide sequences from all possible sources. Furthermore, it is possible to make combinations of promoters and/or spacers and/or introns and/or terminators in combination with one and the same expressible nucleotide sequence.

In a preferred embodiment the cells to be evolved are produced by inserting concatemers comprising the multitude of cassettes into a host cell, in which the concatemers can be maintained and the expressible nucleotide sequences can be expressed in a co-ordinated way. The cassettes comprised in the concatemers may be cut out from the host cell and re-assembled due to their uniform structure with - preferably - compatible restriction sites between the cassettes.

The cells as defined in the present invention are preferably collected into populations for use in the present invention. The composition of cells subjected to evolution is then produced by selecting cells from a population or from several sub-populations. A population of individual cells is a population of expression constructs prepared from randomly assembled or even concatenated expressible nucleotide sequences derived from a plurality of species of donor organisms, in which expressible nucleotide sequences are operably associated with regulatory regions that drives expression of the expressible nucleotide sequences in an appropriate host cell. The host cells used are capable of producing functional gene products of the donor organisms. Upon expression in the host cell, gene products of the donor organism(s) may interact to form novel biochemical pathways.

The population according to this embodiment of the invention may in any one cell comprise a unique and preferably random combination of a high number of expression cassettes being heterologous to the host cells. Through this random combination of expression cassettes novel and unique combinations of gene products are obtained in each cell. Such populations are especially adapted in the discovery of novel metabolic pathways created through the non-native combinations of gene products.

In a preferred embodiment the population may be defined as a population comprising a collection of individual cells, the cells being denoted
cell₁, cell₂, ...., cellᵢ, wherein i ≥ 2,
each cell comprising at least one concatemer of individual oligonucleotide cassettes, each concatemer comprising a nucleotide sequence of the following formula:

   [rs₂-SP-PR-X-TR-SP-rs₁]ₙ

   wherein rs₁ and rs₂ together denote a restriction site, SP denotes a spacer of at least two bases, X denotes an expressible nucleotide sequence, PR denotes a promoter, capable of regulating the expression of X in the cell, TR denotes a terminator, and n ≥ 2, and
   wherein at least one concatemer of cell₁ is different from a concatemer of cell₂.

In the present context the nucleotide sequence of the formula [rs₂-SP-PR-X-TR-SP-rs₁]ₙ is also referred to as an expression cassette of the formula [rs₂-SP-PR-X-TR-SP-rs₁]ₙ.

Sub-populations may comprise cells as defined above for populations, but mostly the cells of a sub-population will have at least one trait in common, such as common promoter combinations, genetic material from a common species, a common phenotype or the like.

The function of the populations and sub-populations is to act as a source of diversity when obtaining the composition of cells to be evolved. Thus, in one embodiment the composition is a collection of subcompositions, wherein a subcomposition is a collection of individual cells having at least one phenotype in common. In a preferred embodiment the composition comprises at least 2 individual subcompositions, said subcompositions being different, such as at least 5 individual sub-compositions, such as at least 10 individual sub-compositions, wherein each sub-composition comprises at least 10 individual cells, such as at least 50 individual cells, such as at least 100 individual cells, such as at least 10³ individual cells, such as at least 10⁴ individual cells, such as at least 10⁵ individual cells, such as at least 10⁶ individual cells, such as at least 10⁷ individual cells, such as at least 10⁸ individual cells, such as at least 10⁹ individual cells.

The composition of cells preferably comprises at least 20 individual cells, such as at least 50 individual cells, such as at least 100 individual cells, such as at least 150 individual cells, such as at least 200 individual cells, such as at least 250 individual cells, such as at least 500 individual cells, such as at least 750 individual cells, such as at least 1000 individual cells, such as at least 10⁴ individual cells, such as at least 10⁵ individual cells, such as at least 10⁶ individual cells, such as at least 10⁷ individual cells, such as at least 10⁸ individual cells, such as at least 10⁹ individual cells.

In a preferred embodiment at least a majority of the individual cells have a genetic patterns or genotypes, thereby representing a great diversity.

The term "founding population" or a "founder populations" shall mean a Cell Population that has not itself been subjected to a selection round, in the present context also referred to as composition of cells. Optionally the expression constructs within the cell population are constructed such that genetic material from species that are known from prior art to produce compounds of a desired structure class, or compounds that have a desired functional effect, or are associated with a desired functional effect independent of knowledge of the compounds, predominate.

The term "daughter population" is a cell population having been subjected to at least one selection round. In the present context the daughter population is also referred to as a further modified composition.

### Controlling The Genetic Basis of the Diversity

### Sources of Genes

The natural world contains a significant amount of genetic diversity. Various authorities estimate that there are at least 10⁷ different species, and that each of these species contains on average at least 10⁴ genes. Even allowing for the fact that many of these genes are relatively conserved between species this represents a high level of genetic diversity.

One approach that can be envisaged for the purposes of the current invention is to source genetic material so as to maximise the taxonomic diversity of the genes obtained.

A second is to preferentially source genetic material from organisms that are known or reputed to produce molecules of the structural class or with the functional effects desired or are known or reputed to have a desired functional effect without the molecule being known, or are taxonomically related to any such organism.

A third approach is selection of genes of particular interest.

A fourth approach is to select genes that generally extend the host metabolic pathways.

Optionally these approaches can be combined in any suitable manner.

Genes can be sourced through the collection and processing of genetic material of various forms. The expressible nucleotide sequences that can be inserted into the vectors, concatemers, and cells according to this invention encompass any type of nucleotide such as RNA, DNA. Such a nucleotide sequence could be obtained e.g. from cDNA, which by its nature is expressible. But it is also possible to use sequences of genomic DNA, coding for specific genes. Preferably, the expressible nucleotide sequences correspond to full length genes such as substantially full length cDNA, but nucleotide sequences coding for shorter peptides than the original full length clones may also be used. Shorter peptides may still retain the catalytic activity of the native proteins. Thus, a preferred embodiment of this invention is to source and collect messenger transcripts (mRNA) for obtaining cDNA.

Another way to obtain expressible nucleotide sequences is through chemical synthesis of nucleotide sequences coding for known peptide or protein sequences. Thus the expressible DNA sequences does not have to be a naturally occurring sequence, although it may be preferable for practical purposes to primarily use naturally occurring nucleotide sequences. Whether the DNA is single or double stranded will depend on the vector system used.

By the term "Expression state" is meant a state of gene expression (i.e the mRNA transcript popuilation) in a specific cell, tissue, combination of tissues or organism or organisms of a given species as sampled at at any one time. Different expression states are found in different individuals, or in the same individual at different point in time, or in the same individual at different points its life-cycle or in the same individual under differing external conditions. The expression states of given cells or tissues of a given individual will also vary with respect to other cells or tissues of the same individual. Different expression states may also be obtained in the same organ or tissue in any one species or individual by exposing the tissues or organs to different environmental conditions comprising but not limited to changes in developmental stage, age, disease, infection, drought, humidity, salinity, exposure to xenobiotics, physiological effectors, temperature, pressure, pH, light, gaseous environment, chemicals such as toxin.

In the following the invention is described in the order in which the steps of obtaining a transformed host cell containing an evolvable artificial chromosome may be performed, starting with the entry vector.

In most cases the orientation with respect to the promoter of an expressible nucleotide sequence will be such that the coding strand is transcribed into a proper mRNA. It is however conceivable that the sequence may be reversed generating an antisense transcript in order to block expression of a specific gene.

Each cell of the *cell population* is initially produced by combining genes selected from at least one expression state. It is of course also possible from the onset to combine genes from two, three, four or more expression states in one host cell or to combine genes from different organisms in one cell. In some embodiments of the invention it is preferred to combine genes from a large variety of organisms into a single host in a manner so that each cell comprises
in the range of 10 to 1000 heterologous expressible nucleotide sequences, at least one of said sequences being incorporated into an artificial chromosome in the cell, and/or
at least two expression cassettes of the following formula:

[rs₂-SP-PR-X-TR-SP-rs₁].

A wide variety of combinations of expressible nucleotide sequences from all possible sources may occur in the cells. Furthermore, it is possible to make combinations of promoters and/or spacers and/or introns and/or terminators in combination with one and the same expressible nucleotide sequence.

Thus in any one cell there may preferably be expressible nucleotide sequences from two different expression states. Furthermore, these two different expression states may be from one species or advantageously from two different species. Any one host cell may also comprise expressible nucleotide sequences from at least three species, such as from at least four, five, six, seven, eight, nine or ten species, or from more than 15 species such as from more than 20 species, for example from more than 30, 40 or 50 species, such as from more than 100 different species, for example from more than 300 different species, such as from more than 500 different species, for example from more than 750 different species, thereby obtaining combinations of large numbers of expressible nucleotide sequences from a large number of species. In this way potentially unlimited numbers of combinations of expressible nucleotide sequences can be combined across different expression states. These different expression states may represent at least two different tissues, such as at least two organs, such as at least two species, such as at least two genera. The different species may be from at least two different phylae, such as from at least two different classes, such as from at least two different divisions, more preferably from at least two different sub-kingdoms, such as from at least two different kingdoms. Thus expressible nucleotide sequences may be combined from a eukaryote and a prokaryote into one and the same cell.

According to another embodiment of the invention, the expressible nucleotide sequences may be from one and the same expression state. The products of these sequences may interact with the products of the genes in the host cell and with each other and form new enzyme combinations leading to novel biochemical pathways.

### Controlling Gene Expression - Expression Cassettes

Genes primarily give rise to selectable phenotypes through transcription of the gene to RNA and translation of the RNA to protein. Furthermore phenotypes are often the result of interactions between multiple genes and their gene products

Thus it is an element of the current invention that the heterologous genes are provided in a format whereby their individual and collective expression (transcription to RNA) can be controlled.

It is likely that through the combination of a high number of non-native genes in a host cell combinations of genes or single genes are inserted that are lethal or sub-lethal to the host cell. Through the co-ordinated expression of the genes in the host cell it is possible not only to initiate the expression of any subset of genes but also to repress such expression, e.g. of lethal or sub-lethal genes.

Through external regulation of the promoters controlling the expressible nucleotides sequences novel and non-naturally occurring combinations of expressed genes can be obtained. Since these novel and non-natural combinations of gene products are found in one and the same cell, the heterologous gene products may affect the metabolism of the host cell in novel ways and thus cause it to produce novel primary or secondary metabolites and/or known metabolites in novel amounts and/or known metabolites in novel compartments of the cell or outside the cells. The novel metabolic pathways and/or novel or modified metabolites may be obtained without substantially recombining the introduced genes with a segment in the host genome or an episome of the host cells by as well as without intra- or extra concatemeric recombination.

By having expressible nucleotide sequences under the control of a number of independently inducible or repressible promoters, a large number of different expression states can be created inside one single cell by selectively turning on and off groups of the inserted expressible nucleotide sequences. The number of independently inducible and/or repressible promoters in one cell may vary from 1 to 10, such as 2, 3, 4, 5, 6, 7, 8, or 9, or even up to 15, 20, 25 or above 50 promoters.

In the evolution steps the functionality of the controllable promoters of the cells is used, since due to the controllable promoters it is possible during the screening and selection step to switch promoters on and off, thereby creating a greater diversity of expressed genes.

The term promoter is used with its normal meaning, i.e. a DNA sequence to which RNA polymerase binds and initiates transcription. The promoter determines the polarity of the transcript by specifying which strand will be transcribed.
- Bacterial promoters normally consist of -35 and -10 (relative to the transcriptional start) consensus sequences which are bound by a specific sigma factor and RNA polymerase.
- Eukaryotic promoters are more complex. Most promoters utilized in expression vectors are transcribed by RNA polymerase II. General transcription factors (GTFs) first bind specific sequences near the transcriptional start and then recruit the binding of RNA polymerase II. In addition to these minimal promoter elements, small sequence elements are recognized specifically by modular DNA-binding / trans-activating proteins (e.g. AP-1, SP-1) which regulate the activity of a given promoter.
- Viral promoters may serve the same function as bacterial and eukaryotic promoters. Upon viral infection of their host, viral promoters direct transcription either by using host transcriptional machinery or by supplying virally encoded enzymes to substitute part of the host machinery. Viral promoters are recognised by the transcriptional machinery of a large number of host organisms and are therefore often used in cloning and expression vectors.

Promoters may furthermore comprise regulatory elements, which are DNA sequence elements which act in conjunction with promoters and bind either repressors (e.g., lacO/ LAC Iq repressor system in E. coli) or inducers (e.g., gal1 /GAL4 inducer system in yeast). In either case, transcription is virtually "shut off' until the promoter is derepressed or induced, at which point transcription is "turned-on". The choice of promoter in the cassette is primarily dependent on the host organism into which the cassette is intended to be inserted. An important requirement to this end is that the promoter should preferably be capable of functioning in the host cell, in which the expressible nucleotide sequence is to be expressed.

Preferably the promoter is an externally controllable promoter, such as an inducible promoter and/or a repressible promoter. The promoter may be either controllable (repressible/inducible) by chemicals such as the absence/presence of chemical inducers, e.g. metabolites, substrates, metals, hormones, sugars. The promoter may likewise be controllable by certain physical parameters such as temperature, pH, redox status, growth stage, developmental stage, or the promoter may be inducible/repressible by a synthetic inducer/repressor such as the gal inducer.

In order to avoid unintentional interference with the gene regulation systems of the host cell, and in order to improve controllability of the co-ordinated gene expression the promoter is preferably a synthetic promoter. Suitable promoters are described in US 5,798,227, US 5,667,986. Principles for designing suitable synthetic eukaryotic promoters are disclosed in US 5,559,027, US 5,877,018 or US 6,072,050.

Synthetic inducible eukaryotic promoters for the regulation of transcription of a gene may achieve improved levels of protein expression and lower basal levels of gene expression. Such promoters preferably contain at least two different classes of regulatory elements, usually by modification of a native promoter containing one of the inducible elements by inserting the other of the inducible elements. For example, additional metal responsive elements IR:Es) and/or glucocorticoid responsive elements (GREs) may be provided to native promoters. Additionally, one or more constitutive elements may be functionally disabled to provide the lower basal levels of gene expression.

Preferred examples of promoters include but is not limited to those promoters being induced and/or repressed by any factor selected from the group comprising carbohydrates, e.g. galactose; low inorganic phosphase levels; temperature, e.g. low or high temperature shift; metals or metal ions, e.g. copper ions; hormones, e.g. dihydrotestosterone; deoxycorticosterone; heat shock (e.g. 39°C); methanol; redox-status; growth stage, e.g. developmental stage; synthetic inducers, e.g. gal inducer. Examples of such promoters include ADH 1, PGK 1, GAP 491, TPI, PYK, ENO, PMA 1, PHO5, GAL 1, GAL 2, GAL 10, MET25, ADH2, MEL 1, CUP 1, HSE, AOX, MOX, SV40, CaMV, Opaque-2, GRE, ARE, PGK/ARE hybrid, CYC/GRE hybrid, TPI/α2 operator, AOX 1, MOX A.

More preferably, however the promoter is selected from hybrid promoters such as PGK/ARE hybrid, CYC/GRE hybrid or from synthetic promoters. Such promoters can be controlled without interfering too much with the regulation of native genes in the expression host.

In the following, examples of known yeast promoters that may be used in conjunction with the present invention are shown. The examples are by no way limiting and only serve to indicate to the skilled practitioner how to select or design promoters that are useful according to the present invention.

Although numerous transcriptional promoters which are functional in yeasts have been described in the literature, only some of them have proved effective for the production of polypeptides by the recombinant route. There may be mentioned in particular the promoters of the PGK genes (3-phosphoglycerate kinase, TDH genes encoding GAPDH (Glyceraldehyde phosphate dehydrogenase), TEF1 genes (Elongation factor 1), MFα1 (α sex pheromone precursor) which are considered as strong constitutive promoters or alternatively the regulatable promoter CYCI which is repressed in the presence of glucose or PHO5 which can be regulated by thiamine. However, for reasons which are often unexplained, they do not always allow the effective expression of the genes which they control. In this context, it is always advantageous to be able to have new promoters in order to generate new effective host/vector systems. Furthermore, having a choice of effective promoters in a given cell also makes it possible to envisage the production of multiple proteins in this same cell (for example several enzymes of the same metabolic chain) while avoiding the problems of recombination between homologous sequences.

In general, a promoter region is situated in the 5' region of the genes and comprises all the elements allowing the transcription of a DNA fragment placed under their control, in particular:
(1) a so-called minimal promoter region comprising the TATA box and the site of initiation of transcription, which determines the position of the site of initiation as well as the basal level of transcription. In Saccharomyces cerevisiae, the length of the minimal promoter region is relatively variable. Indeed, the exact location of the TATA box varies from one gene to another and may be situated from -40 to - 120 nucleotides upstream of the site of the initiation (Chen and Struhl, 1985, EMBO J., 4, 3273-3280)
(2) sequences situated upstream of the TATA box (immediately upstream up to several hundreds of nucleotides) which make it possible to ensure an effective level of transcription either constitutively (relatively constant level of transcription all along the cell cycle, regardless of the conditions of culture) or in a regulatable manner (activation of transcription in the presence of an activator and/or repression in the presence of a repressor). These sequences, may be of several types: activator, inhibitor, enhancer, inducer, repressor and may respond to cellular factors or varied culture conditions.

Examples of such promoters are the ZZA1 and ZZA2 promoters disclosed in US 5,641,661, the EF1-α protein promoter and the ribosomal protein S7 gene promoter disclosed in WO 97/44470,, the COX 4 promoter and two unknown promoters (SEQ ID No: 1 and 2 in the document) disclosed in US 5,952,195. Other useful promoters include the HSP150 promoter disclosed in WO 98/54339 and the SV40 and RSV promoters disclosed in US 4,870,013 as well as the PyK and GAPDH promoters disclosed in EP 0 329 203 A1.

More preferably the invention employs the use of synthetic promoters. Synthetic promoters are often constructed by combining the minimal promoter region of one gene with the upstream regulating sequences of another gene. Enhanced promoter control may be obtained by modifying specific sequences in the upstream regulating sequences, e.g. through substitution or deletion or through inserting multiple copies of specific regulating sequences. One advantage of using synthetic promoters is that they can be controlled without interfering too much with the native promoters of the host cell.

One such synthetic yeast promoter comprises promoters or promoter elements of two different yeast-derived genes, yeast killer toxin leader peptide, and amino terminus of IL-1β (WO 98/54339).

Another example of a yeast synthetic promoter is disclosed in US 5,436,136 (Hinnen et al), which concerns a yeast hybrid promoter including a 5' upstream promoter element comprising upstream activation site(s) of the yeast PHO5 gene and a 3' downstream promoter element of the yeast GAPDH gene starting at nucleotide -300 to -180 and ending at nucleotide -1 of the GAPDH gene.

Another example of a yeast synthetic promoter is disclosed in US 5,089,398 (Rosenberg et al). This disclosure describes a promoter with the general formula -

(P.R.(2)-P.R.(1))-

wherein:
P.R.(1) is the promoter region proximal to the coding sequence and having the transcription initiation site, the RNA polymerase binding site, and including the TATA box, the CAAT sequence, as well as translational regulatory signals, e.g., capping sequence, as appropriate;
P.R.(2) is the promoter region joined to the 5'-end of P.R.(1) associated with enhancing the efficiency of transcription of the RNA polymerase binding region;

In US 4,945,046 (Horii et al) discloses a further example of how to design a synthetic yeast promoter. This specific promoter comprises promoter elements derived both from yeast and from a mammal. The hybrid promoter consists essentially of Saccharomyces cerevisiae PHO5 or GAP-DH promoter from which the upstream activation site (UAS) has been deleted and replaced by the early enhancer region derived from SV40 virus.

Co-ordinated expression of gene subsets can also be utilised to identify which heterologous genes are responsible for the production of a given phenotype.

In the following the sequence of steps to be taken when starting with the isolation of mRNA until insertion to an entry vector for providing the cells according to the invention is described. In short the sequence may include the following steps
i) isolating mRNA from an expression state,
ii) obtaining substantially full length cDNA clones corresponding to the mRNA sequences,
iii) inserting the substantially full length cDNA clones into a cloning site in a cassette in a primary vector, said cassette being of the general formula in 5'→3' direction:

   [RS1-RS2-SP-PR-CS-TR-SP-RS2'-RS1']

   wherein CS denotes a cloning site.

### Expression cassettes

The expression cassettes according to the present invention are preferably arranged as a cassette of nucleotides in a highly ordered sequence, the cassette having the general formula in 5'→3' direction:

[RS1-RS2-SP-PR-CS-TR-SP-RS2'-RS1']

wherein RS1 and RS1' denote restriction sites, RS2 and RS2' denote restriction sites different from RS1 and RS1', SP denotes a spacer sequence of at least two nucleotides, PR denotes a promoter, CS denotes a cloning site, and TR denotes a terminator, all of them being as discussed elsewhere in this specification.

It is an advantage to have two different restriction sites flanking both sides of the expression construct. By treating the primary vectors with restriction enzymes cleaving both restriction sites, the expression construct and the primary vector will be left with two non-compatible ends. This facilitates a concatenation process, since the empty vectors do not participate in the concatenation of expression constructs.

In principle, any restriction site, for which a restriction enzyme is known can be used. These include the restriction enzymes generally known and used in the field of molecular biology such as those described in Sambrook, Fritsch, Maniatis, "A laboratory Manual", 2nd edition. Cold Spring Habor Laboratory Press, 1989.

The restriction site recognition sequences preferably are of a substantial length, so that the likelihood of occurrence of an identical restriction site within the cassette is minimised. Thus the first restriction site may comprise at least 6 bases, but more preferably the recognition sequence comprises at least 7 or 8 bases. Restriction sites having 7 or more non N bases in the recognition sequence are generally known as "rare restriction sites" (see example 6). However, the recognition sequence may also be at least 10 bases, such as at least 15 bases, for example at least 16 bases, such as at least 17 bases, for example at least 18 bases, such as at least 18 bases, for example at least 19 bases, for example at least 20 bases, such as at least 21 bases, for example at least 22 bases, such as at least 23 bases, for example at least 25 bases, such as at least 30 bases, for example at least 35 bases, such as at least 40 bases, for example at least 45 bases, such as at least 50 bases. Preferably the first restriction site RS1 and RS1' is recognised by a restriction enzyme generating blunt ends of the double stranded nucleotide sequences. By generating blunt ends at this site, the risk that the vector participates in a subsequent concatenation is greatly reduced. The first restriction site may also give rise to sticky ends, but these are then preferably non-compatible to the sticky ends resulting from the second restriction site, RS2 and RS2'.

According to a preferred embodiment of the invention, the second restriction site, RS2 and RS2' comprises a rare restriction site. Thus, the longer the recognition sequence of the rare restriction site the more rare it is and the less likely is it that the restriction enzyme recognising it will cleave the nucleotide sequence at other - undesired - positions.

The rare restriction site may furthermore serve as a PCR priming site. Thereby it is possible to copy the cassettes via PCR techniques and thus indirectly "excise" the cassettes from a vector.

Single-stranded compatible ends may be created by digestion with restriction enzymes. For concatenation a preferred enzyme for excising the cassettes would be a rare cutter, i.e. an enzyme that recognises a sequence of 7 or more nucleotides. Examples of enzymes that cut very rarely are the meganucleases, many of which are intron encoded, like e.g. I-Ceu I, I-Sce I, I-Ppo I, and PI-Psp I (see eample 6d for more). Other preferred enzymes recognize a sequence of 8 nucleotides like e.g. Asc I, AsiS I, CciN I, CspB I, Fse I, MchA I, Not I, Pac I, Sbf I, Sda I, Sgf I, SgrA I, Sse232 I, and Sse8387 I, all of which create single stranded, palindromic compatible ends.

Other preferred rare cutters, which may also be used to control orientation of individual cassettes in the concatemer are enzymes that recognize non-palindromic sequences like e.g. Aar I, Sap I, Sfi I, Sdi I, and Vpa (see example 6c for more).

Alternatively, cassettes can be prepared by the addition of restriction sites to the ends, e.g. by PCR or ligation to linkers (short synthetic dsDNA molecules). Restriction enzymes are continuously being isolated and characterised and it is anticipated that many of such novel enzymes can be used to generate single-stranded compatible ends according to the present invention.

It is conceivable that single stranded compatible ends can be made by cleaving the vector with synthetic cutters. Thus, a reactive chemical group that will normally be able to cleave DNA unspecifically can cut at specific positions when coupled to another molecule that recognises and binds to specific sequences. Examples of molecules that recognise specific dsDNA sequences are DNA, PNA, LNA, phosphothioates, peptides, and amides. See e.g. Armitage, B.(1998) Chem. Rev. 98: 1171-1200, who describes photocleavage using e.g. anthraquinone and UV light; Dervan P.B. & Bürli R.W. (1999) Curr. Opin. Chem. Biol. 3: 688-93 describes the specific binding of polyamides to DNA; Nielsen, P.E. (2001) Curr. Opin. Biotechnol. 12: 16-20 describes the specific binding of PNA to DNA, and Chemical Reviews special thematic issue: RNA/DNA Cleavage (1998) vol. 98 (3) Bashkin J.K. (ed.) ACS publications, describes several examples of chemical DNA cleavers.

Single-stranded compatible ends may also be created e.g. by using PCR primers including dUTP and then treating the PCR product with Uracil-DNA glycosylase (Ref: US 5,035,996) to degrade part of the primer. Alternatively, compatible ends can be created by tailing both the vector and insert with complimentary nucleotides using Terminal Transferase (Chang, LMS, Bollum TJ (1971) J Biol Chem 246:909).

The spacer sequence located between the RS2 and the PR sequence is preferably a non-transcribed spacer sequence. The purpose of the spacer sequence(s) is to minimise recombination between different concatemers present in the same cell or between cassettes present in the same concatemer, but it may also serve the purpose of making the nucleotide sequences in the cassettes more "host" like. A further purpose of the spacer sequence is to reduce the occurrence of hairpin formation between adjacent palindromic sequences, which may occur when cassettes are assembled head to head or tail to tail. Spacer sequences may also be convenient for introducing short conserved nucleotide sequences that may serve e.g. as PCR primer sites or as target for hybridization to e.g. nucleic acid or PNA or LNA probes allowing affinity purification of cassettes.

The cassette may also optionally comprise another spacer sequence of at least two nucleotides between TR and RS2. When cassettes are cut out from a vector and concatenated into concatemers of cassettes, the spacer sequences together ensure that there is a certain distance between two successive identical promoter or terminator sequences. This distance may comprise at least 50 bases, such as at least 60 bases, for example at least 75 bases, such as at least 100 bases, for example at least 150 bases, such as at least 200 bases, for example at least 250 bases, such as at least 300 bases, for example at least 400 bases, for example at least 500 bases, such as at least 750 bases, for example at least 1000 bases, such as at least 1100 bases, for example at least 1200 bases, such as at least 1300 bases, for example at least 1400 bases, such as at least 1500 bases, for example at least 1600 bases, such as at least 1700 bases, for example at least 1800 bases, such as at least 1900 bases, for example at least 2000 bases, such as at least 2100 bases, for example at least 2200 bases, such as at least 2300 bases, for example at least 2400 bases, such as at least 2500 bases, for example at least 2600 bases, such as at least 2700 bases, for example at least 2800 bases, such as at least 2900 bases, for example at least 3000 bases, such as at least 3200 bases, for example at least 3500 bases, such as at least 3800 bases, for example at least 4000 bases, such as at least 4500 bases, for example at least 5000 bases, such as at least 6000 bases.

The number of the nucleotides between the spacer located 5' to the PR sequence and the one located 3' to the TR sequence may be any. However, it may be advantageous to ensure that at least one of the spacer sequences comprises between 100 and 2500 bases, preferably between 200 and 2300 bases, more preferably between 300 and 2100 bases, such as between 400 and 1900 bases, more preferably between 500 and 1700 bases, such as between 600 and 1500 bases, more preferably between 700 and 1400 bases.

If the intended host cell is yeast, the spacers present in a concatemer should perferably comprise a combination of a few ARSes with varying lambda phage DNA fragments.

Preferred examples of spacer sequences include but are not limited to: Lamda phage DNA, prokaryotic genomic DNA such as E. coli genomic DNA, ARSes.

The cloning site in the cassette in the primary vector should be designed so that any nucleotide sequence can be cloned into it.

The cloning site in the cassette preferably allows directional cloning. Hereby is ensured that transcription in a host cell is performed from the coding strand in the intended direction and that the translated peptide is identical to the peptide for which the original nucleotide sequence codes.

However according to some embodiments it may be advantageous to insert the sequence in opposite direction. According to these embodiments, so-called antisense constructs may be inserted which prevent functional expression of specific genes involved in specific pathways. Thereby it may become possible to divert metabolic intermediates from a prevalent pathway to another less dominant pathway.

The cloning site in the cassette may comprise multiple cloning sites, generally known as MCS or polylinker sites, which is a synthetic DNA sequence encoding a series of restriction endonuclease recognition sites. These sites are engineered for convenient cloning of DNA into a vector at a specific position and for directional cloning of the insert.

Cloning of cDNA does not have to involve the use of restriction enzymes. Other alternative systems include but are not limited to:
- Creator^{™} Cre-loxP system from Clontech, which uses recombination and loxP sites
- use of Lambda attachment sites (att-λ), such as the Gateway^{™} system from Life Technologies.
Both of these systems are directional.

The role of the terminator sequence is to limit transcription to the length of the coding sequence. An optimal terminator sequence is thus one, which is capable of performing this act in the host cell.

In prokaryotes, sequences known as transcriptional terminators signal the RNA polymerase to release the DNA template and stop transcription of the nascent RNA.

In eukaryotes, RNA molecules are transcribed well beyond the end of the mature mRNA molecule. New transcripts are enzymatically cleaved and modified by the addition of a long sequence of adenylic acid residues known as the poly-A tail. A polyadenylation consensus sequence is located about 10 to 30 bases upstream from the actual cleavage site.

Preferred examples of yeast derived terminator sequences include, but are not limited to: ADN1, CYC1, GPD, ADH1 alcohol dehydrogenase.

Depending on the nature of the host cell, it may be advantageous that at least one cassette comprises an intron between the promoter and the expressible nucleotide sequence, more preferable that substantially all cassettes comprise an intron between the promoter and the expressible nucleotide sequence. The choice of intron sequence depends on requirements of the host cell.

Thus, optionally the cassette in the vector comprises an intron sequence, which may be located 5' or 3' to the expressible nucleotide sequence. The design and layout of introns is well known in the art. The choice of intron design largely depends on the intended host cell, in which the expressible nucleotide sequence is eventually to be expressed. The effects of having intron sequence in the expression cassettes are those generally associated with intron sequences.

Examples of yeast introns can be found in the literature and in specific databases such as Ares Lab Yeast Intron Database (Version 2.1) as updated on 15 April 2000. Earlier versions of the database as well as extracts of the database have been published in: "Genome-wide bioinformatic and molecular analysis of introns in Saccharomyces cerevisiae." by Spingola M, Grate L, Haussler D, Ares M Jr. (RNA 1999 Feb;5(2):221-34) and "Test of intron predictions reveals novel splice sites, alternatively spliced mRNAs and new introns in meiotically regulated genes of yeast." by Davis CA, Grate L, Spingola M, Ares M Jr, (Nucleic Acids Res 2000 Apr 15;28(8):1700-6).

### Primary vectors (entry vectors)

By the term entry vector is meant a vector for storing and amplifying cDNA or other expressible nucleotide sequences using the cassettes according to the present invention. The entry vectors or primary vectors are preferably able to propagate in E. coli or any other suitable standard host cell. It should preferably be amplifiable and amenable to standard normalisation and enrichment procedures.

The entry vector may be of any type of DNA that has the basic requirements of a) being able to replicate itself in at least one suitable host organism and b) allows insertion of foreign DNA which is then replicated together with the vector and c) preferably allows selection of vector molecules that contain insertions of said foreign DNA. In a preferred embodiment the vector is able to replicate in standard hosts like yeasts, bacteria and it should preferably have a high copy number per host cell. It is also preferred that the vector in addition to a host specific origin of replication, contains an origin of replication for a single stranded virus, such as e.g. the f1 origin for filamentous phages. This will allow the production of single stranded nucleic acid which may be useful for normalisation and enrichment procedures of cloned sequences. A vast number of cloning vectors have been described which are commonly used and references may be given to e.g. Sambrook,J; Fritsch, E.F; and Maniatis T. (1989) Molecular Cloning: A laboratory manual. Cold Spring Harbour Laboratory Press, USA, Netherlands Culture Collection of Bacteria (www.cbs.knaw.nl/NCCB/collection.htm) or Department of Microbial Genetics, National Institute of Genetics, Yata 1111 Mishima Shizuoka 411-8540, Japan (www.shigen.nig.ac.jp/cvector/cvector.html). A few type-examples that are the parents of many popular derivatives are M13mp10, pUC18, Lambda gt 10, and pYAC4. Examples of primary vectors include but are not limited to M13K07, pBR322, pUC18, pUC19, pUC118, pUC119, pSP64, pSP65, pGEM-3, pGEM-3Z, pGEM-3Zf(-), pGEM-4, pGEM-4Z, πAN13, pBluescript II, CHARON 4A, λ⁺, CHARON 21A, CHARON 32, CHARON 33, CHARON 34, CHARON 35, CHARON 40, EMBL3A, λ2001, λDASH, λFIX, λgt10, λgt11, λgt18, λgt20, λgt22, λORF8, λZAP/R, pJB8, c2RB, pcos1EMBL

Methods for cloning of cDNA or genomic DNA into a vector are well known in the art. Reference may be given to J. Sambrook, E.F. Fritsch, T. Maniatis: Molecular Cloning, A Laboratory Manual (2nd edition, Cold Spring Harbor Laboratory Press, 1989).

One example of a circular model entry vector is described in Figure 3. The vector, EVE contains the expression cassette, R1-R2-Spacer-Promoter-Multi Cloning Site-Terminator-Spacer-R2-R1. The vector furthermore contains a gene for ampicillin resistance, AmpR, and an origin of replication for E.coli, ColE1.

The entry vectors EVE4, EVE5, and EVE8 shown in Figures 4, 5, and 6. These all contain Srfl as R1 and Ascl as R2. Both of these sites are palindromic and are regarded as rare restriction sites having 8 bases in the recognition sequence. The vectors furthermore contain the AmpR ampicillin resistance gene, and the ColE1 origin or replication for E.coli as well as f1, which is an origin of replication for filamentous phages, such as M13. EVE4 (Fig. 4) contains the MET25 promoter and the ADH1 terminator. Spacer 1 and spacer 2 are short sequences deriving from the multiple cloning site, MCS. EVE5 (Fig. 5) contains the CUP1 promoter and the ADH1 terminator. EVE8 (Fig. 6) contains the CUP1 promoter and the ADH1 terminator. The spacers of EVE8 are a 550 bp lambda phage DNA (spacer 3) and an ARS sequence from yeast (spacer 4).

### Nucleotide library (entry library)

Methods as well as suitable vectors and host cells for constructing and maintaining a library of nucleotide sequences in a cell are well known in the art. The primary requirement for the library is that is should be possible to store and amplify in it a number of primary vectors (constructs) according to this invention, the vectors (constructs) comprising expressible nucleotide sequences from at least one expression state and wherein at least two vectors (constructs) are different.

One specific example of such a library is the well known and widely employed cDNA libraries. The advantage of the cDNA library is mainly that it contains only DNA sequences corresponding to transcribed messenger RNA in a cell. Suitable methods are also present to purify the isolated mRNA or the synthesised cDNA so that only substantially full-length cDNA is cloned into the library.

Methods for optimisation of the process to yield substantially full length cDNA may comprise size selection, e.g. electrophoresis, chromatography, precipitation or may comprise ways of increasing the likelihood of getting full length cDNAs, e.g. the SMART^{™} method (Clonetech) or the CapTrap^{™} method (Stratagene).

Preferably the method for making the nucleotide library comprises obtaining a substantially full length cDNA population comprising a normalised representation of cDNA species. More preferably a substantially full length cDNA population comprises a normalised representation of cDNA species characteristic of a given expression state.

Normalisation reduces the redundancy of clones representing abundant mRNA species and increases the relative representation of clones from rare mRNA species.

Methods for normalisation of cDNA libraries are well known in the art. Reference may be given to suitable protocols for normalisation such as those described in US 5,763,239 (DIVERSA) and WO 95/08647 and WO 95/11986. and Bonaldo, Lennon, Soares, Genome Research 1996, 6:791-806; Ali, Holloway, Taylor, Plant Mol Biol Reporter, 2000, 18:123-132.

Enrichment methods are used to isolate clones representing mRNA which are characteristic of a particular expression state. A number of variations of the method broadly termed as subtractive hybrisation are known in the art. Reference may be given to Sive, John, Nucleic Acid Res, 1988, 16:10937; Diatchenko, Lau, Campbell et al, PNAS, 1996, 93:6025-6030; Carninci, Shibata, Hayatsu, Genome Res, 2000, 10:1617-30, Bonaldo, Lennon, Soares, Genome Research 1996, 6:791-806; Ali, Holloway, Taylor, Plant Mol Biol Reporter, 2000, 18:123-132. For example, enrichment may be achieved by doing additional rounds of hybridization similar to normalization procedures, using e.g. cDNA from a library of abundant clones or simply a library representing the uninduced state as a driver against a tester library from the induced state. Alternatively mRNA or PCR amplified cDNA derived from the expression state of choice can be used to subtract common sequences from a tester library. The choice of driver and tester population will depend on the nature of target expressible nucleotide sequences in each particular experiment.

Finally, enrichment may be achieved by subtractive hybridisation followed by colony picking.

In the library an expressible nucleotide sequence coding for one peptide is preferably found in different but similar vectors under the control of different promoters. Preferably the library comprises at least three primary vectors with an expressible nucleotide sequence coding for the same peptide under the control of three different promoters. More preferably the library comprises at least four primary vectors with an expressible nucleotide sequence coding for the same peptide under the control of four different promoters. More preferably the library comprises at least five primary vectors with an expressible nucleotide sequence coding for the same peptide under the control of five different promoters, such as comprises at lest six primary vectors with an expressible nucleotide sequence coding for the same peptide under the control of six different promoters, for example comprises at least seven primary vectors with an expressible nucleotide sequence coding for the same peptide under the control of seven different promoters, for example comprises at least eight primary vectors with an expressible nucleotide sequence coding for the same peptide under the control of eight different promoters, such as comprises at least nine primary vectors with an expressible nucleotide sequence coding for the same peptide under the control of nine different promoters, for example comprises at least ten primary vectors with an expressible nucleotide sequence coding for the same peptide under the control of ten different promoters.

The expressible nucleotide sequence coding for the same peptide preferably comprises essentially the same nucleotide sequence, more preferably the same nucleotide sequence.

By having a library with what may be termed one gene under the control of a number of different promoters in different vectors, it is possible to construct from the nucleotide library an array of combinations of genes and promoters. Preferably, one library comprises a complete or substantially complete combination such as a two dimensional array of genes and promoters, wherein substantially all genes are found under the control of substantially all of a selected number of promoters.

According to another embodiment of the invention the nucleotide library comprises combinations of expressible nucleotide sequences combined in different vectors with different spacer sequences and/or different intron sequences. Thus any one expressible nucleotide sequence may be combined in a two, three, four or five dimensional array with different promoters and/or different spacers and/or different introns and/or different terminators. The two, three, four or five dimensional array may be complete or incomplete, since not all combinations will have to be present.

The library may suitably be maintained in a host cell comprising prokaryotic cells or eukaryotic cells. Preferred prokaryotic host organisms may include but are not limited to Escherichia coli, Bacillus subtilis, Streptomyces lividans, Streptomyces coelicolor Pseudomonas aeruginosa, Myxococcus xanthus.

Yeast species such as Saccharomyces cerevisiae (budding yeast), Schizosaccharomyces pombe (fission yeast), Pichia pastoris, and Hansenula polymorpha (methylotropic yeasts) may also be used. Filamentous ascomycetes, such as Neurospora crassa and Aspergillus nidulans may also be used. Plant cells such as those derived from Nicotiana and Arabidopsis are preferred. Preferred mammalian host cells include but are not limited to those derived from humans, monkeys and rodents, such as chinese hamster ovary (CHO) cells, NIH/3T3, COS, 293, VERO, HeLa etc (see Kriegler M. in "Gene Transfer and Expression: A Laboratory Manual", New York, Freeman & Co. 1990).

### Concatemers

For the purposes of providing a method for assembling multiple expression cassettes ("cassettes") into a single host cell, and allowing their facile remixing between cells, the expression cassettes are assembled into concatemers.

A concatemer is a series of linked units. The concatemers according to the invention may comprise a selection of expressible nucleotide sequences from just one expression state and can thus be assembled from one library representing this expression state or it may comprise cassettes from a number of different expression states. The concatemers according to the invention are especially suitable for ligating into an artificial chromosome, which may be inserted into a host cell for coordinated expression. For this purpose, the variation among and between cassettes may be such as to minimise the chance of cross over as the host cell undergoes cell division such as through minimising the level of repeat sequences occurring in any one concatemer, since it is not an object of this embodiment of the invention to obtain recombination of concatemers with a segment in the host genome or an epitope of the host cells nor is it an object to obtain intra- or extra concatemeric recombination.

According to a preferred embodiment of the invention the concatemer comprises at least a first cassette and a second cassette, said first cassette being different from said second cassette. More preferably, the concatemer comprises cassettes, wherein substantially all cassettes are different. The difference between the cassettes may arise from differences between promoters, and/or expressible nucleotide sequences, and/or spacers, and/or introns and/or terminators.

The number of cassettes in a single concatemer is largely determined by the host species into which the concatemer is eventually to be inserted and the vector through which the insertion is carried out. The concatemer thus may comprise at least 10 cassettes, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, for example at least 2000 cassettes.

Each of the cassettes may be laid out as described above.

Thus, in a preferred embodiment a concatemer is used to denote a number of serially linked nucleotide cassettes, wherein at least two of the serially linked nucleotide units comprises a cassette having the basic structure

[rs₂-SP-PR-X-TR-SP-rs₁]

wherein
rs₁ and rs₂ together denote a restriction site,
SP denotes a spacer of at least two nucleotide bases,
PR denotes a promoter, capable of functioning in a cell,
X denotes an expressible nucleotide sequence,
TR denotes a terminator, and
SP denotes a spacer of at least two nucleotide bases.
wherein the variables of the cassette have the meaning as defined elsewhere in this specification. Optionally the cassettes comprise an intron sequence between the promoter and the expressible nucleotide sequence and/or between the terminator and the expressible nucleotide sequence as discussed above.

According to one aspect of the invention, a concatemer comprises cassettes with expressible nucleotide from different expression states, so that non-naturally occurring combinations or non-native combinations of expressible nucleotide sequences are obtained.

According to a preferred embodiment of the invention the concatemer comprises at least a first cassette and a second cassette, said first cassette being different from said second cassette. More preferably, the concatemer comprises cassettes, wherein substantially all cassettes are different. The difference between the cassettes may arise from differences between promoters, and/or expressible nucleotide sequences, and/or spacers, and/or terminators, and/or introns.

The concatenation may be carried out in different ways.

Cassettes to be concatenated are normally excised from a vector or they are synthesised through PCR. After excision the cassettes may be separated from the vector through size fractionation such as gel filtration or through tagging of known sequences in the cassettes. The isolated cassettes may then be ligated together either through interaction between sticky ends or through ligation of blunt ends.

More preferably the cassettes may be concatenated without an intervening purification step through excision from a vector with two restriction enzymes, one leaving sticky ends on the cassettes and the other one leaving blunt ends in the vectors.

An alternative way of producing concatemers free of vector sequences would be to PCR amplify the cassettes from a single stranded primary vector. The PCR product must include the restriction sites RS2 and RS2' which are subsequently cleaved by its cognate enzyme(s). Concatenation can then be performed using the digested PCR product, essentially without interference from the single stranded primary vector template or the small double stranded fragments, which have been cut form the ends.

When the vectors comprising the cassettes are single stranded, the cassettes may be excised and be made double stranded through PCR techniques, which only prime the cassette sequence and not the vector sequence. Sticky ends can be made by cleaving with a restriction enzyme leaving sticky ends and the cassettes can be assembled without interaction from the single stranded vector fragments.

The concatemer may be assembled or concatenated by concatenation of at least two cassettes of nucleotide sequences each cassette comprising a first sticky end, a spacer sequence, a promoter, an expressible nucleotide sequence, a terminator, and a second sticky end.

After concatenation has been completed, concatemers of the desired size may be selected through size selection, such as selection for concatemers having at least 10 cassettes, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, for example at least 2000 cassettes. The number of cassettes in each concatemer may be controlled by size fractionation after concatenation, since the size of the concatemers is approximately proportional to the number of cassettes.

Preferably at least one inserted concatemer in each cell comprises a selectable marker. Selectable markers generally provide a means to select, for growth, only those cells which contain a vector. Such markers are of two types: drug resistance and auxotrophic. A drug resistance marker enables cells to detoxify an exogenously added drug that would otherwise kill the cell. Auxotrophic markers allow cells grow in media lacking an essential component by enabling cells to synthesise the essential component (usually an amino acid).

Illustrative and non-limiting examples of common selectable markers with a brief description of their mode of action follow:

### Prokaryotic

- Ampicillin: interferes with a terminal reaction in bacterial cell wall synthesis. The resistance gene (bla) encodes beta-lactamase which cleaves the beta-lactam ring of the antibiotic thus detoxifying it.
- Tetracycline: prevents bacterial protein synthesis by binding to the 30S ribosomal subunit. The resistance gene (tet) specifies a protein that modifies the.bacterial membrane and prevents transport of the antibiotic into the cell.
- Kanamycin: binds to the 70S ribosomes and causes misreading of messenger RNA. The resistant gene (nptH) modifies the antibiotic and prevents interaction with the ribosome.
- Streptomycin: binds to the 30S ribosomal subunit, causing misreading of messenger RNA. The resistance gene (Sm) modifies the antibiotic and prevents interaction with the ribosome.
- Zeocin: this new bleomycin-family antibiotic intercalates into the DNA and cleaves it. The Zeocin resistance gene encodes a 13,665 dalton protein. This protein confers resistance to Zeocin by binding to the antibiotic and preventing it from binding DNA. Zeocin is effective on most aerobic cells and can be used for selection in mammalian cell lines, yeast, and bacteria.
- Auxotrophic markers.

### Eukaryotic

- Hygromycin: a aminocyclitol that inhibits protein synthesis by disrupting ribosome translocation and promoting mistranslation. The resistance gene (hph) detoxifies hygromycin -B- phosphorylation.
- Histidinol: cytotoxic to mammalian cells by inhibiting histidyl-tRNA synthesis in histidine free media. The resistance gene (hisD) product inactivates histidinol toxicity by converting it to the essential amino acid, histidine.
- Neomycin (G418): blocks protein synthesis by interfering with ribosomal functions. The resistance gene ADH encodes amino glycoside phosphotransferase which detoxifies G418.
- Uracil: Laboratory yeast strains carrying a mutated gene which encodes orotidine -5'- phosphate decarboxylase, an enzyme essential for uracil biosynthesis, are unable to grow in the absence of exogenous uracil. A copy of the wild-type gene (ura4+, S. pombe or URA3 S. cerevisiae) carried on the vector will complement this defect in transformed cells.
- Adenosine: Laboratory strains carrying a deficiency in adenosine synthesis maybe complemented by a vector carrying the wild type gene, ADE 2.
- Amino acids: Vectors carrying the wild-type genes for LEU2, TRP 1, HIS 3 or LYS 2 may be used to complement strains of yeast deficient in these genes.
- Zeocin: this new bleomycin-family antibiotic intercalates into the DNA and cleaves it. The Zeocin resistance gene encodes a 13,665 dalton protein. This protein confers resistance to Zeocin by binding to the antibiotic and preventing it from binding DNA. Zeocin is effective on most aerobic cells and can be used for selection in mammalian cell lines, yeast, and bacteria.

The number of concatemers in one single cell may be at least one concatemer per cell, preferably at least 2 concatemers per cell, more preferably 3 per cell, such as 4 per cell, more preferably 5 per cell, such as at least 5 per cell, for example at least 6 per cell, such as 7, 8, 9 or 10 per cell, for example more than 10 per cell. As described above, each concatemer may preferably comprise up to 1000 cassettes, and it is envisages that one concatemer may comprise up to 2000 cassettes. By inserting up to 10 concatemers into one single cell, this cell may thus be enriched with up to 20,000 new expressible genes, which under suitable conditions may be turned on and off by regulation of the regulatable promoters. However it may be more preferable to provide cells having anywhere between 10 and 1000 novel genes, such as 20-900 novel genes, for example 30 to 800 novel genes, such as 40 to 700 novel genes, for example 50 to 600 novel genes, such as from 60 to 300 novel genes. The genes may advantageously be located on 1 to 10 such as from 2 to 5 different concatemers in the cells. Each concatemer may advantageously comprise from 10 to 1000 genes, such as from 10 to 750 genes, such as from 10 to 500 genes, such as from 10 to 200 genes, such as from 20 to 100 genes, for example from 30 to 60 genes.

The concatemers may be inserted into the host cells according to any known transformation technique, preferably according to such transformation techniques that ensure stable and not transient transformation of the host cell. The concatemers may thus be inserted as an artificial chromosome which is replicated by the cells as they divide or they may be inserted into the chromosomes of the host cell. The concatemer may also be inserted in the form of a plasmid such as a plasmid vector, a phage vector, a viral vector, a cosmid vector, that is replicated by the cells as they divide. Any combination of the three insertion methods is also possible. One or more concatemers may thus be integrated into the chromosome(s) of the host cell and one or more concatemers may be inserted as plasmids or artificial chromosomes. One or more concatemers may be inserted as artificial chromosomes and one or more may be inserted into the same cell via a plasmid.

The basic requirements for a functional artificial chromosome have been described in US 4,464,472. An artificial chromosome or a functional minichromosome, as it may also be termed must comprise a DNA sequence capable of replication and stable mitotic maintenance in a host cell comprising a DNA segment coding for centromere-like activity during mitosis of said host and a DNA sequence coding for a replication site recognized by said host.

Suitable artificial chromosomes include a Yeast Artificial Chromosome (YAC) (see e.g. Murray et al, Nature 305:189-193; or US 4,464,472), a mega Yeast Artificial Chromosome (mega YAC), a Bacterial Artificial Chromosome (BAC), a mouse artificial chromosome, a Mammalian Artificial Chromosome (MAC) (see e.g. US 6,133,503 or US 6,077,697), an Insect Artificial Chromosome (BUGAC), an Avian Artificial Chromosome (AVAC), a Bacteriophage Artificial Chromosome, a Baculovirus Artificial Chromosome, a plant artificial chromosome (US 5,270,201), a BIBAC vector (US 5,977,439) or a Human Artificial Chromosome (HAC).

The artificial chromosome is preferably so large that the host cell perceives it as a "real" chromosome and maintains it and transmits it as a chromosome. For yeast and other suitable host species, this will often correspond approximately to the size of the smallest native chromosome in the species. For Saccharomyces, the smallest chromosome has a size of 225 Kb.

MACs may be used to construct artificial chromosomes from other species, such as insect and fish species. The artificial chromosomes preferably are fully functional stable chromosomes. Two types of artificial chromosomes may be used. One type, referred to as SATACs [satellite artificial chromosomes] are stable heterochromatic chromosomes, and the other type are minichromosomes based on amplification of euchromatin.

Mammalian artificial chromosomes provide extra-genomic specific integration sites for introduction of genes encoding proteins of interest and permit megabase size DNA integration, such as integration of concatemers according to the invention.

According to another embodiment of the invention, the concatemer may be integrated into the host chromosomes or cloned into other types of vectors, such as a plasmid vector, a phage vector, a viral vector or a cosmid vector.

A preferable artificial chromosome vector is one that is capable of being conditionally amplified in the host cell, e.g. in yeast. The amplification preferably is at least a 10 fold amplification. Furthermore, it is advantageous that the cloning site of the artificial chromosome vector can be modified to comprise the same restriction site as the one bordering the cassettes described above, i.e. RS2 and/or RS2'.

It is also conceivable that recombination can be used to generate concatemers, e.g. through the modification of techniques like the Creator system (Clontech) which uses the Cre-loxP mechanism (ref: Sauer B 1993 Methods Enzymol 225:890-900) to directionally join DNA molecules by recombination or like the Gateway system (Life Technologies, US 5,888,732) using lambda *att* attachment sites for directional recombination (Landy A 1989, Ann Rev Biochem 58:913). It is envisaged that also lambda cos site dependent systems can be developed to allow concatenation.

The concatemer may be assembled or concatenated by concatenation of at least two cassettes of nucleotide sequences each cassette comprising a first sticky end, a spacer sequence, a promoter, an expressible nucleotide sequence, a terminator, and a second sticky end. A flow chart of the procedure is shown in figure 2a.

Preferably concatenation further comprises
starting from a primary vector [RS1-RS2-SP-PR-X-TR-SP-RS2'-RS1'],
wherein X denotes an expressible nucleotide sequence,
RS1 and RS1' denote restriction sites,
RS2 and RS2' denote restriction sites different from RS1 and RS1',
SP denotes a spacer sequence of at least two nucleotides,
PR denotes a promoter,
TR denotes a terminator,

i) cutting the primary vector with the aid of at least one restriction enzyme specific for RS2 and RS2' obtaining cassettes having the general formula [rs₂-SP-PR-X-TR-SP-rs₁] wherein rs₁ and rs₂ together denote a functional restriction site RS2 or RS2',
ii) assembling the cut out cassettes through interaction between rs₁ and rs₂.

According to an especially preferred embodiment, vector arms each having a RS2 or RS2' in one end and a non-complementary overhang or a blunt end in the other end are added to the concatenation mixture together with the cassettes described above to further simplify the procedure (see Fig. 2b). One example of a suitable vector for providing vector arms is disclosed in Fig. 7 TRP1, URA3, and HIS3 are auxotrophic marker genes, and AmpR is an antibiotic marker gene. CEN4 is a centromer and TEL are telomeres. ARS1 and PMB1 allow replication in yeast and E. coli respectively. BamH I and Asc I are restriction enzyme recognition sites. The nucleotide sequence of the vector is set forth in SEQ ID NO 4. The vector is digested with BamHI and Ascl to liberate the vector arms, which are used for ligation to the concatemer.

The ratio of vector arms to cassettes determines the maximum number of cassettes in the concatemer as illustrated in figure 9. The vector arms preferably are artificial chromosome vector arms such as those described in Fig. 8.

It is of course also possible to add stopper fragments to the concatenation solution, the stopper fragments each having a RS2 or RS2' in one end and a non-complementary overhang or a blunt end in the other end. The ratio of stopper fragments to cassettes can likewise control the maximum size of the concatemer.

As an alternative to providing vector arms for the concatenation procedure is possible to ligate the concatemer into an artificial chromosome selected from the group comprising yeast artificial chromosome, mega yeast artificial chromosome, bacterial artificial chromosome, mouse artificial chromosome, human artificial chromosome.

The number of concatemers in one single cell may be at least one concatemer per cell, preferably at least 2 concatemers per cell, more preferably 3 per cell, such as 4 per cell, more preferably 5 per cell, such as at least 5 per cell, for example at least 6 per cell, such as 7, 8, 9 or 10 per cell, for example more than 10 per cell. As described above, each concatemer may preferably comprise up to 1000 cassettes, and it is envisages that one concatemer may comprise up to 2000 cassettes. By inserting up to 10 concatemers into one single cell, this cell may thus be enriched with up to 20,000 heterologous expressible genes, which under suitable conditions may be turned on and off by regulation of the regulatable promoters.

Often it is more preferable to provide cells having anywhere between 10 and 1000 heterologous genes, such as 20-900 heterologous genes, for example 30 to 800 heterologous genes, such as 40 to 700 heterologous genes, for example 50 to 600 heterologous genes, such as from 60 to 300 heterologous genes or from 100 to 400 heterologous genes which are inserted as 2 to 4 artificial chromosomes each containing one concatemer of genes. The genes may advantageously be located on 1 to 10 such as from 2 to 5 different concatemers in the cells. Each concatemer may advantageously comprise from 10 to 200 genes, such as from 20 to 100 genes, for example from 30 to 60 genes, or from 50 to 100 genes.

### Screens, Sorting & Selection

In seeking to evolve molecules with defined pharmaceutical, industrial, nutritional properties one must have a method of selecting for those genetic patterns that encode for phenotypes that are consistent with these properties.

Each cell in a cell population, given that it is genetically different from other cells, has an intrinsic variability that can potentially express itself in one or more ways. For the purposes of the current invention the term Output shall be taken to mean a property of the cell that is consequent to the expression of one or more expression cassettes. Optionally the property may be consequent to both the expression of one or more expression cassettes and the expression of a certain set of host genes.

Outputs can be measured according to various different criteria. These criteria may be directly or indirectly linked to the functional or structural properties that are being optimised. Alternatively they may be inversely linked to functional or structural properties that are not desired.

Outputs can be measured either directly or by means of a reporter construct. For the purposes of this document the term Reporter Construct shall be taken to mean a genetic or molecular device for measuring whether a given cell or subset of cells in a cell population vary in respect of a given output from other cells or subsets of cells in the cell population. Example reporter constructs include a genetic construct that produces a fluorescent protein in response to the activation of a transcription factor by an output. Another example of a reporter construct is a coloured enzyme substrate, to which an enzyme is added that converts the substrate to another molecule with a different colour. Should the cell produce an output that inhibits the enzyme, the colour change will not occur.

Outputs that can be measured without a reporter construct include without limitation the survival of cells subjected to the screening criteria, cells able to metabolise a predetermined substance, cells able to produce a substance that preferentially absorbs electromagnetic radiation at one or more frequencies, cells having enzymatic efficacy in the media etc.

Reporter constructs can be placed proximal either before or after the expression construct is engineered into the cell. Methods of incorporating the reporter construct into a proximal location include but are not limited to standard transformation techniques, the mating of two different yeast mating types, or systems providing physical proximity between cell and reporter construct, for example gel microdroplet co-encapsulation of cell and reporter construct.

The term Proximal shall be taken to mean a location that is either in the same cell as the expression construct or sufficently close to said cell such that the concentration of a molecule or molecules diffusing from an intact or lysed cell, or being actively pumped from the cell, is at least one picomole in the vicinity of the location

Outputs of cells that may be measured either by proximal reporter contructs or by other means include, but are not limited to:
- Novel spectral properties
- Induced cytochrome oxidase activity
- Changed size, morphology, stickiness or adhesive properties or lack thereof
- Ability to grow on substrates they cannot normally grow on
- Ability to grow on sublethal substrates
- Ability to grow in the absence of normal essential requirements
- Ability to grow on media comprising one or more inhibitors
- Ability to grow under changed physical conditions, such as temperature, osmolarity, electromagnetic radiation including light of certain wavelengths.
- Ability to grow under magnetic field of certain force.
- Secretion or the lack of it from the cell
- The inhibition or prevention of inhibition of an enzyme
- The activation of a receptor.
- The prevention of an activating molecule binding to a receptor.
- The inhibition or promotion of binding of small molecules or proteins to nucleic acid or peptide sequences.
- The inhibition or promotion of transcription or translation of post translational processing.
- Changes in the transport or localisation of molecules within the cell or within organelles.
- Changes in the DNA content or morphology of the cell.
- The production of small molecules with certain properties that allow their selective isolation (e.g. all the chromoatography principles available to the skilled practitioner).
- The production of small molecules with certain spectroscopic properties (defined broadly to include visible light, microwaves, IR, UV, X-ray, etc.).
- Changes in the morphology of the cell, including the prevention or promotion of cell differentiation.
- The induction of apoptotic pathways.

With the exception of assays where the end point is survival, there is a need to sort the cells with the desired outputs from the cells without.

The use of fluorescence activated cell sorters (FACS) is one of the prefered ways of sorting cells since it's a technique that allows very rapid measurements on particles or cells.

The most important feature of flow cytometry is that it allows measurements at the single cell or capsule level and it is thus possible to identify the cells or capsules with the differential phenotypes.

Other important features of FACS are the ability to measure several different properties of a cell or capsule simultaneously and the ability to sort up to 100000 cells per second. Thereby screening of the large number of cell populations necessary for evolving cells according to the present invention is feasible and realistic.

Flow cytometry and the use of FACS are standard technology. General flow cytometry methods are for example described in "Flow cytometry,A practical approach", 3rd edition, Oxford University, Press, 2000, and "Flow cytometry Applications in cell culture", 1996, Marcel Dekker, Inc.

Other ways of sorting cells are e.g., colony picking either manually or with the use of automation.

As previously described a fitness function (F') can be defined that encapsulates the desired phenotype of the cell and mathematically relates this to one or more measured outputs. For example the fitness function may be defined as the multiple of a cell's absorption at two different wavelengths or alternatively it may be defined as the level of inhibition of one enzyme, divided by the inhibition of another enzyme, or it may be defined as the level of cytotoxic poison that a cell can survive, multiplied by the rate or reproduction of the cell in the absence of the cytotoxic or it may be defined in numerous other ways

In each screening round cells are selected that have outputs that correspond to one or more elements of the fitness function. In a preferred embodiment early screening rounds only measure one output whilst later screening rounds measure multiple outputs.

Those cells with the highest *fitness scores* in the population are removed from the screening environment for later use and/or analysis. Cells with lower F' scores may be discarded. By the highest F' score can be meant a predetermined percentage with the highest score, such as the best 1%, 5%, 10 % or 50%, or for very intense selection or very large cell populations the best 1‰, the best 0.1 ‰, the best 0.01 ‰, the best 0.001 ‰, or the best 0.0001%. Alternatively an absolute fitness score can be defined and only those cells that exceed this score are selected. By this approach the percentage of cells that are selected may vary.

In a preferred embodiment of this invention the screening and selection processes should be conducted on a repetitive or iterative basis, with each iteration being conducted on a daughter population.

For each iteration of the screening step, the fitness score that the cells are categorised upon is defined and the cell population subjected to screening. Over a series of iterations the fitness score is elaborated such that it progressively approaches the desired target value. The fitness score may be elaborated either by being increased or by having additional factors added into the equation that derives the fitness score.

The screening criteria are hence progressively optimised towards the desired functionality through the necessary rounds or cycles of screening and selection. The steps are repeated until at least one cell having the desired functionality has been evolved, such as repeated at least twice, such as at least three times, such as at least four times, such as at least five times, such as at least ten times, such as at least twenty times, such as least fifty times, such as at least one hundred times, such as at least two hundred times

In another embodiment the steps are repeated until at least two cell lines, or at least five cell lines, or at least 10 cell lines, having the desired functionality have been evolved. In a preferred embodiment at least a part of the cell lines evolved have different genetic patterns or genotypes, in a more preferred embodiment all the cell lines evolved have different genetic patterns or genotypes. By the term cell lines is meant cells originating from cells having met the screening criteria related to the determined screening functionality.

The screening criteria (or threshold) for one or more outputs may be increased for each repeat. Increasing criteria may for example be increasing concentration of a chemical, such as a toxin, in growth media for each repeat, or decreasing concentration of one or more nutrition components in the growth media or decreasing sensitivity or proximity of a reporter construct. Other examples of increasing criteria may be repetitive changes of temperature, either increasing or decreasing depending on the cell type chosen.

The screening criteria may also change character per repeat, such as starting with a concentration of a chemical substance in the growth media, and adding a physical parameter, such as light, in the next repeat, or starting with measuring the activity against one enzyme and adding activity against another enzyme in the next repeat.

It is also within the scope of the present invention that screening criteria may be a mixture of the criteria discussed above, ie. increased concentration of a chemical combined with changes of physical parameters, and/or increased concentration of one chemical combined with changed concentration of another.

Through this approach and in accordance with the general principles of evolution, over a series of screening and selection cycles host lines that most demonstrate the required characteristics are selected for and come to dominate the population. Over a series of screens the required fitness score is raised or elaborated, favouring those combinations that have led to an improvement in the expression of the desired characteristics.

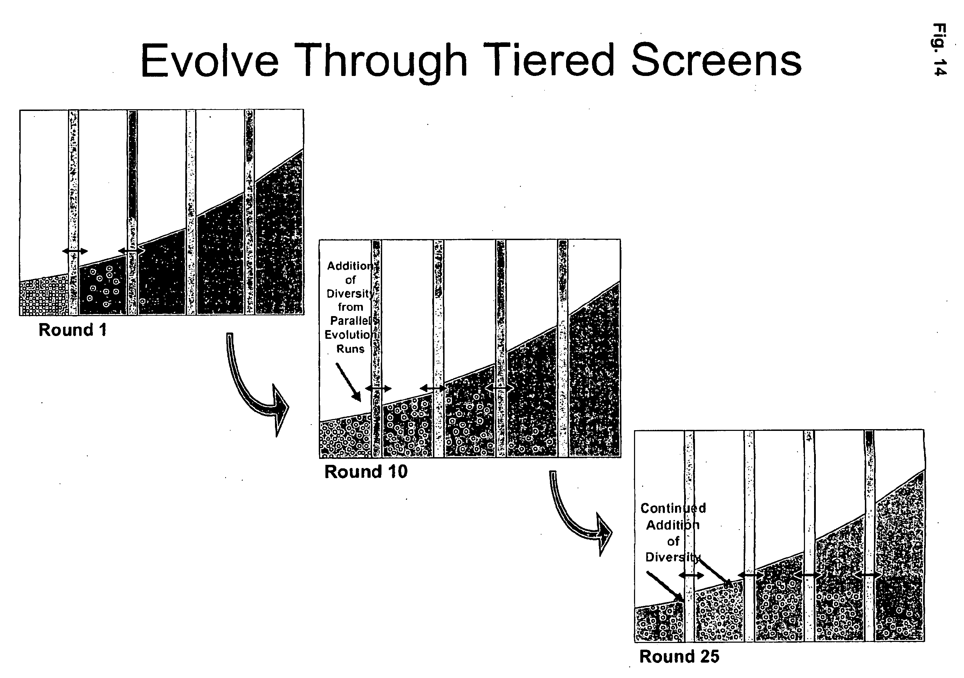

In one embodiment the host cell lines that are a priori believed to be interesting for a given target are selected and the selected lines evolved through a series of screens as set out in Figure 14.

In another embodiment the approach is one of an escalator of selection pressure using screens that move from the general / low activity to the specific / high activity with the generation of new genetic patterns between each step.

In another embodiment the fitness score is deliberately raised only marginally between selection cycles, such as by no more than 50% or by no more than 25% or by no more than 10% or by no more than 5% or by no more than 1%. Such gradualist selection pressures allow low level responses to be built upon over a series of selection cycles. By selecting marginal improvements in the fitness score such an approach maximises the genetic diversity at each stage in the selection process.

In another embodiment the approach is to walk down a specific multi-step metabolite pathway in a manner analogous to playing a slot machine. Once the first step of the pathway is obtained the genetic material for that step is put on "hold" by increasing its relative abundance such that most cells in the cell population contain said genetic material and the other genetic materials are then varied (spun or permed) until the second step is achieved, which is then also put on "hold". This process is repeated until the entire pathway is obtained.

In another embodiment the approach is to reverse up a specific multi-step metabolite pathway. Once the last step of the pathway is obtained the genetic material for that step is put on "hold" by increasing its relative abundance such that most cells in the cell population contain said genetic material and the other genetic materials are then varied (spun or permed) until the next but last step is achieved, which is then also put on "hold". This process is repeated until the entire pathway is obtained.

Also, a combination of both embodiments may be conducted, so that the pathway is built up from "both ends".

Yet another embodiment is to introduce multiple screening readouts (for specificity, ADME, toxicity etc.) such that molecules are optimised on multiple criteria all at once

In one embodiment of the invention the cells are subjected to the selection criteria under conditions that maximise the number of genes expressed by the cells, including the genes being heterologous to the cells. Alternatively the cells are subjected to the selection criteria under conditions that ensure a certain percentage or set of the genes being heterologous to the cells are expressed

It should be understood that the above approaches are general in concept and lend themselves to the construction of many variants, depending on the desired goal.

Furthermore, it should be understood that by using a cell-based system an advantage is that the compounds may be selected also on parameters not being included in the fitness function, in that the system inherently promotes evolution of compounds exhibiting properties such as not being toxic to the cell, as well as compounds that diffuse rapidly within the cell.

Examples of the approaches to build known or structural class focused pathways are as follows:
For small to medium sized pathways, i.e. pathways of up to 6-7 steps from metabolites of the host cell, the screening strategy relies on enriching the founder population with relevant genes and on the reasonably high probability of assembling over a series of selection rounds pathways that produce a low level of the desired property.
For large pathways (i.e. more than 6-7 steps) the screening strategy mostly involves dividing the pathway into subsets and a) defining screening parameters for each subset in order to build a pathway forwards or b) identifying intermediate metabolites that are feed to the cell population in order to assemble the pathway backwards.
For example in the case of retinoid like compounds it is well known that carotenoids are metabolised by specific tissues in specific classes of organisms to produce retinoids. It is thus possible to first evolve a population of cells that produce carotenoids and then mix the genes of this population(s) with those of a population(s) enriched for retinoid genes and in this manner evolve a population that produce retinoid like compounds.

Another example is the case of Taxol like compounds, for which the exact biosynthetic pathway is not known but is predicted to be somewhere between 12 and 20 enzymatic steps from yeast metabolites and several of the intermediate compounds have been isolated. It is thus possible to start by feeding a metabolite that is a few steps from Taxol in order to identify a population of cells able to produce Taxol like compounds from this precursor. Once this is achieved, the genes responsible for that small pathway are locked, e.g., integrated in the host's genome, or incorporated in artificial chromosomes at such high levels that statistical they occur in most cells and a second evolution process is started. This time the precursor being fed to the cell population is an earlier metabolite from the Taxol biosynthesis. By repeating this partial evolutions a number of times, it is possible to evolve a population of cells that produce Taxol like compounds starting with host metabolites.

Finally it should be said it is also possible to produce a class of compounds using a combination of both approaches described, i.e., by starting simultaneous evolution processes that cover the pathway backwards and forwards.

Interesting drug target for the present invention are for example the following:
3β hydroxysteroid dehydrogenase
3-hydroxy-3-methylglutaryl coenzyme A
5-adenosyl homocysteine hydrolase
5-HT₃ receptor
5-HT₄ receptor
23S rRNA of the 50S ribosomal unit
30S rRNA from 50S ribosomal unit
50S ribosomal unit binding site

α2 antiplasmin
α-adrenergic receptor
α-subunit of Na⁺/K⁺ ATPase (3 isoforms)
α-amylase
α-glucosidase
ACTH receptor
Adenosine deaminase
Adrenocortical steroid synthesis
Adrenocorticosteroid receptor
Adrenergic receptor β₁, β₂
Adrenocorticotropic hormone
Androgen receptor
Angiotensin-converting enzyme (ACE)
Angiotensin II formation
Angiotensin II receptor
Antiplatelet/antithrombotic agent
Arginine vasopressin receptor
Angiotensin receptors, AT1, AT2
ATP-sensitive K⁺ channel
Antigcoagulant protein C
Antigcoagulant protein S
Androgen receptor
Apoptosis
Aminoacyl tRNA site on 30S ribosomal unit (tetracycline)
Acetylcholinesterase
Adrenergic receptors α1, α2, β1, β2, β3
Aromatase
ATP sensitive K⁺ channels
Ascorbic acid

β-amyloid
β-adrenergic receptor
β-lactamase
β-subunit of DNA-dependent RNA polymerase
β-adrenergic receptors, b1
β-tubulin subunit of microtubules
Benzodiazepine receptor
Butyrylcholinesterase
Bradykinin receptors, B₁, and B₂

Carbonic anhydrase, type IV, II
Ca²⁺ channel
Ca²⁺ channel, Voltage-activated T-type
Catechol-O-methyltranferase
Calcitonin
Cell surface receptors for sulfonylureas on pancreatic β cells
*Cell surface receptors for glitinides on pancreatic* β *cells*
Cholecystokinin (CCK_{A}, CCK_{B})
Choline acetyltransferase
Cholinesterase
Carnitine
Calcineurin
Corticosteroid nuclear receptor
Cyclophilin, cyclosporin binding protein
CD₃ glycoprotein on T lymphocytes
*CD33 receptor*
*CD20 receptor*
CG-rich DNA (actinomycin)
Coagulation factor II, VII, IX, X
Corticosteroid adrenocorticotropin receptors
Cyclooxygenase 1, 2 (COX-1, COX-2)
Cyclic nucleotide phosphodiesterase
Cyclooxygenase
Cytochrome P450 reductase
Cytochrome P450 11β (11β hydroxylase)
Cytochrome P450 17α C17-20 lyase
Cytochrome P450 aldo, aldosterone synthase
Cytochrome P450 side chain cleavage (scc) enzyme
Cytochrome P450-dependent sterol 14 α-demethylase

_{D}-alanyl _{D}-alanine synthetase
Dihydropteroate synthetase
Deoxycytidine kinase
Dihydroorotate dehydrogenase
Dihydrofolate reductase
Dopamine D1-D5 receptors
DNA chain elongation factor
DNA cross-linking
DNA-dependent RNA polymerase
DNA gyrase, subunit a
DNA methylation
DNA polymerases I+III
DNA primase
DNA topoisomerase
DNA alkylation
DNA topoisomerase IV
DNA alkylation (oxamniquine)

Erythropoietin
Endo-β-d-glucuronidase
Estrogen receptor

Factors VII; VIII
Fusion protein (respiratory syncytial virus)
FKBP, tacrolimus binding protein, FK506 binding protein
Folic acid
Follicle-stimulating hormone (FSH)
FSH receptor

Glycerol phosphate oxidase
GABA_{A} receptor (6α variants, 3β, 2δ, 3γ variants
GABA transaminase
GABA_{A}-associated ion channel
Glutamic acid decarboxylase
Glutamate/aspartate receptors, AMPA, GLU 1-4, KA, GLU 5-7, NMDA 1,2_{A-D}, mGLU 1-7
Glycinamide ribonucleotide transformylase
Granulocyte colony-stimulating factor receptor
GHRH receptor
*Glucagon receptor*
Glucoamylase
Glucocorticoid receptor (GR)
GnRH receptor
Gonadotropin releasing hormone (GnHR)
Guanylyl kinase
G-protein coupled adenosine receptor
Ganglionic adrenergic neurons/norepinephrine transporter
Guanylate cyclase (nitroprusside)
Guanylyl cyclase (NO)
Granulocyte colony-stimulating factor
Granulocyte-macrophage colony-stimulating factor
Growth hormone receptor
Growth hormone-releasing hormone (GHRH)
Glycine receptor α, β

H⁺, K⁺ ATPase, proton pump
H₁ histamine receptor
H₂ histamine receptor
HCl secretion by gastric cells
Helicase
HIV Protease
HSV thymidine kinase
Hemoglobulin protease
Heparin antagonist
Hypoxanthine-guanine phosphoribosyl transferase
*Her-2* receptor
Histamine receptors H₁, H₂, H₃
Hepatic sulfotransferase as a catalyst

Intercellular adhesion molecule 1
Interleukin 1 receptor
Interleukin (IL-1, -2, -3, -4, -5, -6, -7, -8, -9, -10, -11, -12
Interleukin-2 receptor
IGF-1 receptor, IGF-2 receptor
Iodothyrinine-59-deiodinase, type 1, type 2
Influenza A virus M₂ protein
Inosine 5' phosphatedehydrogenase
Insulin-like growth factor 1
Interleukin-2 receptor
Inosinate dehydrogenase
Interferon a
Interferon a receptor
Inosine monophosphate dehydrogenase
Integrase
Interferon α
Interferon α receptor
Interferon γ
Insulin
Insulin-like growth factor (IGF-1, IGF-2)
Insulin receptor, α and β subunits
Insulin transporter

Kallikrein, aprotinin, C-esterase, α2 macroglobulin
Kinin

_{L}-alanyl racemase
L-aromatic amino acid decarboxylase
L-type voltage-sensitive Ca²⁺ channel
Leukocyte integrins
Leukotriene A hydrolase
Leukotriene B₄ receptor
Leukotriene C₄ receptor
Leukotriene C synthase
Leukotriene D₄/E₄ receptor
Lipocortin (protein), inhibits phospholiphase A₂
Lipoxygenases (12-lipoxygenase (platelets), 5-lipoxygenase (leukocytes)
LH/choriogonadotropin (CG) receptor
Luteinizing hormone (LH)
Lactamase
Lipoprotein lipase

M₁ receptor, muscarinic cholinergic
µ and δ receptor in gastrointestinal tract
Macrophage colony-stimulating factor
Microbial dihydrofolate reductase
Microtubular protein
Mineralocorticoid receptor
Mineralocorticoid receptor (MR)
Monoamine oxidase (MAO)-A
Monoamine oxidase (MAO)-B
Muscarinic receptor, M₁, 3 subunits
Muscarinic receptor, M₂ , 3 subunits
Muscarinic receptor, M₃ , 3 subunits
Muscarinic receptor, M₄ , 3 subunits
Mycobacterial RNA polymerase
N-acyl hydrolase
Na⁺ channel, α1, β1, β3
Na⁺ channel α, β, γ
Na⁺/Cl-symporter
Na⁺/K⁺/2Cl-symporter
Niacin receptor
Nicotinic acid
Nicotinic receptor
Nicotinic cholinergic receptors, muscle N_{M} α, β, δ, γ, ε
Nicotinic cholinergic receptors, neuronal, N_{N} α2, α3, α4, α5, α6, α7, α8, α9, β2, β3, β4
Neuramidase
Neuropeptide Y, Y1, Y2 receptors
Noradrenaline transporter

Opioid receptors µ₁₋₂, δ₁₋₂, κ₁₋₃
Oxytocin & receptor

Platelet-derived growth factor
Parathyroid hormone (PTH)
Peroxidase
Progesterone receptor
Prolactin
Prolactin receptor
Parasite β-tubulin
Parasite dihydrofolate reductase
Parasite glutamate gated Cl⁻ channel
Penicillin-binding protein 1a (PBP 1a, 1b), transpeptidase
PBP 2a, 2b
PBP 3, 4, 5, 6, 7
Platelet glycoprotein IIb/IIIa (fibrinogen receptor)
Plasma protein transferrin (β1 glycprotein)
Pyridoxine receptor
Penicilloyl enzyme
Peptidyl site of the 50S ribosomal unit
Primase
Phosphodiesterase (type IV, cyclic nucleotide phosphodiesterase)
Phospholiphase A₂, C
Platelet-activating factor
Prostacyclin synthase
Plasmodial heme polymerase
Progesterone receptor
Pyridoxine
Phospholipase Cβ
Purine receptors, P1 (A_{1,2a,2b,3}), P_{2X}, P_{2Y}
Peroxisome proliferator-activated receptor
Pancrelipase
Potassium channel
Prostaglandin 15-OH dehydrogenase
Prostaglandin D-DP receptor
Prostaglandin E1, E2, E3-EP receptor
Prostaglandin F-FP receptor
Prostaglandin I2-IP receptor
Prostaglandin I₂ (PGI₂) receptor
Prostaglandin F₂ receptor
Prostaglandin synthetase
Prostaglandin I₂ receptor

Reverse transcriptase
Ribosomal protein from 50S ribosomal unit (streptomycin)
Rh 0
Riboflavin receptor
Retinoic acid α, X receptors
Ribonucleoside diphosphate reductase
Ribonucelotide reductase

Somatostatin
Somatostatin receptors, several
Steroid 5 α reductase 1, 2
Sucrase
Squalene epoxidase
Stem cell factor, c-kit ligand
Serotonin receptors (5-HT) 5-HT_{1A-F}, 5-HT_{2A-C}, 5-HT₃, 5-HT₄₋₇
Succinic semialdehyde dehydrogenase
Spindle formation
Scission of DNA
Secretion of vasopressin K receptor

Topoisomerase I, II, III, IV
Tubulin
Thrombopoietin
Thrombin
Tissue plasminogen activtor
Thymidylate synthetase
Tachykinins, NK1, NK2, NK3
Tryptaminergic receptor
Thromboxane A₂ TP receptor, platelet and non-platelet
Thromboxane synthase
Thyroid-stimulating hormone (TSH) receptor, TRα 1,2, TRβ 1,2
Tumor necrosis factor receptor
Trypanothione reductase
Type I cyclic nucleotide phosphodiesterase
Type III cyclic AMP phosphodiesterase
Type V cyclic nucleotide phosphodiesterase
Transpeptidase
Thymic lymphocyte antibodies
Tumour necrosis factor alpha
Thiamine

Uridine monophosphate pyrophosphorylase

Vascular cellular adhesion molecule 1 receptor
Vasopressin receptors V₁ₐ, V_{1b}, V₂,
Viral DNA polymerase
Vitamin A nuclear receptor
Vitamin E
Vitamin K & receptor
Vitamin B₁₂ receptor
Vitamin D nuclear receptor
Voltage-activated Ca²⁺ channel, L-type

### Generation of Novel Genetic Compositions

It is a requirement of evolutionary processes that new patterns are generated either in parallel to or sequential to selection steps. In systems where the patterns are based on genetic elements this requires that either new genetic elements are introduced or new combinations of existing genetic elements are created or both.

In the present invention new patterns can be achieved through one or more of the following processes. The term combining or remixing shall be taken to mean a process of generating new combinations of expression constructs using one or more of these approaches. The combination or remixing may be conducted at any step of the selection process and a preferred timing is when cells having elements of the predetermined functionality have been found in at least one of the compositions, and preferably in at least 0.1%, such as at least 1%, such as at least 2%, such as at least 5%, such as at least 10% or at least 50% of compositions. The term Daughter Population shall be taken to mean a cell population that is predominately genetically descendant from those cells in one or more cell populations that had a fitness score above a certain threshold and that is further characterised by most of the cells in the daughter population having been generated through a remixing step.

In principle the combination or remixing may be conducted by at least the following approaches: physical isolation and remixing of expression cassettes, physical isolation and remixing of artificial chromosomes containing expression cassettes, sexual crosses, cell- or protoplast fusion (vide Hugerat Y, Spencer F, Zenwirth D, Simchen G (1994). Genomics 22(1), p. 108-117), and YAC-duction (vide Curran BP, Bugeja VC (1996), Methods Mol. Biol. 53, p 45-49.

Physical isolation of the expression cassettes and subsequently mixing the cassettes is preferred. One advantage of this approach is that any accumulating host mutations are removed by the remixing of genes into new host lines. Reporter genes can also be introduced as part of this process, allowing for the introduction of intracellular reporter assays. The remixing is preferably carried out in vitro by removing the expressible sequences from at least two different cells, combining the individual expressible sequences in vitro, and introducing at least two combined expressible sequences into at least two cells.

Due to the common structure of the expression cassettes according to a preferred embodiment of the invention, these may easily be excised from the host cells again using a restriction enzyme specific for the rs₁-rs₂ restriction site According to the present invention the enzyme specific for the rs₁-rs₂ restriction site is preferably a rare cutter therefore the likelihood of cutting host genomic DNA fragments with a size similar to the size of the expression cassettes is very limited. After excision the expression cassettes may be mixed with other expression cassettes of similar structure and be re-concatenated and re-inserted into another host cell in another combination creating a greater diversity during the evolution steps.

The combination of expressible sequences may of course also be a combination of full length chromosomes in the cells, such as combination of artificial chromosomes. Combination of the artificial chromosomes may be achieved in at least 4 ways depending on the host cells. These are physical isolation, crosses, protoplast fusion and YAC-duction as described herein.

An alternative way of physically remixing expression cassettes is to isolate the artificial chromosomes from one or more cell populations and re-transform new host cells. The host cells may or may not already contain artificial chromosomes containing expression cassettes.

Thus, new genetic compositions may be achieved by induction of different mating types in the two (or more) populations followed by sexual crosses yielding cells that are diploid for the normal complement of chromosomes and contains the artificial chromosomes of both partners in the cross. Subsequent mitosis (with or without haploidisation) will then result in new vegetative cells containing new combinations of artificial chromosomes.

### Addition of new genetic material.

The remixing is preferably conducted with addition of new genetic material from another cell composition. The other composition may be chosen from compositions capable of expressing at least one predetermined phenotype, such as a protein or a metabolite, or it may be chosen at random.

In one embodiment it is desirable to conduct selection in a series of isolated populations that are then brought together once they have independently evolved useful traits. In this manner the use of independent selections for same phenotype provides different genetic backgrounds (a form of parallel evolution) that can then ideally act synergistically with each other.

In another embodiment the result of selection on two or more compositions is mixed at a certain step of evolution to create further modified compositions when aiming for at least one cell having the desired functionality.

Recombination of the expressible sequences, i.e. changes of the genetic material by for example cross-over, may be optionally avoided, due the construction of the genetic inserts, in particular spacer sequences, as well as due to a general attempt to suppress recombination in the cells. Thereby combination of the genetic material is favoured, leading to combination of intact genes or cDNA material, without the risk of destroying the function of the genetic material due to recombination.

After having obtained daughter populations exhibiting the desired functionality, the daughter population may then be subjected to further steps of screening and selection in order to optimise the cells.

### Evolved Cells

In another aspect the invention relates to the cells evolved having the desired functionality. In a preferred embodiment the cells evolved have a genetic construction as defined above in relation to the starting material, however often having another combination of heterologous genetic material than the starting material.

The evolved cells may be subjected to analysis with respect to the gene(s) responsible for the desired functionality in order to possibly optimise the genes leading to the desired phenotype.

However, the cells may also be used as such as production cells capable of producing a novel metabolite or a novel pathway. In this respect it is preferred that the cells evolved are cells suitable for production in for example fermentation tanks.

### Novel Molecules and Pathways

The aim of the evolution method according to the present invention is to evolve cells capable of producing new substances, such as new metabolites, new proteins, and/or new pathways.

Thus, in a further aspect the present invention relates to a substance produced by the cells evolved according to the present invention, said substance being metabolites, proteins, carbohydrates, poly- and oligosaccharides, and ribonucleic acids. Since some of the interactions that produce the novel phenotypes are mediated by enzymes it is likely that the result will include novel compounds with chiral centres, which are especially difficult to produce via chemical synthesis.

Creation of novel pathways, may lead to the capability of creating cells capable of metabolising, i.e. converting, a compound, which is not metabolisable by the native, un-evolved cell. Thus, in particular the substance is a metabolite.

### Examples

### EXAMPLES OF APPPROACH

Examples of how the current invention can be used to evolve cells with potential pharmaceutical, industrial, agronomic, or nutritional utility are now provided.

### Evolution of specific structural classes via short to medium sized pathways

### Example 1: Evolution of Carotenoid like compounds

### UTILITY

Carotenoids are natural pigments displaying yellow, orange, pink, red and blue colours. A major role is protection against oxidative damage. Carotenoids are both pharmaceutically relevant (used to treat bronchial asthma and involved in the prevention of cancer) and of commercial value.

### SCREENING & SELECTION STRATEGY

- Production of a different colour by host cell. Screen done using a fluorescence activated cell sorter (FACS)
- Anti-oxidant protection. Screen done using methylene blue as a producer of singlet oxygen species.

### PROCEDURE

- STEP 1: Essentially full length cDNA libraries are made from the species in the list provided in this example.
- STEP 2: cDNA libraries are made using a pool of 4 entry vectors: pEVE4, pEVE5, pEVE8 and pEVE9 in a proportion of 30:30:1:30. See Figures 4, 5, 6 and 7.
- STEP 3: Each cDNA library is normalised essentially as method 4 described in Bonaldo, MF et al. (1996) Genome Res. 6: 791-806.
- STEP 4: Coding sequences from a non-normalised yeast (*Saccharomyces cerevisiae*) cDNA library are amplified by PCR and are used as driver for subtractive hybridization against single stranded circular DNA prepared from the normalized library (Bonaldo, MF et al. (1996) Genome Res. 6: 791-806), in order to remove household genes. Remaining single stranded circles are purified, converted to double stranded DNA and used to transform E.coli DH5α.
- STEP 5: EVAC (Evolvable Artificial Chromosome) containing cell populations are made using 10 different normalised and enriched cDNA libraries in each.

### Preparation of expression cassettes

1. inoculate 5 ml of LB-medium (Sigma) containing 100 µg/L ampicillin with library inoculum corresponding to a 10+ fold representation of library. Grow overnight.
2. make plasmid miniprep from 1.5 ml of culture (E.g. Qiaprep spin miniprep kit)
3. digest plasmid w. Srf 1
4. dephosphorylate fragments and heat inactivate phosphatase( 20 min, 80°C)
5. digest w. Asc1
6. run 1/10 of reaction in 1 % agarose gel to estimate amount of fragment

### Preparation of pYAC4-Asc arms

1. inoculate 150 ml of LB medium (Sigma) with a single colony of DH5α containing pYAC4-Ascl
2. grow to OD₆₀₀ ∼ 1, harvest cells and make plasmid preparation
3. digest 100µg pYAC4-Ascl w. BamH1 and Asc1
4. dephosphorylate fragments and heat inactivate phosphatase( 20 min, 80°C)
5. purify fragments(e.g. Qiaquick Gel Extraction Kit)
6. run 1 % agarose gel to estimate amount of fragment

### Preparation of EVACs

1. mix expression cassette fragments with YAC-arms so that cassette/arm ration is ∼1000/1
2. if needed concentrate mixture (use e.g. Microcon YM30) so fragment concentration > 75 ng/µL of reaction
3. add 1 U T4 DNA ligase, incubate 16C, 1-3 h . Stop reaction by adding 1 µL of 500 mM EDTA
4. run pulsed field gel (CHEF III, 1% LMP agarose, ½ strength TBE, angle 120, temperature 12 C, voltage 5.6V/cm, switch time ramping 5 - 25 s, run time 30 h) Load sample in 2 lanes.
5. Stain part of the gel that contains molecular weight markers
6. cut sample lanes corresponding to MW. 100 - 500 kb
7. agarose gel in high NaCL agarase buffer. 1 u agarase / 100 mg gel
8. concentrate preparation to < 20 µL
9. transform suitable yeast strain w. preparation using electroporation: 100 ml of YPD is inoculated with one yeast colony and grown to OD₆₀₀ ≡ 1.3 to 1.5. The culture is harvested by centrifuging at 4000 × g and 4°C. The cells are resuspended in 16 ml sterile H₂O. Add 2 ml 10 × TE buffer, pH 7.5 and swirl to mix. Add 2 ml 10 × lithium acetate solution (1 M, pH 7.5) and swirl to mix. Shake gently 45 min at 30°C. Add 1.0 ml 0.5 M DTE while swirling. Shake gently 15 min at 30°C. The yeast suspension is diluted to 100 ml with sterile water. The cells are washed and concentrated by centrifuging at 4000 × g, resuspending the pellet in 50 ml ice-cold sterile water, centrifuging at 4000 × g, resuspending the pellet in 5 ml ice-cold sterile water, centrifuging at 4000 × g and resuspending the pellet in 0.1 ml ice-cold sterile 1 M sorbitol. The electroporation was done using a *Bio-Rad Gene Pulser*. In a sterile 1.5-ml microcentrifuge tube 40 µl concentrated yeast cells were mixed with 5 µl 1:10 diluted EVAC preparation. The yeast-DNA mix is transferred to an ice-cold 0.2-cm-gap disposable electroporation cuvette and pulsed at 1.5 kV, 25 µF, 200 Ω. 1 ml ice-cold 1 M sorbitol is added to the cuvette to recover the yeast. Aliquots are spread on selective plates containing 1 M sorbitol. Incubate at 30°C until colonies appear.

- STEP 6: The EVAC containing cell libraries produced in step 5) are pooled into one screening population.
- STEP 7: The screening population is divided into two equal portions. One of the portions is screened for anti-oxidant properties (step 8) and the other for differential colour production (step 9).
- STEP 8: Anti-oxidant screen:
a. The screening population is amplified ten times and divided in 10 portions.
b. The sub populations are grown in liquid culture under selective conditions for the artificial chromosomes to an OD₆₀₀ of 0.6 - 1.0.
c. The heterologous genes are induced/de-repressed by re-suspending the cells in a medium lacking methionine and with 200 µM Cu₂SO₄ and the cells are grown under inducting conditions for 24 hours prior to screening.
d. Each sub population is exposed to 1 out of a range of 10 concentrations of Methylene blue. Immediately after exposure to Methylene blue, the cells are irradiated with a 200 W halogen lamp for 15 minutes.
e. Survival rates are determined after 2 hours. The surviving cell population exposed to the highest concentration of Methylene blue, where cells statistically representing 10% of the original cell lines survived, is selected.
- STEP 9: Differential colour production screen:
a. The screening population is grown in liquid culture under selective conditions for the artificial chromosomes to an OD₆₀₀ of 0.6 - 1.0.
b. The heterologous genes are induced/de-repressed by re-suspending the cells in a medium lacking methionine and with 200 µM Cu₂SO₄.
c. The cells are grown under inducting conditions for 24 hours prior to screening.
d. Flow cytometry is used to isolate the cells with the highest expression level of different colours, essentially as described in An, G. H et al, 1991, Bio/Technology 9:70-73.
e. The cells representing (by statistics) the 10% strongest colour expressing cell lines are selected.

Remixing of genetic diversity selected in steps 8 and 9 is done by excising the expression cassettes, mixing them and religating into new EVACs. This process is described in steps 10 to 13.
- STEP 10: Each of the populations selected in steps 8 and 9 is amplified and equal amounts of each amplified population are pooled.
- STEP 11: Total DNA is isolated following standard procedures.
- STEP 12: The total DNA is digested with Ascl and DNA fragments of the appropriate size (2-10kB) are isolated.
- STEP 13: New EVACs containing the purified DNA fragments and 10% of cassettes (w/w) that have not been used to assemble EVACs in any previous synthesis are synthesised essentially as described in step 5.
- STEP 14: Steps 7 to 13 are repeated 5 times always taking forward the best 10% of cell lines from each screen.
- STEP 15: The new cell population resulting from the completion of the fifth cycle is not divided and is grown in liquid culture under selective conditions for the artificial chromosomes to an OD₆₀₀ of 0.6 - 1.0. The heterologous genes are induced/de-repressed by re-suspending the cells in a medium lacking methionine and with 200 µM Cu₂SO₄
- STEP 16: The combined population is now screened for colour. Coloured cells are screened for anti-oxidant activity. The concentration of Methylene blue used is the highest concentration where, after 2 hours of exposure to the methylene blue, the number of surviving coloured cells represents 5% of the cell lines in the original population. These cells are selected.
- STEP 17: The selected population is amplified and new EVACs are produced as described in steps 11 to 13.
- STEP 18: Steps 15 to 17 are repeated 30 times always taking forward cells representing the best 5% of cell lines.
- STEP 19: Cell lines that can survive a 10X higher concentration of Methylene blue than the original population and have clearly visible bright yellow to red colours are taken out of the evolution process. The genes responsible for these activities are subcloned and characterised by DNA sequencing.
- STEP 20: Cells with the characteristics described in step 19 and that have significantly different genotypes are analysed using standard natural product chemistry in order to identify the compound(s) responsible for the phenotype.

### PRIORITISED SPECIES AND TAXONOMIC GROUPS

Sourced species are divided into:
- Species that produce carotenoids: plants, algae, some fungii and photosynthetic bacteria
- Species that modify carotenoids to produce other carotenoids: some animals
- Specific genes

Species that produce carotenoids:
Plants: Actinidia deliciosa (Kiwi); Arabidopsis thaliana; *Brassica rapa, Tagetes erecta* (Marigold flowers), *Olea europaea* (olive), *Lactuca sativa var. romaine* (romaine lettuce), *Quercus robur* (oak), *Pinus pinaster* (maritime pine), *Capsicum annuum* (Pepper), *Bixa orellana, Sarcina lutea, Viola tricolor, Lonicera japonica, Delonix regia, Zea mays* (maize), *Eschscholzia californica, Carica papaya* (papaya), *Daucus carota* (carrot), *Lycopersicon esculentum* (tomato), *Crocus sativus* (saffron), *Verbascum phlomoides, Physalis alkekengi, Gentiana spp., Nicotiana tabacum, Pittosporum tobira*
Algae: *Rhizophora mangle* (red mangrove), *Haematococcus pluvialis* (Green Algae), *Enteromorpha linza* (Patagonian macroalga), *Ulva lactuca* (Sea lettuce), *Caulerpa mexicana, Gigartina sp, Polysiphonia sp, Porphyra sp, Macrocystis pyrifera* (Giant kelp), *Sargassun sp., Nanochlorum eucaryotum*, Dunaliella bardawil, Scenedesmus *obliquus, Oscillatoria rubescens, Phormidium luridum, Arthrospira spp., Astasia ocellata, Fucus vesiculosus, Bathycoccus prasinos, Micromonas pusilla, Botryococcus braunii,*
Fungi: *Xanthophyllomyces dendrorhous, Neurospora crassa, Cantharellus cibarius, Phycomyces blakesleeanus, Puccinia graminis, Epicoccum spp., Lycogola epidendron*
Bacteria: Roseiflexus castenholzii, Streptococcus faecium, *Rhodopseudomonas acidophila*, Erwinia herbicola, Agrobacterium aurantiacum, *Halorhodospira abdelmalekii Halorhodospira halochloris, Anabaena PCC 7120,* Chlorobium tepidum, Cholroflexus aurantiacus. Thermus thermophilus, flexibacter spp., Rhodobacter capsulatus, *Staphylococcus aureus, Deinococcus radiodurans, Meiothermus ruber, Chloroflexus aurantiacus*

Species that modify carotenoids:
Birds: *Carduelis tristis, Cardinalis cardinalis,* flamingo
Fishes: *Carassius auratus* (goldfish), *Micropterus salmoides* (black bass), *Paracheirodon axelrodi* (cardinal tetra), *Amphiprion ocellaris* (common clown), *Zebrasoma flavescens* (Yellow tang), *Synchiropus splendidus* (mandarinfish), *Lactoria cornuta* (long-horned cowfish)
Invertebrates: *Cucumaria japonica* (sea cucumber), *lanthella basta* (sponge), *Clibanarius erythropus* (hermit crab), *Daphnia magna, Homarus americanus* (Lobster), *Paralithodes brevipes* (King Crab), *Fusinus perplexus* (seashell), *Halichondria okadai* (sponge), *Suberites massa* (sponge), *Pentacta australis* (sea cucumber), *Pseudocentrotus depressus,* (sea urchin), *Ophiuroidda spp.* (brittle star), *Papilio xuthus* (butterfly), *Mytilus coruscus* (japanese sea mussel), *Crassostrea gigas* (oyster), *Glossodoris spp.,* (sea slug), *Fromia elegans* (star fish), *Actinia equina* (strawberry beadlet anemone), *Anemonia viridis* (anemone), *Hippolysmata graghami,* (shrimp), *Lysmata debelius* (shrimp), *Halocynthia papillosa* (sea squirt), *Crossaster papposus* (starfish)

Specific Carotenoid genes :
ggps, psy, pds, zds, lcy-b, lcy-e, bhy, zep *(Gentiana sp.)*, idi, crtC, crtF *(Rhodobacter capsulatus)*, crtE, crtB, crtl, crtY, crtZ *(Erwinia uredovora*), zds *(Nostoc anabaena),* pds (*Synechococcus PCC7942),* crtE, crtB, crtl, crtY, crtZ *(Erwinia herbicola),* crtM, crtN *(Staphylococcus aureus),* crtl, crtYb *(Xanthophyllomyces dendrorhous),* ccs, crtL *(Capsicum annuum),* crtL, bchy *(Nicotiana tabacum),* lcy-b, lcy-e *(Prochlorococcus sp.),* idi *(Saccharomyces cerevisiae)*, crtl, crtYe, crtYf, crtEb *(Corynebacterium sp.),* psy-1 *(Lycopersicon esculentum),* al1 *(Neurospora crassa)*

### Example 2: Evolution of Omega fatty acid like compounds

### UTILITY

Unsaturated fatty acids are important components for normal cellular function, are involved in cell membrane fluidity and serve as precursors to eicosanoids, including prostaglandins and leukotrines. In mammals, these eicosanoids are involved in inflammatory responses, regulation of blood pressure, and reproductive function

### SCREENING & SELECTION STRATEGY

- Cell membrane fluidity (Flow cytometry screen)
- Tolerance to ethanol (survival assay)

### PROCEDURE.

The same procedure as that described in Example 1 is performed, except that the following changes are made to the following numbered steps
- Step 7: The screening population is divided into 2 equal portions. One of the portions is screened for tolerance to ethanol (step 8) and the other for cell membrane fluidity (step 9)
- Step 8: Tolerance to ethanol:
a. The screening population is amplified ten times and divided in 10 portions.
b. The sub populations are grown in liquid culture under selective conditions for the artificial chromosomes to an OD₆₀₀ of 0.6 - 1.0.
c. The heterologous genes are induced/de-repressed by re-suspending the cells in a medium lacking methionine and with 200 µM Cu₂SO₄ and the cells are grown under inducting conditions for 36 hours prior to screening.
d. Each sub population is exposed to 1 out of a range of 10 concentrations of ethanol. Survival rates are determined after overnight culture. The cell population from the highest concentration of ethanol where cells statistically representing 10% of the original cell lines survived, is selected.
- Step 9: Cell membrane fluidity screen:
a. The screening population is grown in liquid culture under selective conditions for the artificial chromosomes to an OD₆₀₀ of 0.6 - 1.0.
b. The heterologous genes are induced/de-repressed by re-suspending the cells in a medium lacking methionine and with 200 µM Cu₂SO_{4 .} The cells are grown under inducting conditions for 20 hours with 1 tenth of the library being analysed by flow cytometry after every 2 hours.
c. For each of the sub populations flow cytometry is used to isolate the 10 % of cell lines with the most fluid cell membranes but which are still growing normally. This is done essentially as described in Benderitter M. et al, Cytometry, 2000, 39(2), 151-7
- Step 10: Each of the populations selected in steps 8) and 9) is amplified. For each of the selected populations, steps 11-13 are repeated separately.
- Step 16: The combined population is now screened for ethanol resistance. Surviving cells are screened for increased membrane fluidity. The concentration of ethanol used is the highest concentration where, after overnight exposure to ethanol, the number of surviving cells whose membrane fluidity exceeds the average fluidity by more than 2 x the standard deviation at 30 °C represents 5% of the cell lines in the original population.
- Step 19: Cells that can survive a 1.5 x higher concentration of Ethanol than the original population and have a cell membrane fluidity that exceeds the average fluidity of the original population by more than 5 x the standard deviation at 30 °C are taken out of the evolution process. The genes responsible for these activities are characterised by subcloning and DNA sequencing.

### PRIORITISED SPECIES AND TAXONOMIC GROUPS

- Plants (in particular seeds)
- Animals (in particular adipose tissues)
- Fish
- Random group of taxonomically diverse eukaryotic species

### Evolution of specific structural classes via longer pathways

### Example 3: Evolution of Retinoid like compounds

### UTILITY:

Retinoids are derivatives of vitamin A and are modulators of cellular proliferation as well as effectors of morphogenic changes. Activity of retinoids as antineoplastic agents has been demonstrated in several *in vivo* experimental carcinogen models (mainly for skin, respiratory tract, urinary bladder, breast, digestive tract) Cellular responses to retinoids are generally mediated by two families of nuclear receptors (RARs and RXRs) that belong to the steroid-thyroid hormone (or nuclear) receptor superfamily and behave as ligand-activated transcription factors that bind as dimers to the cis-acting response elements of target genes.

Different retinoic acid receptor isotypes display a characteristic pattern of tissue distribution, RARα being the most ubiquitously distributed. RARß plays an important role in lung development and has been proposed to have a tumour suppressor function in lung.

### SCREENING & SELECTION STRATEGY

- To obtain carotenoid like compounds screens for colour production and anti-oxidant protection are used (see example 1).
- To obtain retinoid like compounds an activation assay of retinoic acid receptor, RARß is used. This assay employs a reporter system. Reporter construct is initially placed intra-cellularly and later on extra-cellularly.

### PROCEDURE

The same procedure as that described in Example 1 is performed, except that the following changes are made to the following numbered steps.
- Step 5: EVAC containing cell populations are made using 10 different normalised and enriched cDNA libraries in each. EVACs are transformed into the cell population evolved in example 1.
1. Grow the carotenoid producing cell population to mid log, 2 x 10⁶ to 2 x 10⁷ cells/ml in liquid medium, at 30°C and with aeration, under selective conditions for the EVACs.
2. Spin to pellet cells at 400 x g for 5 minutes; discard supernatant.
3. Resuspend cells in a total of 9 ml TE, pH 7.5. Spin to pellet cells and discard supernatant.
4. Gently resuspend cells in 5 ml 0.1 M Lithium/Cesium Acetate solution, pH 7.5.
5. Incubate at 30°C for 1 hour with gentle shaking.
6. Spin at 400 x g for 5 minutes to pellet cells and discard supernatant.
7. Gently resuspend in 1 ml TE, pH 7.5. Cells are now ready for transformation.
8. In a 1.5 ml tube combine:
   - 100 µl yeast cells
   - 5 µl carrier DNA (10 mg/ml)
   - 5 µl Histamine Solution
   - 5/100 of an EVAC preparation in a 10 µl volume (max). (One EVAC preparation is made of 100 µg of entry vector library plasmid mixture)
9. Gently mix and incubate at room temperature for 30 minutes.
10. In a separate tube, combine 0.8 ml 50% (w/v) PEG 4000 and 0.1 ml TE and 0.1 ml of 1 M LiAc for each transformation reaction. Add 1 ml of this PEG/TE/LiAc mix to each transformation reaction. Mix cells into solution with gentle pipetting.
11. Incubate at 30°C for 1 hour.
12. Heat shock at 42°C for 15 minutes; cool to 30°C.
13. Pellet cells in a microcentrifuge at high speed for 5 seconds and remove supernatant.
14. Resuspend in 200 µl of rich media and plate in appropriate selective media
15. Incubate at 30°C for 48-120 hours until transformed colonies appear.
- Step 7: The screening population is not divided
- Step 8: Retinoic Acid Receptor Activation:
a. The screening population is amplified ten times.
b. The EVAC containing cell population is mated with a reporter strain containing a reporter construct in an yeast vector and a yeast expression plasmid containing the cDNA for the human retinoic acid receptor (RARβ) and cultured under selection for the haploid cells, the reporter system and the EVACs. The reporter gene used is β- galactosidase. The reporter strain is constructed essentially as described in Salerno et al. 1996, Nucleic Acids Res. 24(4), 566-72.
c. The heterologous genes are induced/de-repressed by re-suspending the cells in a medium lacking methionine and with 200 µM Cu₂SO₄. The cells are grown under induction conditions for 18 hours. Growth and β-galactosidase activity are assayed in 96 well microtiter plates essentially as described in Coldham et al., 1997, Environ. Health Perspect., 105(7), 734-42.
d. Cells with the 10% highest β-galactosidase activity are selected.
- Step 9: There is no step 9.

Remixing of genetic diversity selected in steps 8 and 9 is done by physical re-isolation and re-transformation of EVACs.
- Step 10: The population selected in step 8 is grown in 5 ml of YPD to an OD₆₀₀ > 1.0
- Step 11: Two 100 µl plugs of total DNA are produced as described in BioRad's "CHEF genomic DNA plug kits" manual, Procure n.2
- Step 12: EVACs are purified and isolated:
a. Plugs are cut and loaded into 3 slots of a pulsed field gel
b. Run PFGE
   i. For EVACs < 1000 kb : Chef III, 1% Agarose, 1/2 strength TBE, 6V/cm, 14°C, 120° angle, 50 - 90 sec. Switch time, 22 h runtime.
   ii. For EVACs > 1000 kb. Chef III, 1% Agarose, 1/2 strength TBE, 6V/cm, 14°C, 120° angle, 60-120 sec. Switch time, 24 h runtime
c. stain one lane to identify position of EVACs
d. cut corresponding part of the two non-stained lanes and digest the agarose by agarase treatment following standard procedures e.g. Pulsed Field Gel Electrophoresis. A practical approach.(Ed. A.P. Monaco) Oxford University Press 1995.
e. Concentrate agarased preparation to 100 µL by ultrafiltration.(e.g. Microcon YM-30, Millipore)
f. add 400 TE to retentate and repeat concentration step. Repeat and concentrate to 25 µL
- Step 13: EVACs are transformed into yeast as before.
- Step 16: Step 8 is essentially repeated but the cell population is co-cultured together with a reporter strain under selection for both strains. Thus compounds that activate RARβ now have to be reasonably hydrophilic in order to cross the cell membranes.
- Step 19: Commencing with the tenth cycle, in step 16, cells that show a similar activation of RARβ as 0.1 µM of retinoic acid under similar circumstances are taken out of the evolution process. The genes responsible for these activities are characterised by subcloning and DNA sequencing

### PRIORITISED SPECIES AND TAXONOMIC GROUPS:

- Species that metabolise carotenoids to retinoids: mammals (specially liver and retina tissues), fishes (liver), insects and other animals.

### Example 4: Evolution of Taxol like compounds

### UTILITY.

Taxol is a higly effective anticancer drug used widely in the treatment of various carcinomas, melanomas and sarcomas. The unique mode of action of this drug, as well as its outstanding potency makes Taxol one of the most efficacious anticancer agents in current use. Taxol promotes assembly of microtubules and prevents depolymerization. This induces bundles of stable microtubules and blocks cell cycle mitosis.

### SCREENING & SELECTION STRATEGY

- Stabilisation of microtubule assembly. The microtubule assembly assay is done using microtiter plates and measuring the absorbance.
- Growth inhibition of murine fibroblast cells. The growth inhibition assay is done using gel microdroplets and flow cytometry.

### PROCEDURE

Taxol's biosynthesis pathway is thought to involve between 12 and 20 enzymatic steps starting from geranylgeranyl diphosphate. The first committed step in the formation of taxol involves the cyclisation of geranylgeranyl diphosphate. After a few more enzymatic steps, an intermediate called Baccatin-III is synthesised. This intermediate is available from renewable natural sources and can thus be used as a precursor to feed a cell population in order to evolve a population of cells able to produce Taxol like compounds. Once this has been achieved, the genes identified in the process should be locked. The number of steps from a yeast metabolite to Baccatin-III can be reduced by incorporating in the Taxol producing population the first genes of the pathway which are known: crtE (phytoene synthase) which can modify a yeast metabolite, IPP, to produce geranylgeranyl diphosphate, taxadiene synthase and taxadiene 5α-hydroxylase. After a second module of evolution where no precursor feeding is used, a population of cells able to produce Taxol like compounds will have been established.

For each modular evolution the procedure as that described in Example 3 is performed, except that the following changes are made to the following numbered steps.
- Step 7: The screening population is divided into 2 equal portions. One of the portions is screened for stabilisation of microtubule assembly (step 8) and the other growth inhibition of murin fibroblast cells (step 9)
- Step 8: Stabilisation of microtubule assembly:
a. The heterologous genes are induced/de-repressed by re-suspending the cells in a medium lacking methionine and with 200 µM Cu₂SO₄ and the cells are grown under inducting conditions for 24 hours prior to screening.
b. The EVAC containing cells are co-encapsulated with 5 mg tubulin /ml.
c. Micro capsules are suspended in polymerisation buffer and incubated at 37 C for 1 h. A fraction of the population is passed through the flow cytometer to determine the average light scatter.
d. In parallel a control population of microcapsules are incubated in the same buffer lacking GTP. The population is passed through the flow cytometer to establish light scatter of microcapsules containing depolymerised tubulin.
e. The suspension is cooled to 5 C for 30 min. and microcapsules maintaining a light scatter closest to the light scatter on the capsules containing polymerised tubulin is recovered and processed through step 10.
f. The 10% of the cell population with the highest light scatter is selected.
- Step 9: Growth inhibition of murine fibroblast cells:
a. The screening population is grown in liquid culture under selective conditions for the artificial chromosomes to an OD₆₀₀ of 0.6 - 1.0.
*b*. The heterologous genes are induced/de-repressed by re-suspending the cells in a medium lacking methionine and with 200 µM Cu₂SO₄ a. An EVAC containing cell population is grown under induction conditions.
c. Murine fibroblast cells are co-encapsulated with the EVAC containing cells in gel microdroplets in fibroblast growth media.
d. Incubation is conducted for 24 hours, after which the droplets are screened by flow cytometry and the level of cell proliferation by the bacteria in each droplet is measured.
e. Droplets with the lowest cell proliferation are selected such that 10% of host cell lines entering the screen are selected.
- Step 10: Each of the populations selected in steps 8) and 9) is amplified. For each of the selected populations, steps 11-13 are repeated separately.
- Step 16: The entire population is now screened for stabilisation of microtubule assembly. Selected cells are then screened for growth inhibition of murine fibroblast cells.
- Step 19: Cells that have an activity similar to that produced by a 10 nM concentration of Taxol are taken out of the evolution process. The genes responsible for these activities are characterised by subcloning and DNA sequencing.

### PRIORITISED SPECIES AND TAXONOMIC GROUPS

Sourced species are divided into:
- Species that produce Taxol and other Taxol like compounds,
- Organisms phylogeneticly related to the Taxol producers
- Organisms known to have relevant pharmacological activities, i.e., anticancer
- Specific genes coding for enzymes known to be involved in the Taxol biosynthesis

Species that produce Taxol:
Plants: Taxus brevifolia, Taxus cuspidata, Taxus yunnanensis, Taxus canadensis, Taxus baccata, Taxus wallichiana, Taxus mairei, Taxus chinensis, Taxus media; Fungii: Taxomyces andreanae, Taxomyces wallichiana, Taxomyces baccata, Taxomyces canadensis

Organisms phylogeneticaly related to the Taxol producers:
Same family: *Taxus globosa, Taxus biternata, Taxus caespitosa, Taxus recurvata, Taxus umbraculifera, Taxus concorta, Taxus sumatrana, Torreya grandis, Torreya nucifera;* Cupressaceae Family : *Callitris arborea, Chamaecyparis lawsoniana, Cupressus arizonica, Juniperus chinensis, Juniperus recurvus, Tetraclinis articulata, Thuja occidentalis, Widdringtonia cupressoides;* Podocarpaceae Family *Podocarpus ferrugineus*; Araucariaceae Family*: Agathis alba, Araucaria imbricata, Agathis australis,* Pinaceae Family: *Abies balsamea, Abies webbiana, Cedrus deodora, Larix europaea, Picea rubens, Pinus australis, Pinus pinaster, Pinus wallichiana, Pseudotsuga taxifolia, Tsuga canadensis,* Cephalotaxaceae family: C. fortunei, C. Hauringtonic, Sciadopityaceae family: Sciadopitys verticillata

Organisms reported to have antineoplastic activity:
PLANTS*:Chelidonium majus* (Celandine-plant), *Rheum officinale* (Chinese Rhubarb, Root), *Rheum rhabarbarum* (Rhubarb, Root), *Allium cepa* (Onion, bulb), *Aloe vera* (Aloe, plant), *Arachis hypogaea* (Groundnut, Seed), *Brassica oleracea var. capitata* (Cabbage, Leaf), *Cassia tora* (Sickle Senna, Seed), *Coptis chinensis* (Chinese Goldthread, Rhizome), *Coptis japonica* (Huang-Lia, Rhizome), *Coptis spp* (Generic Goldthread, Rhizome), *Corydalis spp* (Fumewort, Plant), *Eschscholzia californica* (California Poppy, Shoot), *Glaucium flavum* (Horned Poppy, Root), *Papaver somniferum* (Opium Poppy, Plant), *Polygonum multiflorum* (Chinese Cornbind, Root), *Rheum palmatum* (Chinese Rhubarb, Root), *Rumex hymenosepalus* (Canaigre, Root), *Sanguinaria canadensis* (Bloodroot, Root), *Senna alata* (Ringworm Bush, Plant), *Adonis vernalis* (Spring Adonis, Plant),
ANIMALS, Sponges: *Corticium sp, Zyzzya cf. fuliginosa, Chondropsis sp, Diacarnus erythraenus*; jellyfish: *Carybdea rastonii, Chrysaora quinquecirrha;* anemones: *Actinia equina, Anemonia viridis*, Insects: *Papilio polyxenes, Drosophila melanogaster, Rhodnius prolixus, Apis mellifera, Lacanobia oleracea;* Spiders: *Tarantula keratouveitis, Loxosceles deserta, Loxosceles reclusa;* Crabs: Clibanarius longitarsus, Tachypleus tridentatus, Uca pugilat, Worms: *Schistosoma mansoni;* Snails: *Lippia sidoides, Lymnaea stagnalis, Stylocheilus longicauda, Biomphalaria glabrata;* Snakes: *Bothrops jararaca, Crotalus durissus, Vipera aspis, Sistrurus Malarius Barbouri;* Sea urchin: *Toxopneustes pileolus*; Starfish: *Acalycigorgia inermis, Asterina pectinifera, Fromia monilis,*

### Evolution of cells towards specific drug targets

### Example 5: Evolution of HIV protease inhibitors

### UTILITY.

Acquired immunodeficiency syndrome (AIDS) is a fatal pathogenic disease caused by the HIV virus. AIDS is prevalent in almost every country on the globe, and it is estimated that 36 million people are infected worldwide. HIV Protease is an enzyme expressed by the HIV Virus. Molecules that inhibit HIV Protease have utility in the treatment of HIV infections. Desirable attributes of such molecules include activity against HIV, specificity for HIV protease, ability to cross cell membranes in order to reach the virus and others.

### SCREENING & SELECTION STRATEGY

Assays that measure the activity of HIV Protease can be constructed by labelling the protein substrate of the protease with a fluorescent dye such that in the undigested substrate the fluorescence is quenched but in the enzymatically digested substrate the fluorescence is unquenched. Procedures for constructing such screens are given in "Activity and dimerization of human immunodeficiency virus protease as a function of solvent composition and enzyme concentration." Jordan SP, Zugay J, Darke PL, Kuo LC. J Biol Chem 267, 20028-20032 (1992).

### PROCEDURE.

The same procedure as that described in Example 1 is performed, except that the following changes are made to the following numbered steps
- Step 7: The screening population is not divided.
- Step 8: The EVAC containing cell population is grown for 12 hours under conditions that induce expression. A reporter assay constructed as above is incorporated proximal to the host cell in a microdroplet. The microdroplet is incubated in screening medium. At 2 hour intervals 10% of the microdroplets are run through a flow cytometer and screened for level of fluorescence. In each 2 hour batch those droplets with the lowest level of fluorescence are selected such that statistically 10% of host cell lines are represented in the selected cells. Selected cells are immediately placed into media that halts induction of the heterologous genes. The selected cells from each batch are pooled
- Step 9: There is no step 9
- Step 10: The population selected in step 8 is amplified
- Step 16: Step 16 is conducted as per step 8. The screening criteria are set such that 5% of the cell lines entering the screen are selected
- Step 19: Cells that inhibit HIV protease to the same extent as Indinavir does at a 25nM concentration are taken out of the evolution process. The genes responsible for these activities are characterised by subcloning and DNA sequencing

### PRIORITISED SPECIES AND TAXONOMIC GROUPS

- Random group of taxonomically diverse eukaryotic species

### Example 6: Evolution of DNA Topoisomerase II Poisons

### UTILITY.

Topoisomerase II is an essential enzyme in cell division - it regulates the topology of DNA and in particular performs a process whereby it cuts open double stranded DNA, passes another strand through the break, and then reseals the break. Compounds (such as doxorubicin and etoposide) that cause the toposiomerase II enzyme to generate but not seal these DNA double strand breaks have proven utility as anti-cancer agents. New compounds with such activity but different pharmacological properties have utility as compounds for the treatment of cancer, and indeed other proliferative diseases.

### SCREENING & SELECTION STRATEGY

Topoisomerase II poisons (such as doxorubicin and etoposide) are preferentially toxic to cells that have high levels of topoisomerase II - their toxic effect is achieved by causing the topoisomerase enzyme to generate double strand breaks in DNA.

Their effects are antagonised by compounds (such as chloroquine and dexrazoxane) that act on the enzyme without causing the double strand breaks. These properties can be used to construct a process for selecting and evolving cells that produce compounds that act as topoisomerase poisons Yeast host cells are described in the scientific literature that express functional human topoisomerase II enzyme and depending on the temperature not the wild type yeast topisomerase II enzyme, Wasserman R. et al, Cancer Research, 1993, 53, 3591.

### PROCEDURE

The same procedure as that described in Example 1 is performed, except that the following changes are made to the following numbered steps
- Step 5.: EVAC containing cell populations are made using 10 different normalised and enriched cDNA libraries in each in one of the above described mutant strains.
- Step 7: The screening population is not divided.
- Step 8: Topoisomerase Poison activity:
a. The population is grown in liquid culture under selective conditions for the artificial chromosomes to an OD₆₀₀ of 0.6 - 1.0.
b. The heterologous genes are induced/de-repressed by re-suspending the cells in a medium lacking methionine and with 200 µM Cu₂SO₄ and at 35°C. The cells are grown under induction conditions for 12 hours. Every 3 hours 25 % of the cell population is screened for DNA damage by flow cytometry. The 25% of cells in each titre showing least DNA damage are discarded and the remainder immediately placed in non-inducing media that contains dexrazoxane at 100 micromolar concentration
c. Those cells that survive and are selected from step 8b. are resuspended and grown under induction conditions for a further 12 hours, but this time with dexrazoxane at 100 micromolar concentration throughout the 12 hour period At the end of the period the cells are screened for DNA damage by flow cytometry and cells representing the 25% of cell lines showing least DNA damage are selected.
- Step 9: There is no step 9
- Step 10: The population selected in step 8 is amplified
- Step 16: Is conducted as per step 8.
- Step 19: Commencing with the tenth cycle cells that, in step 8b, show DNA damage of an equivalent level to that caused by the addition of 5 micromolar etoposide are taken out of the evolution process. The genes responsible for these activities are characterised by subcloning and DNA sequencing

### PRIORITISED SPECIES AND TAXONOMIC GROUPS

- Plants that produce podophyllotoxins
- Species generally known to have anticancer activity

### Example 7: Evolution of p53 Activators

### UTILITY

p53 is a well known tumour suppressor gene that induces apoptosis in otherwise oncogenic cells. Dysfunctional p53 can mean that the apoptosis does not occur and the oncogenic cell proliferates. A large proportion of tumours have dysfunctional p53 protein.

p53 protein acts as a transcription factor. Many forms of dysfunctional p53 have lost the ability to act as a transcription factor. Tumour cells with such a dysfunctional form accumulate the p53 protein but transcription and hence apoptosis does not occur. In such tumour cells, compounds that restore the ability of such dysfunctional p53 to initiate transcription, and hence apoptosis would have utility as anti-cancer agents. Rastinejad F., Science, 1999, 286, 2507-2510.

### SCREENING & SELECTION STRATEGY

p53 is a transcription factor. A standard intracellular reporter system for p53 comprises a) a genetic construct whereby a gene encoding for GFP or a similar reporter protein is placed under the control of a heterologous p53 inducible promoter, together with b) an inducible genetic construct encoding for a dysfunctional p53. Such a construct can be used to screen for compounds that activate the transcription of the GFP.

### PROCEDURE

The same procedure as that described in Example 1 is performed, except that the following changes are made to the following numbered steps
- Step 5.: EVAC containing cell populations are made using 10 different normalised and enriched cDNA libraries in each. The cell populations are then further transformed according to standard protocols with a p53 reporter system as described above
- Step 7: The screening population is not divided
- Step 8: p53 activation screen:
a. The population is grown in liquid culture under selective conditions for the artificial chromosomes and the p53 reporter system to an OD₆₀₀ of 0.6 - 1.0.
b. The heterologous genes are induced/de-repressed by re-suspending the cells in a medium lacking methionine and with 200 µM Cu₂SO₄ and the cells are grown under induction conditions for 36 hours. Any cells producing GFP are discarded.
c. The dysfunctional p53 is induced and induction maintained for 5 days, alongside induction/de-repression of the EVAC genes. Cells are observed for production of GFP after 1 hour and then at 12 hour intervals throughout the five days. Those cells that produce GFP are selected.
- Step 9: There is no step 9
- Step 10: The populations selected in step 8) is amplified
- Step 16: The entire population is screened as per step 8). The screening criteria are set such that 5% of the cell lines entering the screen are selected
- Step 19: Cells that produce GFP within 1 hour of induction are taken out of the evolution process. The genes responsible for these activities are characterised by subcloning and DNA sequencing.

### PRIORITISED SPECIES AND TAXONOMIC GROUPS

- Organisms reported to have anticancer properties (see list in example 4)
- Random group of taxonomically diverse eukaryotic species

### Example 8: Evolution of Fumarate Reductase inibitors

### UTILITY

Fumarate reductase reduces fumarate to succinate and is an essential step in anaerobic metabolism for many organisms including pathogens from such genera as Leishmania, Helicobacter, Staphylococcus and Streptococcus.

Compounds that inhibit the activity of fumarate reductase can prevent such parasites or pathogens from completing their life cycle and hence are of utility in controlling such diseases. Because fumarate reductase does not occur in humans, such compounds should not have significant toxicity to humans.

### SCREENING & SELECTION STRATEGY

Fumarate reductase activity is measured by the rate at which it oxidises NADH upon the addition of fumarate. The progress of the enzymatic reaction is measured spectrophotometrically at 340nm. For detailed protocols see Chen et al., Antimicrob. Agents Chemother., 2002, 2023-2029.

### PROCEDURE

The same procedure as that described in Example 1 is performed, except that the following changes are made to the following numbered steps
- Step 7: The screening population is not divided
- Step 8: Fumarate reductase screen:
a. The screening population is amplified ten times
b. The heterologous genes are induced/de-repressed by re-suspending the cells in a medium lacking methionine and with 200 µM Cu₂SO₄ and the cells are grown under inducting conditions for 24 hours prior to screening.
c. Fumarate reductase is co-encapsulated with EVAC containing cells in a gel microdroplet.
d. Microdroplets containing fumarate reductase but not EVAC containing cells were incubated in a liquid media containing 100 micromolar NADH and 1mM fumarate and incubated at 30 C. Aliquotes were analysed by flow cytometry to determine the optimal incubation time.
e. The microdroplet is placed in a liquid media that contains 100 micromolar NADH and 1mM fumarate and incubated for the optimal period of time.
f. The gel microdroplets are then passed through a flow cytometer and absorption at 340nm measured. The activity of the enzyme is calculated from the level of absorption at 340nm. The cells with the lowest activity of enzyme are selected such that cells representing the 10% of cell lines entering the screen with the greatest inhibition of fumarate reductase are selected
- Step 9: There is no step 9
- Step 10: The populations selected in step 8 is amplified.
- Step 16: This is performed as per step 8. The screening criteria are set such that 5% of the cell lines entering the screen are selected
- Step 19: Cells that show inhibition of fumarate reductase of a same or greater extent than is achieved by adding licochalcone to the droplets at a concentration of 1 micromolar are taken out of the evolution process. The genes responsible for these activities are characterised by subcloning and DNA sequencing.

### PRIORITISED SPECIES AND TAXONOMIC GROUPS

- Plant roots
- Random group of taxonomically diverse eukaryotic species

### Evolution of function independent of specific targets

### Example 9: Evolution of cytoprotectants

### UTILITY

One of the central problems of cancer chemotherapy is that the anti-cancer agents used kill normal cells as well as cancer cells. The side effects of killing the normal cells can be so severe as to be life-threatening, and frequently mean that treatment of the cancer has to be abandoned. Compounds that protect cells against such anti-cancer agents therefore have utility in reducing the side effects of cancer chemotherapy, hence improving both therapeutic outcome and patient life quality. Existing examples of such compounds include dexrazoxane and chloroquine, both of which protect cells agains the cytotoxic effects of cancer agents such as etoposide. However more such protectants are needed.

### SCREENING & SELECTION STRATEGY

Host cells can be induced screened and selected for survival in the presence of cytotoxic anti-cancer agents such as doxorubicin, taxol, vincristine and cisplatin. Over a series of selection rounds the concentration of cytotoxic agent that a cell must survive to be selected can be increased

### PROCEDURE

The same procedure as that described in Example 1 is performed, except that the following changes are made to the following numbered steps
- Step 7: The screening population is divided into two equal portions. One of the portions is screened against etoposide (step 8) and the other screened against vincristine (step 9)
- Step 8: Etoposide screen:
a. The screening population is amplified ten times and divided in 10 portions.
b. The sub populations are grown in liquid culture under selective conditions for the artificial chromosomes to an OD₆₀₀ of 0.6 - 1.0.
c. The heterologous genes are induced/de-repressed by re-suspending the cells in a medium lacking methionine and with 200 µM Cu₂SO₄ and the cells are grown under induction conditions for 36 hours prior to screening.
d. Each sub population is diluted so as to have on average each cell line represented 3 times.
e. Each sub population is exposed to 1 out of a range of 10 concentrations of etoposide. Survival rates are determined after 2 hours. The surviving cell population from the highest concentration of etoposide where cells statistically representing 10% of the cell lines survived is selected.
- Step 9: Vincristine screen:
a. The screening population is amplified ten times and divided in 10 portions.
b. The sub populations are grown in liquid culture under selective conditions for the artificial chromosomes to an OD₆₀₀ of 0.6 - 1.0.
c. The heterologous genes are induced/de-repressed by re-suspending the cells in a medium lacking methionine and with 200 µM Cu₂SO₄ and the cells are grown under induction conditions for 36 hours prior to screening.
d. Each sub population is exposed to 1 out of a range of 10 concentrations of vincristine. Survival rates are determined after 2 hours. The surviving cell population from the highest concentration of vincristine where cells statistically representing 10% of the cell lines survived is selected.
- Step 10: Each of the populations selected in steps 8) and 9) is amplified and equal amounts of each amplified population are pooled

- Step 16: The entire population is now screened for both etoposide and vincristine resistance. The screening criteria are set such that 5% of the cell lines entering the screen are selected
- Step 19: Cells that, compared to the original population, show the ability to resist a 10-fold higher concentration of etoposide, or a 10-fold higher concentration of vincristine, or a combined 5-fold higher dose of both together, are taken out of the evolution process. The genes responsible for these activities are characterised by subcloning and DNA sequencing.

### PRIORITISED SPECIES AND TAXONOMIC GROUPS

- Plant species of the genera Vinca
- Organisms known to produce Taxol (see list in example 4)

### Example 10: Evolution of antibacterials

### UTILITY

The widespread emergence of resistance has significantly limited the efficacy of classical antibiotic therapy for bacterial disease. Fuelled largely by the excessive and often unnecessary use of antibiotics in humans and animals, antibiotic resistance has resulted in increased patient morbidity, mortality and overall cost of health care.

There is a strong medical need for new therapeutics to treat emerging antibioticresistant infections. A premium is placed upon inhibitors that function by a novel or at least different mechanism than currently approved antibiotics, as these would be expected to circumvent current bacterial resistance mechanisms.

### SCREENING & SELECTION STRATEGY

Screening can be done using gel microdroplets and flow cytometry or an overlay system. Use of a multiple drug-resistant strain in the primary screen will *a priori* select for hits that have activity against a multi-drug resistant strain.

The approach set out in this example can be applied to a range of micro-organisms other than the one described here. Mammalian cells can be used in one or more selection rounds and used to select for host cells that are not producing compounds with mammalian cell toxicity.

### PROCEDURE

The same procedure as that described in Example 1 is performed, except that the following changes are made to the following numbered steps
- Step 7: The screening population is not divided.
- Step 8: Antibacterial screen:
a. An EVAC containing cell population is grown under induction conditions.
b. *Staphylococcus aureus* is co-encapsulated with the EVAC containing cells in gel microdroplets.
c. Incubation is conducted for 24 hours, after which the droplets are screened by flow cytometry and the level of cell proliferation by the bacteria in each droplet is measured.
d. Droplets with the lowest cell proliferation are selected such that 10% of host cell lines entering the screen are selected.
- Step 9: There is no Step 9
- Step 10: The population selected in step 8 is amplified.
- Step 16: The population is now directly screened for bacterial cell death rather than simply cessation of growth by use of a fluorescent live/dead marker (fluorescine diacetate). The screening criteria are set such that 5% of the cell lines entering the screen are selected
- Step 19: Cells that have shown an antibacterial activity equal to that achieved with vancomycin at a concentration of 1 µg/ml are taken out of the evolution process. The genes responsible for these activities are characterised by subcloning and DNA sequencing

### PRIORITISED SPECIES AND TAXONOMIC GROUPS

- Fungi
- Random group of taxonomically diverse eukaryotic species

### Example 11: Evolution of Angiogenesis inhibitors

### UTILITY

Angiogenesis is a necessary process to provide adequate blood supply for the growth of many tumours. It is also an important complication of diseases such as diabetes where angiogenesis in the eye can be a cause of visual impairment.

### SCREENING & SELECTION STRATEGY

In vitro screens for angiogenesis inhibition exist based upon measuring the proliferation of endothelial cells in the presence of a growth factor. The specificity of given compounds for inhibition of angiogenesis (as opposed to effects due to general toxicity) can be assessed by investigating the effect of such compounds on the proliferation of non endothelial cells without the addition of a growth factor.

### PROCEDURE

The same procedure as that described in Example 1 is performed, except that the following changes are made to the following numbered steps
- Step 7: The screening population is not divided. The entire population is advanced to step 8
- Step 8: Bovine capillary endothelial cell screen:
a. BCECs are co-encapsulated with host cells into standard gelatinous growth media under conditions that limit host cell proliferation but not that of the endothelial cells. Factors to induce the transcription of the heterologous genes in the host cell are added to the media
b. The BCEC's are stimulated to proliferate by the addition of bovine fibroblast growth factor at 1 ng/ml. After a 72 hour incubation time the number of endothelial cells in each capsule is counted by flow cytometry
c. Those capsules in which the least level of BCEC proliferation has occurred are selected. The number of capsules selected is set such that statistically 10% of the cell lines taken into the screen are selected
- Step 9: There is no step 9
- Step 10: The population selected in step 8) is amplified
- Step 16: The entire population is now screened in an identical manner to step 8). However in every alternate screening round the host cell lines are co-encapsulated with non endothelial bovine cell lines, without the addition of bovine fibroblast growth factor and the capsules in which the greatest cell proliferation has occurred are selected. The number of capsules selected in these alternate rounds is set such that statistically 20% of the cell lines taken into the screen are selected.
- Step 19: Commencing on round 10, host cells that inhibit endothelial cell proliferation by a factor of more than 10 are taken out of the evolution process. The genes responsible for these activities are characterised by subcloning and DNA sequencing.

### PRIORITISED SPECIES AND TAXONOMIC GROUPS

- Plant species of the order Ericales and in particular the genus Camellia (tea
   - Camellia sinensis is known to produce epigallocatechin-3-gallate (EGCG) an angiogenesis inhibitor)

### Other examples:

### Example 12: Rare restriction enzymes with recognition sequence and cleavage points

In this example, rare restriction enzymes are listed together with their recognition sequence and cleavage points.

W=AorT; N = A, C, G, or T

| 12 a) | Unique, palindromic overhang | |
|---|---|---|
| AscI | | GG^CGCG_CC |
| AsiSI | | GCG_AT^CGC |
| CciNI | | GC^GGCC_GC |
| CspBI | | GC^GGCC_GC |
| FseI | | GG_CCGG^CC |
| MchAI | | GC^GGCC_GC |
| NotI | | GC^GGCC_GC |
| PacI | | TTA_AT^TAA |
| SbfI | | CC_TGCA^GG |
| SdaI | | CC_TGCA^GG |
| SgfI | | GCG_AT^CGC |
| SgrAI | | CR^CCGG_YG |
| Sse232I | CG^CCGG_CG | |
| Sse8387I | CC_TGCA^GG | |

12b) No overhang

| | | |
|---|---|---|
| BstRZ246I | ATTT^AAAT | |
| BstSWI | ATTT^AAAT | |
| MspSWI | ATTT^AAAT | |
| Mssl | | GTTT^AAAC |
| Pmel | | GTTT^AAAC |
| Smil | | ATTT^AAAT |
| Srfl | | GCCC^GGGC |
| Swal | | ATTT^AAAT |

12c) Non-palindromic and/or variable overhang

| | | |
|---|---|---|
| Aarl | | CACCTGCNNNN^NNNN_ |
| Abel | | CC^TCA_GC |
| Alol | | |
| | ^NNNNN_NNNNNNNGAACNNNNNNTCCNNNNNNN_NNNNN^ | |
| Bael | | |
| | ^NNNNN_NNNNNNNNNNACNNNNGTAYCNNNNNNN_NNNNN^ | |
| | BbvCl | CC^TCA_GC |
| Cpol | | CG^GWC_CG |
| Cspl | | CG^GWC_CG |
| Pfl27l | | RG^GWC_CY |
| Ppil | | |
| | ^NNNNN_NNNNNNNGAACNNNNNCTCNNNNNNNN_NNNNN^ | |
| PpuMl | | RG^GWC_CY |
| PpuXl | | RG^GWC_CY |
| Psp5ll | | RG^GWC_CY |
| PspPPl | RG^GWC_CY | |
| Rsrll | | CG^GWC_CG |
| Rsr2l | | CG^GWC_CG |
| SanDl | | GG^GWC_CC |
| Sapl | | GCTCTTCN^NNN_ |
| Sdil | | GGCCN_NNN^NGGCC |
| SexAl | | A^CCWGG_T |
| Sfil | | GGCCN_NNN^NGGCC |
| Sse1825l | GG^GWC_CC | |
| Sse8647l | AG^GWC_CT | |
| VpaK32l | GCTCTTCN^NNN_ | |

| 12d) | Meganucleases | |
|---|---|---|
| I-Sce I | | TAGGGATAA_CAGG^GTAAT |
| I-Ceu I | | ACGGTC_CTAA^GGTAG |
| I-Cre I | | AAACGTC_GTGA^GACAGTTT |
| I-Sce II | | GGTC_ACCC^TGAAGTA |
| I-Sce III | GTTTTGG_TAAC^TATTTAT | |
| Endo. Sce I | GATGCTGC_AGGC^ATAGGCTTGTTTA | |
| PI-Sce I | GG_GTGC^GGAGAA | |
| PI-Psp I | TGGCAAACAGCTA_TTAT^GGGTATTATGGGT | |
| I-Ppo I | | CTCTC_TTAA^GGTAG |
| HO | | TTTCCGC_AACA^GT |
| I-Tev I | | NN_NN^NNTCAGTAGATGTTTTTCTTGGTCTACCGTTT |

More meganucleases have been identified, but their precise sequence of recognition has not been determined, see e.g. www.meganuclease.com

### Example 13: Concatemer size limitation experiments (use of stoppers)

Materials used:
pYAC4 (Sigma. Burke et al. 1987, science, vol 236, p 806) was digested w. EcoR1 and BamH1 and dephosphorylated
pSE420 (invitrogen) was linearised using EcoR1 and used as the model fragment for concatenation.
T4 DNA ligase (Amersham-pharmacia biotech) was used for ligation according to manufacturers instructions.

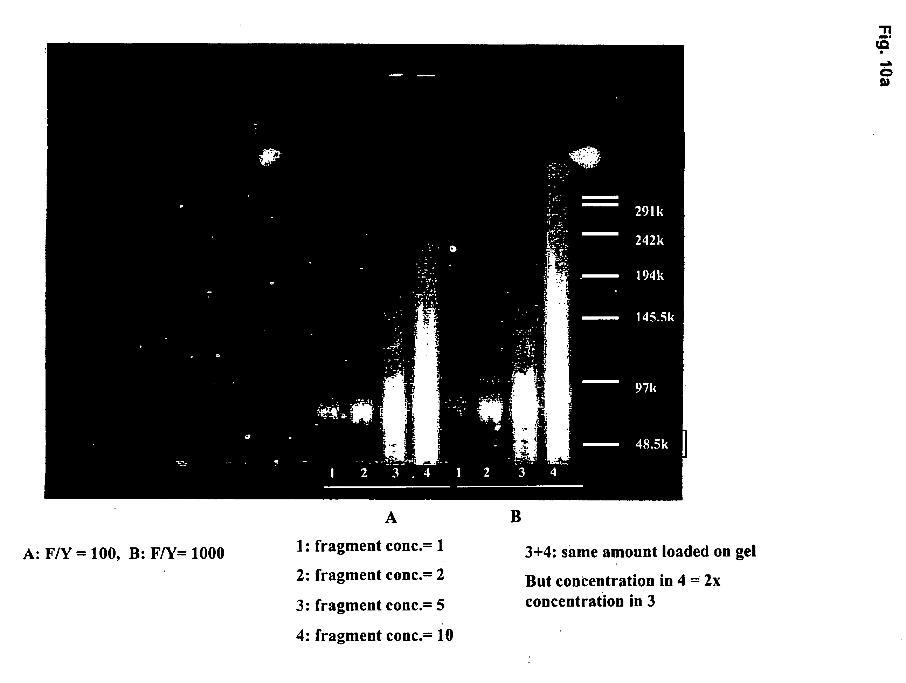

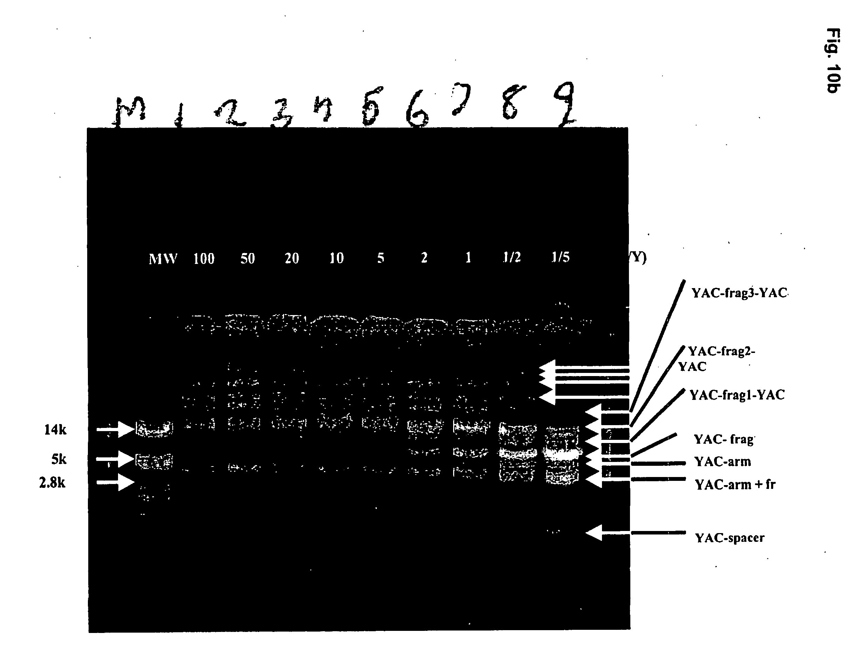

Method: Fragments and arms were mixed in the ratios(concentrations are arbitrary units) indicated on figures. Ligation was allowed to proceed for 1 h at 16C. Reaction was stopped by the addition of 1 µL 500 mM EDTA. Products were analysed by standard agarose GE (1 % agarose, ½ strength TBE) or by PFGE(CHEF III, 1% LMP agarose, ½ strength TBE, angle 120, temperature 12 C, voltage 5.6V/cm, switch time ramping 5 - 25 s, run time 30 h) The results are shown in Figure 10a and 10b.

### Example 14: Expression of different patterns "phenotypes" obtained using the same yeast clones under different expression conditions

Colonies were picked with a sterile toothpick and streaked sequentially onto plates corresponding to the four repressed and/or induced conditions (-Ura/-Trp, -Ura/-Trp/-Met, -Ura/-Trp/+200 µM Cu₂SO₄, -Ura/-Trp/-Met/+200 µM Cu₂SO₄).

## Claims

1. A method for evolving a cell having a desired functionality, said method comprising the steps of
a) obtaining a composition of cells, at least one cell of said composition comprising
a1) in the range of 10 to 1000 heterologous expressible nucleotide sequences, at least one of said sequences being incorporated into an artificial chromosome in the cell, and/or
a2) at least two expression cassettes of the following formula:
[rs₂-SP-PR-X-TR-SP-rs₁]
wherein
rs₁ and rs₂ together denotes a restriction site,
SP individually denotes a spacer,
PR denotes a promoter, capable of functioning in the first cell,
X denotes an expressible nucleotide sequence,
TR denotes a terminator,
b) determining at least one screening functionality,
c) screening the cells of the composition with respect to at least one screening criterion related to the determined screening functionality,
d) selecting cells meeting the at least one screening criterion related to the determined screening functionality,
e) combining the expressible nucleotide sequences of the selected cells with expressible nucleotide sequences from another composition of cells, and
f) repeating steps b) to e) as required until at least one cell has acquired the desired functionality.

2. The method according to claim 1, wherein step c) comprises screening with respect to at least two different screening criteria before selecting cells meeting the at least one screening criterion related to the determined screening functionality.

3. The method according to claim 1 or 2, wherein the step a) comprises one step of combination before obtaining the further modified composition.

4. The method according to any of the preceding claims, wherein the screening criteria is a media based criteria, such as using unusual media substrates, growing cells on toxin comprising media, growing cells on inhibitor comprised media, leading to cells being selected on the basis of survival, superior growth, deviating morphology, stickyness, spectral properties, (modulation of) enzyme activity.

5. The method according to any of the preceding claims wherein the screening criteria is selected from at least one physical criteria, such as temperature, osmolarity, light, and electricity, leading to cells being selected on the basis of survival, superior growth, deviating morphology, stickyness, spectral properties, (modulation of) enzyme activity.

6. The method according to any of the preceding claims, wherein the at least two expressible nucleotide sequences originates from at least two different species.

7. The method according to any of the preceding claims, wherein the strength of the screening criterion/criteria is increased for each repeat and/or the type of screening criterion/criteria is changed for each repeat.

8. The method according to any of the preceding claims, wherein the combination of expressible sequences is a combination of chromosomes in the cells.

9. The method according to any of the preceding claims, wherein the combination of expressible sequences is conducted by removing the expressible sequences from at least two different cells, combining the individual expressible sequences in vitro, and introducing at least two combined expressible sequences into at least two cells.

10. The method according to any of the preceding claims, wherein the desired functionality is a capability of the cell of producing non-native secondary metabolites.

11. The method according to any of the preceding claims, wherein at least one cell of the composition comprises at least one concatemer of individual oligonucleotide cassettes, each concatemer comprising oligonucleotide of the following formula in 5'→3' direction
[rs₂-SP-PR-X-TR-SP-rs₁]ₙ
wherein
rs₁ and rs₂ together denote a restriction site,
SP individually denotes a spacer of at least two nucleotide bases,
PR denotes a promoter, capable of functioning in the cell,
X denotes an expressible nucleotide sequence,
TR denotes a terminator, and
wherein n ≥ 2, and
wherein at least two expressible nucleotide sequences are from different expression states.

12. The method according to any of the preceding claims, wherein substantially all rs₁-rs₂ sequences are recognised by the same restriction enzyme.

13. The method according to claim 12, wherein substantially all rs₁-rs₂ sequences are substantially identical.

14. The method according to claim 1, wherein the cell is a yeast cell selected from the group comprising baker's yeast, Kluyveromyces marxianus, K. lactis, Candida utilis, Phaffia rhodozyma, Saccharomyces boulardii, Pichia pastoris, Hansenula polymorpha, Yarrowia lipolytica, Candida paraffinica, Schwanniomyces castellii, Pichia stipitis, Candida shehatae, Rhodotorula glutinis, Lipomyces lipofer, Cryptococcos curvatus, Candida spp. (e.g. C. palmioleophila), Yarrowia lipolytica, Candida guilliermondii, Candida, Rhodotorula spp., Saccharomycopsis spp., Aureobasidium pullulans, Candida brumptii, Candida hydrocarbofumarica, Torulopsis, Candida tropicalis, Saccharomyces cerevisiae, Rhodotorula rubra, Candida flaveri, Eremothecium ashbyii, Pichia spp., Kluyveromyces, Hansenula, Kloeckera, Pichia, Pachysolen spp., or Torulopsis bombicola.

15. The method according to any of the preceding claims, wherein a restriction site comprises at least 6 bases.

16. The method according to any of the preceding claims, wherein at least one cell of said composition comprises in the range of 60 to 300 heterologous expressible nucleotide sequences.

## Patentansprüche

1. Verfahren zur Entwicklung einer Zelle mit einer gewünschten Funktionalität, wobei das Verfahren die Schritte umfasst
a) Erhalten einer Zusammensetzung von Zellen, worin mindestens eine Zelle der Zusammensetzung umfasst
a1) im Bereich von 10 bis 1000 heterolog exprimierbare Nukleotidsequenzen, worin mindestens eine der Sequenzen in ein artifizielles Chromosom in der Zelle eingebaut ist, und/oder
a2) mindestens zwei Expressionskasetten der folgenden Formel:
[rs₂-SP-PR-X-TR-SP-rs₁]
worin
rs₁ und rs₂ zusammen eine Restriktionsschnittstelle bezeichnen,
SP einzeln einen Abstandshalter bezeichnet,
PR einen Promotor bezeichnet, der in der ersten Zelle funktionieren kann,
X eine exprimierbare Nukleotidsequenz bezeichnet,
TR einen Terminator bezeichnet,
b) Bestimmen von mindestens einer Screening-Funktionalität,
c) Screening der Zellen der Zusammensetzung auf mindestens ein Screening-Kriterium, das mit der bestimmten Screening-Funktionalität assoziiert ist,
d) Auswählen von Zellen, die mindestens das eine Screening-Kriterium erfüllen, das mit der bestimmten Screening-Funktionalität assoziiert ist,
e) Kombinieren der exprimierbaren Nukleotidsequenzen der ausgewählte Zellen mit exprimierbaren Nukleotidsequenzen einer anderen Zusammensetzung von Zellen, und
f) Wiederholen der Schritte b) bis e) nach Bedarf, bis mindestens eine Zelle die gewünschte Funktionalität erworben hat.

2. Verfahren nach Anspruch 1, worin Schritt c) umfasst, Screening auf mindestens zwei unterschiedliche Screening-Kriterien vor Auswählen von Zellen, die das mindestens eine Screening-Kriterium erfüllen, das mit der bestimmten Screening-Funktionalität assoziiert ist.

3. Verfahren nach Anspruch 1 oder 2, worin der Schritt a) umfasst, einen Schritt der Kombination vor Erhalten der weiter modifizierten Zusammensetzung.

4. Verfahren nach einem der vorstehenden Ansprüche, worin das Screening-Kriterium ein Medium basierendes Kriterium ist, wie Verwenden unüblicher Mediensubstrate, Züchten von Zellen in Toxin enthaltenden Medien, Züchten von Zellen in Inhibitor enthaltenden Medien, was zu Zellen führt, die auf der Grundlage des Überlebens, besserem Wachstum, geänderter Morphologie, Anheftungsverhalten, Spektraleigenschaften, (Modulierung der) Enzym-Aktivität ausgewählt werden.

5. Verfahren nach einem der vorstehenden Ansprüche worin die Screening-Kriterium ausgewählt ist unter mindestens einem physikalischen Kriterium, wie Temperatur, Osmolarität, Licht, und Elektrizität, was zu Zellen führt, die auf der Grundlage des Überlebens, besserem Wachstum, geänderter Morphologie, Anheftungsverhalten, Spektraleigenschaften, (Modulierung der) Enzym-Aktivität ausgewählt werden.

6. Verfahren nach einem der vorstehenden Ansprüche, worin die mindestens zwei exprimierbaren Nukleotidsequenzen von mindestens zwei unterschiedlichen Spezies abgeleitet sind.

7. Verfahren nach einem der vorstehenden Ansprüche, worin die Stärke des/der Screening-Kriteriums/Krierien für jede Wiederholung erhöht wird und/oder der Typ des/der Screening-Kriteriums/Krierien für jede Wiederholung verändert wird.

8. Verfahren nach einem der vorstehenden Ansprüche, worin die Kombination an exprimierbaren Sequenzen eine Kombination von Chromosomen in den Zellen ist.

9. Verfahren nach einem der vorstehenden Ansprüche, worin die Kombination an exprimierbaren Sequenzen durchgeführt wird durch
Entfernen der exprimierbaren Sequenzen aus mindestens zwei unterschiedlichen Zellen,
Kombinieren der einzelnen exprimierbaren Sequenzen in vitro, und
Einbringen von mindestens zwei kombinierten exprimierbaren Sequenzen in mindestens zwei Zellen.

10. Verfahren nach einem der vorstehenden Ansprüche, worin die gewünschte Funktionalität eine Fähigkeit der Zelle ist, nicht-native sekundäre Metaboliten herzustellen.

11. Verfahren nach einem der vorstehenden Ansprüche, worin mindestens eine Zelle der Zusammensetzung umfasst, mindestens ein Concatemer einzelner Oligonukleotid-Kassetten, wobei jedes Concatemer ein Oligonukleotid der folgenden Formel in 5'→3' Richtung umfasst
[rs₂-SP-PR-X-TR-SP-rs₁]ₙ
worin
rs₁ und rs₂ zusammen eine Restriktionsschnittstelle bezeichnen,
SP einzeln einen Abstandshalter von mindestens zwei Nukleotidbasen bezeichnet,
PR einen Promotor bezeichnet, der in der Zelle funktionieren kann,
X eine exprimierbare Nukleotidsequenz bezeichnet,
TR einen Terminator bezeichnet, und
worin n ≥ 2, und
worin mindestens zwei exprimierbare Nukleotidsequenzen aus unterschiedlichen Expressions-Zuständen sind.

12. Verfahren nach einem der vorstehenden Ansprüche, worin im Wesentlichen alle rs₁-rs₂ Sequenzen von dem gleichen Restriktionsenzym erkannt werden.

13. Verfahren nach Anspruch 12, worin im Wesentlichen alle rs₁-rs₂ Sequenzen im Wesentlichen identisch sind.

14. Verfahren nach Anspruch 1, worin die Zelle eine Hefezelle ist, ausgewählt aus der Gruppe umfassend Bäcker-Hefe, Kluyveromyces marxianus, K. lactis, Candida utilis, Phaffia rhodozyma, Saccharomyces boulardii, Pichia pastoris, Hansenula polymorpha, Yarrowia lipolytica, Candida paraffnica, Schwanniomyces castellii, Pichia stipitis, Candida shehatae, Rhodotorula glutinis, Lipomyces lipofer, Cryptococcos curvatus, Candida spp. (beispielsweise C. palmioleophila), Yarrowia lipolytica, Candida guilliermondii, Candida, Rhodotorula spp., Saccharomycopsis spp., Aureobasidium pullulans, Candida brumptii, Candida hydrocarbofumarica, Toruiopsis, Candida tropicalis, Saccharomyces cerevisiae, Rhodotorula rubra, Candida flaveri, Eremothecium ashbyii, Pichia spp., Kluyveromyces, Hansenula, Kloeckera, Pichia, Pachysolen spp., oder Torulopsis bombicola.

15. Verfahren nach einem der vorstehenden Ansprüche, worin eine Restriktionsstelle mindestens 6 Basen umfasst.

16. Verfahren nach einem der vorstehenden Ansprüche, worin mindestens eine Zelle der Zusammensetzung heterolog exprimierbare Nukleotidsequenzen im Bereich von 60 bis 300 umfasst.

## Revendications

1. Méthode pour développer des cellules ayant une fonctionnalité souhaitée, la dite méthode comprenant les étapes de :
a) obtention d'une composition de cellules, au moins une cellule comprenant
a1) des séquences nucléotidiques hétérologues susceptibles de s'exprimer dans une gamme de 10 à 1000, au moins une des dites séquences étant incorporée dans un chromosome artificiel dans la cellule, et/ou
a2) au moins deux cassettes d'expression ayant la formule suivante :
[rs₂-SP-PR-X-TR-SP-rs₁]
dans laquelle rs₁ et rs₂ correspondent ensemble à un site de restriction,
SP seul correspond à un spacer,
PR correspond à un promoteur capable de fonctionner dans la première cellule,
X correspond à une séquence nucléotidique susceptible de s'exprimer,
TR correspond à un terminateur,
b) détermination d'au moins une fonctionnalité de criblage,
c) tri des cellules de la composition par rapport à au moins un critère de criblage en relation avec la fonctionnalité de criblage souhaitée,
d) sélection des cellules satisfaisant au au moins un critère de criblage en relation avec la fonctionnalité de criblage souhaitée,
e) combinaison des séquences nucléotidiques susceptibles de s'exprimer à partir des cellules sélectionnées avec des séquences nucléotidiques susceptibles de s'exprimer provenant d'une autre composition de cellules, et
f) répétition des étapes b) à e) comme indiqué jusqu'à ce que au moins une cellule ait acquis la fonctionnalité souhaitée.

2. Méthode selon la revendication 1 dans laquelle l'étape c) comprend un criblage en relation avec aux moins deux critères de criblage différents avant la sélection des cellules satisfaisant au au moins un critère de criblage en relation avec la fonctionnalité de criblage souhaitée.

3. Méthode selon l'une des revendications 1 ou 2 dans laquelle l'étape a) comprend une étape de combinaison avant l'obtention d'une composition modifiée ultérieurement.

4. Méthode selon l'une quelconque des revendications précédentes dans laquelle le critère de criblage est un critère basé sur le milieu, tel que l'utilisation de milieux substrats inhabituels, la croissance de cellules sur des milieux comprenant des toxines, la croissance de cellules sur des milieux comprenant un inhibiteur, ce qui conduit à sélectionner des cellules sur la base de la survie, d'une croissance supérieure, d'une morphologie déviante, du pouvoir collant, des propriétés spectrales, (de la modulation) de l'activité enzymatique.

5. Méthode selon l'une quelconque des revendications précédentes dans laquelle le critère de criblage est choisi parmi au moins un critère physique, tel que la température, l'osmolarité, la lumière et l'électricité, ce qui conduit à sélectionner des cellules sur la base de la survie, d'une croissance supérieure, d'une morphologie déviante, du pouvoir collant, des propriétés spectrales, (de la modulation) de l'activité enzymatique.

6. Méthode selon l'une quelconque des revendications précédentes dans laquelle les au moins deux séquences nucléotidiques susceptibles de s'exprimer sont issues d'au moins deux espèces différentes.

7. Méthode selon l'une quelconque des revendications précédentes dans laquelle le poids du critère de criblage est augmenté à chaque répétition et/ou le type de critère de criblage est changé à chaque répétition.

8. Méthode selon l'une quelconque des revendications précédentes dans laquelle la combinaison des séquences nucléotidiques susceptibles de s'exprimer est une combinaison de chromosomes dans des cellules.

9. Méthode selon l'une quelconque des revendications précédentes dans laquelle la combinaison des séquences nucléotidiques susceptibles de s'exprimer est réalisée en enlevant les séquences nucléotidiques susceptibles de s'exprimer d'au moins deux cellules différentes, en combinant les séquences individuelles susceptibles de s'exprimer in vitro et en introduisant au moins deux séquences nucléotidiques susceptibles de s'exprimer combinés dans au moins deux cellules.

10. Méthode selon l'une quelconque des revendications précédentes dans laquelle la fonctionnalité désirée est la capacité d'une cellule de produire des métabolites secondaires non-natifs.

11. Méthode selon l'une quelconque des revendications précédentes dans laquelle au moins une cellule de la composition comprend au moins un concatémère de cassettes d'oligonucléotide individuel, chaque concatémère comprenant un oligonucléotide ayant la formule suivante dans la directions 5'→ 3'
[rs₂-SP-PR-X-TR-SP-rs₁]ₙ
dans laquelle
rs₁ et rs₂ correspondent ensemble à un site de restriction,
SP seul correspond à un spacer d'au moins deux bases nucléotidiques,
PR correspond à un promoteur capable de fonctionner dans la cellule,
X correspond à une séquence nucléotidique susceptible de s'exprimer,
TR correspond à un terminateur, et
dans laquelle n≥2, et
dans laquelle au moins deux séquences nucléotidiques susceptibles de s'exprimer sont à deux stades différents d'expression.

12. Méthode selon l'une quelconque des revendications précédentes dans laquelle substantiellement toutes les séquences rs₁- rs₂ sont reconnues par la même enzyme de restriction.

13. Méthode selon la revendication 12 dans laquelle substantiellement toutes les séquences rs₁- rs₂ sont identiques.

14. Méthode selon la revendication 1 dans laquelle la cellule est une cellule de levure choisie dans le groupe comprenant la levure de boulanger, Kluyveromyces marxianus, K. lactis, Candida utilis, Phaffia rhodozyma, Saccharomyces boulardii, Pichia pastoris, Hansenula polymorpha, Yarrowia lipolytica, Candida paraffinica, Schwanniomyces castellii, Pichia stipitis, Candida shehatae, Rhodotorula glutinis, Lipomyces lipofer, Cryptococcos curvatus, Candida spp. (e. g. C. palmioleophila), Yarrowia lipolytica, Candida guilliermondii, Candida, Rhodotorula spp., Saccharomycopsis spp., Aureobasidium pullulans, Candida brumptii, Candida hydrocarboiumarica, Torulopsis, Candida tropicalis, Saccharomyces cerevisiae, Rhodotorula rubra, Candida flaveri, Eremothecium ashbyii, Pichia spp., Kluyveromyces, Hansenula, Kloeckera, Pichia, Pachysolen spp.,or Torulopsis bombicola.

15. Méthode selon l'une quelconque des revendications précédentes dans laquelle le site de restriction comprend au moins 6 bases.

16. Méthode selon l'une quelconque des revendications précédentes dans laquelle au moins une cellule de ladite composition comprend des séquences nucléotidiques hétérologues susceptibles de s'exprimer dans une gamme de 60 à 300.
